# EUROPEAN PATENT SPECIFICATION

(11) **EP 1 817 057 B1**
(45) Date of publication and mention of the grant of the patent: **02.06.2010**
(21) Application number: 05851520.6
(22) Date of filing: 10.11.2005
(51) Int. Cl.: A61K 45/06, A61K 38/20, A61K 31/29, A61P 35/00

(54) **SODIUM STIBOGLUCONATE AND IL-2 FOR TREATING CANCER**
STIBOGLUCONATE-NATRIUM UND IL-2 ZUR KREBSBEHANDLUNG
STIBOGLUCONATE DE SODIUM ET IL-2 POUR LE TRAITEMENT DU CANCER

(30) Priority: 11.11.2004 US 987217
(43) Date of publication of application: 15.08.2007
(73) Proprietor: THE CLEVELAND CLINIC FOUNDATION, Cleveland, OH 44195 (US)
(72) Inventor: YI, Taolin, Solon, Ohio 44139 (US)
(74) Representative: Graf von Stosch, Andreas
(86) International application number: PCT/US2005/040825
(87) International publication number: WO 2006/053162

(56) References cited:
- WO-A-03/063788
- WO-A-03/070158
- US-A1- 2003 161 893
- US-A1- 2005 215 629
- FAN KEKE ET AL: "Sodium stibogluconate interacts with IL-2 in anti-Renca tumor action via a T cell-dependent mechanism in connection with induction of tumor-infiltrating macrophages." JOURNAL OF IMMUNOLOGY (BALTIMORE, MD. : 1950) 15 NOV 2005, vol. 175, no. 10, 15 November 2005 (2005-11-15), pages 7003-7008, XP002371259 ISSN: 0022-1767
- ESLAMI Z ET AL: "Synergism of IL-2-stimulated splenocytes and Pentostam enhances the killing of Leishmania donovani in vitro." CLINICAL IMMUNOLOGY AND IMMUNOPATHOLOGY. OCT 1996, vol. 81, no. 1, October 1996 (1996-10), pages 74-81, XP002371260 ISSN: 0090-1229
- OPAS E E ET AL: "Alendronate inhibition of protein-tyrosine-phosphatase-meg1." BIOCHEMICAL PHARMACOLOGY. 15 SEP 1997, vol. 54, no. 6, 15 September 1997 (1997-09-15), pages 721-727, XP002371261 ISSN: 0006-2952
- PATENT ABSTRACTS OF JAPAN vol. 012, no. 391 (C-537), 18 October 1988 (1988-10-18) & JP 63 139134 A (KYOWA HAKKO KOGYO CO LTD), 10 June 1988 (1988-06-10)

## Description

This application claims the benefit of U.S. patent application No. 10/987,217, filed November 11, 2004.

### FIELD OF THE INVENTION

This invention relates to protein tyrosine phosphatase inhibitors, and the use of protein tyrosine phosphatase inhibitors in combination with T cell activators to treat diseases.

### BACKGROUND OF THE INVENTION

Various publications or patents are referred to in parentheses throughout this application to describe the state of the art to which the invention pertains.

Intracellular protein tyrosine phosphorylation is regulated by extracellular stimuli, such as that provided by cytokines. This regulation acts to control cell growth, differentiation and functional activities. Literally hundreds of protein tyrosine phosphatases ("PTPases") are known including SHP-1, PTB1B, MKP1, PRL-1, PRL-2, and PRL-3. The signaling mechanism that regulates intracellular protein tyrosine phosphorylation depends on the interplay of protein tyrosine kinases ("PTK") (which initiate signaling cascades through phosphorylating tyrosine residues in protein substrates) and protein tyrosine phosphatases (which terminate signaling via substrate dephosphorylation). Chemical compounds that modulate the activity of protein tyrosine kinases or phosphatases can induce cellular changes through affecting the balance of intracellular protein tyrosine phosphorylation and redirecting signaling. This is well illustrated by the successful treatment of human chronic myelogenous leukemia and gastrointentinal stromal tumors with PTK inhibitor STI-571 (Berman et al., Hum. Pathol. 32, 578 (2001); Druker, et al., N. Engl. J. Med. 344, 1031 (2001); Mauro et al., Curr. Opin. Oncol. 13,3 (2001)). STI-571 targets bcr/abl or c-kit which are aberrantly activated protein kinases that play a key pathogenic molecule in the diseases.

Acute myeloid leukemia ("AML") is characterized by the accumulation of myeloid blast cells that are arrested at various differentiation stages and unable to terminally differentiate. Based on morphology, cytochemistry, immunological markers and cytogenetics, AML can be divided into distinct subclasses according to the French-American-British (FAB) classification. Treatment for most subclasses of AML is unsatisfactory. Treatment usually includes intensive chemotherapy administered as induction treatment to induce complete hematological remission and consolidation therapy to eradicate residual disease. Consolidation therapy with chemotherapy alone or in combination with autologous stem cell transplantation is associated with a relatively high risk of relapse and a long-term disease-free survival of less than 50%. Consolidation therapy with allotransplantation has a lower relapse risk but a higher treatment-related mortality (Lowenberg et al., N. Eng. J. Med. 341, 1051 (1999) ("Lowenberg")).

The potential of differentiation induction therapy in AML treatment is highlighted by the recent success of all-trans retinoic acid (ATRA) in the treatment of acute promyelocytic leukemia (APL, M3 subclass) (Kogan et al., Oncogene 18, 5261 (1999) ("Kogan")). ATRA has been shown to induce complete remission and increased long term APL-free survival exceeding 75% (Fenaux et al., Blood 94, 1192 (1999)). This therapeutic effect of ATRA derives from its activity in inducing terminal differentiation of APL cells through its binding to aberrantly generated chimeric proteins of retinoic acid receptor a (RAR-alpha) that results in degradation of the chimeric proteins and altered transcription regulation (Kogan). As generation of chimeric proteins of RAR-alpha is restricted to APL cells, differentiation induction therapy with ATRA showed only limited benefit in the treatment of other AML subclasses (Lowenberg). Therapeutic use of ATRA is compromised by serious systemic toxicity (Tallman et al., Blood 95, 90 (1999)) and induced ATRA resistance (Melnick et al., Blood 93, 3167 (1999)). Nevertheless, the marked success of ATRA in the subgroup of APL cases has provided evidence indicating the efficacy of differentiation induction therapy in AML treatment and prompted extensive efforts to identify other differentiation induction therapeutics. Several candidates were reported recently, including arsenic derivatives and histone deacetylase inhibitors (He et al., Oncogene 18, 5278 (1999)).

Several lines of evidence have indicated that AML cell differentiation is affected by cellular protein tyrosine phosphorylation regulated by the balance ofPTKs and PTPases. Granulocytic maturation of HL-60 promyelocytic leukemia cells was shown to produce a decrease in cellular protein tyrosine phosphorylation and increases in both tyrosine kinase and protein phosphotyrosine phosphatase activities (Frank et al., Cancer Res. 48 (1988)). Hematopoietic protein tyrosine phosphatase (HePTP) amplification and overexpression were found in AML cells and cell lines and may contribute to abnormal AML cell growth and arrest of differentiation (Zanke et al., Leukemia 8, 236 (1994)). The involvement of hematopoietic cell phosphatase SHP-1 was indicated by its increased expression during HL-60 cell differentiation (Zhao et al., Proc. Nat. Acad. Sci USA 91, 5007 (1994)) and its inhibition of Epo-induced differentiation of J2E leukemic cells (Bittorf et al., Biol. Chem. 380, 1201 (1999)). Interestingly, PTK inhibitor STI-571 was shown to enhance ATRA-induced differentiation of APL cells although it alone had no differentiation induction activity (Berman et al., Rev. Infect Dis. 10, 560 (1988)).

Over-expression of PRL family tyrosine phosphatases (e.g., PRL-1, PRL-2 and PRL-3) plays a potentially pathogenic role in human malignancies. PRL-1 (phosphatase of regenerating liver-1) was initially identified as one of the genes expressed during liver regeneration (Diamond, et al., Mol. Cell. Biol. 14, 3752 (1994) ("Diamond")). PRL-2 and PRL-3 were found based their homology to PRL-1 (Montagna, et al., Hum. Genet. 96, 532 (1995); Zeng, et al., Biochem. Biophys. Res. Commun. 244,421 (1998) ("Zeng-1998")). PRLs are closely related phosphatases with at least 75% amino acid sequence similarity (Zeng-1998), In normal adult tissues, PRLs are expressed predominantly in skeletal muscle with lower expression levels detectable in brain (PRL-1), liver (PRL-2) and heart (PRL-3) (Diamond; Zeng-1998). Physiologic functions of the PRLs are unclear at present although involvement of PRL-1 in proliferation was suggested by its increased expression in regenerating liver (Diamond).

A potential role in maintenance of differentiating epithelial tissues was proposed based on their selective expression in terminally differentiate cells in kidney and lung (PRL-1) (Kong, et al., Am. J. Physiol. Gastrointest. Liver Physiol. 279, G613 (2001)) as well as mouse intestine (PRL-3) (Zeng, et al., J. Biol. Chem. 275, 21444 (2000)). Over-expression of PRL-3, resulting from gene amplification or other defects, was found to associate with tumor metastasis of human colorectal cancer in a recent studies (Saha, et al., Science 294, 1343 (2001) ("Saha")). Potential involvement of PRL-3 over-expression in other human malignancies is indicated by the localization of PRL-3 gene at human chromosome 8q, extra copies of this region were often found in the advanced stages of many different tumor types (Saha). Consistent with an oncogenic role of PRL over-expression in cancer, ectopic expression of PRL PTPases has been found to enhance cell growth, cause cell transformation and/or promote tumor growth in nude mice (Cates, et al., Cancer Lett. 110, 49 (1996); Diamond). Although PRL PTPases could be inhibited by sodium orthovanadate (Diamond, Matter, et al., Biochem. Biophys. Res. Commun. 283, 1061 (2001)), which broadly inhibits all phosphatases (Burke et al., Biopolymers 47, 225 (1998)), clinically usable inhibitors of PRLs have not been reported. The oncogenic mechanism and regulated signaling events/molecules of the phosphatases remains undefined.

Cancers and other diseases including immune deficiency, hepatitis B, and hepatitis are often treated with cytokines. Renal cell carcinoma (RCC), for instance, is a malignant disease with approximately 31,200 new cases and 12,000 deaths each year in the USA (Greenlee, R. T., M. B. Hill-Harmon, T. Murray, and M. Thun. 2001. Cancer statistics, 2001. Ca Cancer J Clin 51:15). A large proportion of RCC patients have initially, or develop following treatment of localized carcinoma, advanced disease that is poorly responsive to conventional treatments, including chemotherapy and radiation therapy (Mulders, P., R. Figlin, J. B. deKemion, R. Wiltrout, M. Linehan, D. Parkinson,W. deWolf, and A. Belldegrun. 1997. Renal cell carcinoma: recent progress andfuture directions. Cancer Res 57:5189). These patients have a median survival rate of only 8 months and a 5-year survival rate of less than 10% (Motzer, R. J., and P. Russo. 2000. Systemic therapy for renal cell carcinoma. J Urol 163:408). Immunotherapy, based on activation of anti-tumor immunity using cytokines or immune cells, has been investigated as an alternate systemic approach for the treatment of advanced RCC (Rosenberg, S. A. 2001. Progress in human tumour immunology and immunotherapy. Nature 411:380). Surprisingly, interleukin-2 (IL-2) was shown to induce response rates of 10-20% in advanced RCC patients and has been approved for RCC treatment (Bleumer, I., E. Oosterwijk, P. De Mulder, and P. F. Mulders. 2003. Immunotherapy for renal cell carcinoma. Eur Urol 44:65).

Several cytokines that induce signaling of the janus family kinase/signal transducer and activator of transcription (Jak/Stat) pathways (Stark et al., Harvey Lect. 93, 1 (1997)) have been approved for use clinical use in a number of diseases (D. J. Vestal et al., Pharmacology of Interferons: Induced Protein Cell Activation and Antitumor Activity, In Cancer Chemotherapy Biotherapy (3rd ed. 2001) ("Vestal"). Interferons (IFNs) are one example of cytokines that signal along the Jak/Stat pathway that have been approved for clinical use (Vestal). IFN-alpha is one example of a cytokine beneficial in treating human malignancies, including melanoma (Borden et al., Semin. Cancer Biol. 10, 125 (2000)). However, the clinical efficacy of IFN-alpha is often limited by resistance of cancer cells to the cytokine. Drugs that target IFN-alpha signaling molecules might augment IFN-alpha anticancer activity to overcome resistance, but none have been reported thus far. And, in a broader sense, any cytokine to which cancer cells may develop a resistance could benefit from drugs that target the signaling molecules involved in the resistance.

IL-2 is an activator of T lymphocytes and a number of other immune cells (Rosenberg, S. A. 2000. Interleukin-2 and the development of immunotherapy for the treatment of patients with cancer. Cancer J Sci Am 2000:S2). It binds to its receptor on the cell surface to trigger an intracellular signaling cascade that is down-regulated by several mechanisms, including dephosphorylation of IL-2 signaling molecules by protein tyrosine phosphatases (PTPases) (Rosenberg, S. A. 2000. Interleukin-2 and the development of immunotherapy for the treatment of patients with cancer. Cancer J Sci Am 2000:S2; Ellery, J. M., S. J. Kempshall, and P. J. Nicholls. 2000. Activation of the interleukin 2 receptor: a possible role for tyrosine phosphatases. Cell Signal 12:367). The biological effects mediated by IL-2 include the proliferation and clonal expansion of T-cells, natural killer cells (NK) and B cells. (Abbas et al., Cellular and Molecular Immunology, 4th Ed., Saunders 2000, p. 255). IL-2 stimulates the synthesis of IFN-γ in peripheral leukocytes and also induces the secretion of tumoricidal cytokines, such as the tumor necrosis factors. While IL-2 therapy has been shown effective against a number of cancers refractory to conventional treatments, its clinical usefulness is limited by its dose-related toxicity. High dose IL-2 therapy is associated with vascular leak, shock, pulmonary edema and systemic hypotension. It would thus be highly desirable to reduce IL-2 toxicity and to potentiate its therapeutic efficacy.

### SUMMARY OF THE INVENTION

The invention relates to protein tyrosine phosphatase ("PTPase") inhibitors, and the use of PTPase inhibitors in combination with T-cell activators to treat cancer. Subjects that may be treated include, but are not limited to, animals, which includes mammals, which in turn includes humans. Classes of compounds that were identified as potent PTPase inhibitors include, but are not limited to, the following: pentavalent antimonial compounds, imidazole compounds, and diamidine compounds.

One embodiment of the invention provides a therapeutic composition for treating cancer comprising sodium stibogluconate as a PTPase inhibitor and JL-2 as a T-cell activator. A T cell activator is any agent effective in causing, either directly or indirectly, T cells to execute their effector functions, including the induction of tumor-infiltrating macrophages. T cell activators and T cell effector functions are well known in the art and are described in Abbas et al., Cellular and Molecular Immunology, 4th Ed. 2000, and in Janeway et al., Immunobiology, 5th Ed., 2001. A T cell activator may be a protein, peptide, or organic or inorganic molecule. For example, bisphosphonates and phosphoantigens are well known in the art to be potent T cell activators. The T cell activator is JL-2 and by another unclaimed embodiment functional variants of JL-2. As used herein, a "functional variant" or "variant" of a peptide T cell activator is a peptide which contains one or more modifications to the primary amino acid sequence of a T cell activator peptide while retaining the immunostimulatory effect of the parental protein or peptide T cell activator. If a functional variant of a T cell activator peptide involves an amino acid substitution, conservative amino acid substitutions typically will be preferred, i.e., substitutions which retain a property of the original amino acid such as charge, hydrophobicity, conformation, etc. Examples of conservative substitutions of amino acids include substitutions made among amino acids within the following groups: (1) M, I, L, V; (2) F, Y, W; (3) K, R, H; (4) A, G; (5) S, T; (6) Q, N; and (7) E, D. Stimulation of T cells by the variant peptide T cell activator indicates that the variant peptide is a functional variant.

Accordingly a therapeutic composition for treating cancer comprising sodium stibogluconate and JL-2 as a T-cell activator is disclosed. The sodium stibogluconate may further be separated into fractions of different molecular weight, and some fractions may be discarded.

A therapeutic composition for treating cancer comprising sodium stibogluconate as a PTPase inhibitor and IL-2, is provided. The use of sodium stibogluconate as a PTPase inhibitor along with IL-2 has been surprisingly and unexpectedly discovered to not only potentiate the effectiveness of IL-2, but to also significantly reduce its toxicity. In the composition used other PTPase inhibitor may be selected from the following classes of compounds other pentavalent antimonial compounds, imidazole compounds, or diamidine compounds. The PTPase inhibitor may be a biological equivalent of any of the compounds known to exist in these classes or discovered in the future. The therapeutic composition may comprise mixtures or combinations of those compounds.

Accordingly to the invention a therapeutic composition for treating cancer comprising sodium stibogluconate and IL-2 is provided.

Another embodiment of the invention provides a therapeutic composition for treating cancer under the conditions expressed in the previous embodiments comprising a compound that has been fractionated. When a compound used as a therapeutic composition comprises a mixture of different compounds, the mixture may be fractionated and one or more fractions may be eliminated. One or more fractions may then be used to prepare a therapeutic composition.

Another embodiment of the invention provides a composition for reducing the toxicity of IL-2, comprising a PTPase inhibitor and IL-2, wherein the PTPase inhibitor is sodium stibogluconate. In another inventive embodiment, the PTPase inhibitor is one or more fractions of sodium stibogluconate.

Another embodiment of the invention provides the use of sodium stibogluconate and JL-2 for the preparation of a pharmaceutical composition for treating cancer

Another embodiment of the invention provides the use of sodium stibogluconate and JL-2 for the preparation of a pharmaceutical composition for reducing the toxicity of IL-2.
In one embodiment, the use comprises administering sodium stibogluconate and IL-2 sequentially. In another embodiment, sodium stibogluconate and IL-2 are administered simultaneously.

Another embodiment of the invention provides the use of sodium stibogluconate and JL-2 for the preparation of a pharmaceutical composition for potentiating the therapeutic efficacy of IL-2.
In one embodiment, the use comprises administering sodium stibogluconate and IL-2 sequentially. In another embodiment, sodium stibogluconate and IL-2 are administered simultaneously.

Another embodiment of the invention provides the use of sodium stibogluconate for the preparation of a pharmaceutical composition for potentiating the therapeutic efficacy of IL-2, in a subject undergoing IL-2 treatment.

Another embodiment of the invention provides the use of sodium stibogluconate or sodium stibogluconate and JL-2 for the preparation of a pharmaceutical composition for treating a disease under the conditions expressed in the previous use embodiments comprising fractionating the administered compound or compounds. When a compound used for the preparation of a pharmaceutical composition comprises a mixture of different compounds, the mixture may be fractionated and one or more fractions may be eliminated.

### BRIEF DESCRIPTION OF THE FIGURES

FIG. 1. The hypothetical structures for sodium stibogluconate (A) and meglumine antimonate (B).

FIG. 2. The hypothetical structures for ketoconazole (A), levamisole (B), and pentamidine (C).

FIG. 3. A. Relative PTPase activities of GST fusion proteins of SHP-1, SHP-2, and PTP1B in the presence of various amounts of sodium stibogluconate (SS). B. Relative PTPase activities of GST/SHP-1 fusion protein in the presence of various amounts of sodium stibogluconate or suramine. C. Relative PTPase activities of GST fusion proteins of PTP1B and MKP1 in the presence of various amounts of sodium stibogluconate.

FIG. 4. A. Protein domain structure of GST fusion proteins of SHP-1 and SHP-1 catalytic domain (SHP-1cata). B. Relative PTPase activities of fusion proteins of SHP-1 and SHP-1 cata in the presence of various amounts of sodium stibogluconate (SS).

FIG. 5. Relative PTPase activities of GST fusion protein of SHP-1 preincubated with sodium stibogluconate (SS) or Suramin and then washed (+) or not washed.

FIG. 6. SDS-PAGE gel of total cell lysate of Baf3 cells deprived of IL-3 for 16 hours and then incubated with sodium stibogluconate (SS) (A) or pervandate (B) for various times.

FIG. 7. SDS-PAGE gel of total cell lysate of Baf3 cells showing that sodium stibogluconate (SS) augments IL-3 induced Jak2/Stat5 tyrosine phosphorylation in Baf3 cells.

FIG. 8. A. Sodium stibogluconate (SS) augments the proliferation of Baf3 cells cultured in the presence of IL-3. B. Cell numbers for Baf3 cells cultured for three days with various amounts of IL-3 and in the presence or absence of sodium stibogluconate.

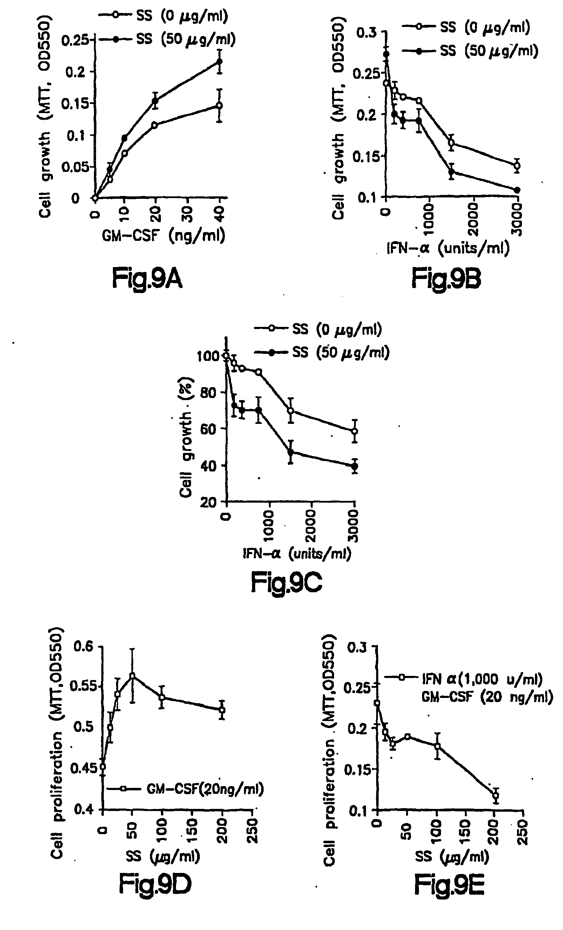

FIG. 9. A. Proliferation of TF-1 cells cultured in the presence of various amounts of GM-CSF and with or without sodium stibogluconate (SS) for three days. B. Proliferation of TF-1 cells cultured in the presence of GM-CSF and various amounts of IFN-alpha with or without sodium stibogluconate for three days. C. The results of B expressed as percent inhibition of cell growth. D. Proliferation of TF-1 cells cultured in the presence of GM-CSF and various amounts of sodium stibogluconate for six days. E. Proliferation of TF-1 cells cultured in the presence of GM-CSF/IFN-alpha and various amounts sodium stibogluconate for six days.

FIG. 10. A. Relative PTPase activities of GST fusion proteins of SHP-1, PTP1B and MKP1 in the presence of various amounts of sodium stibogluconate (SS) or potassium antimonyl tartrate (PSbT). B. SDS-PAGE gel of total cell lysate of Baf3 cells stimulated with IL-3 for various times in the absence or presence of sodium stibogluconate or potassium antimonyl tartrate. C. Proliferation of Baf3 cells cultured in the presence of IL-3 (10 unites/ml) and various amounts of sodium stibogluconate or potassium antimonyl tartrate for three days.

FIG. 11. A. Percentage of NBT-positive cells in NB4 cell culture after exposure to sodium stibogluconate (SS) for 3 and 6 days. B. Percentage of NBT-positive cells in NB4 cell culture after exposure to all-trans retinoic acid (ATRA) or sodium stibogluconate for up to six days. C. Percentage of CD11b-positive cells in NB4 cells cultured in the presence of all-trans retinoic acid or sodium stibogluconate for three days.

FIG. 12. A. Percentage of growth inhibition for NB4, HL-60, and U937 cells cultured for six days in varying amounts of sodium stibogluconate (SS). B. Percentage of NB4 cells at G0/G1, S, or G2/M phases after culture with no additive or in the presence of sodium stibogluconate or all-trans retinoic acid (ATRA). C. Flow cytometry plots for NB4 cells cultured for three days with no additive or in the presence of sodium stibogluconate or all-trans retinoic acid (X-axis shows staining with Annexin V FITC, Y-axis shows staining with propium iodide).

FIG. 13. A. Percentage ofNBT-positive NB4 cells cultured in the presence or absence of sodium stibogluconate (SS) or all-trans retinoic acid (ATRA) for six days then washed and cultured for an additional six days. B. Percentage of NBT-positive NB4 cells cultured in the presence or absence of sodium stibogluconate or all-trans retinoic acid for 0.5 to 24 hours then washed and cultured for an additional six days.

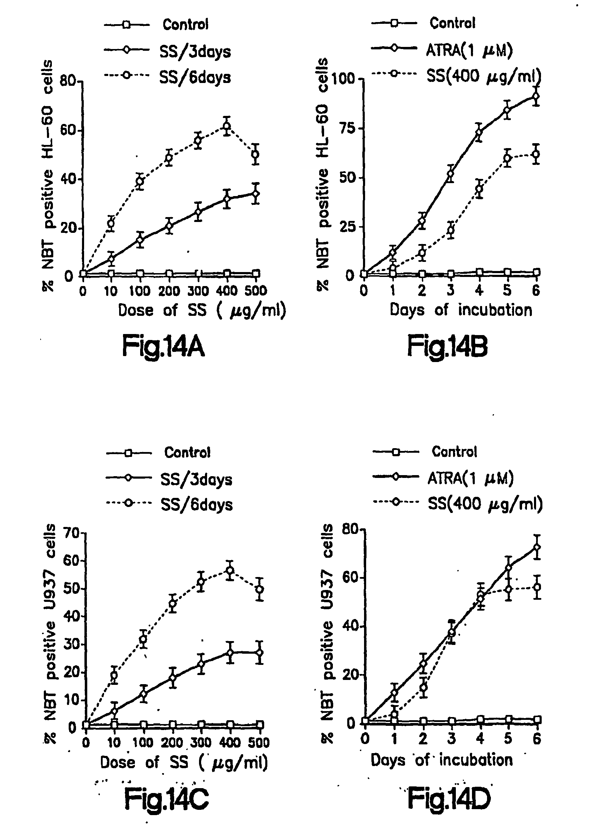

FIG. 14. A. Percentage of NBT-positive cells in HL-60 cells cultured in the absence or presence of various amounts of sodium stibogluconate (SS) for 3 or 6 days. B. Percentage of NBT-positive cells in U937 cells cultured in the absence or presence of various amounts of sodium stibogluconate (SS) for 3 or 6 days. C. Percentage of NBT-positive cells in HL-60 cultured in the presence or absence of all-trans retinoic acid (ATRA) or sodium stibogluconate for 0-6 days. D. Percentage ofNBT-positive cells in U937 cultured in the presence or absence of all-trans retinoic acid or sodium stibogluconate for 0-6 days.

FIG. 15. Percentage of NBT-positive HL-60 (A) and U937 (B) cells cultured in the absence or presence of granulocyte/macrophage colony stimulating factor (GM-CSF), sodium stibogluconate (SS), or both for varying time.

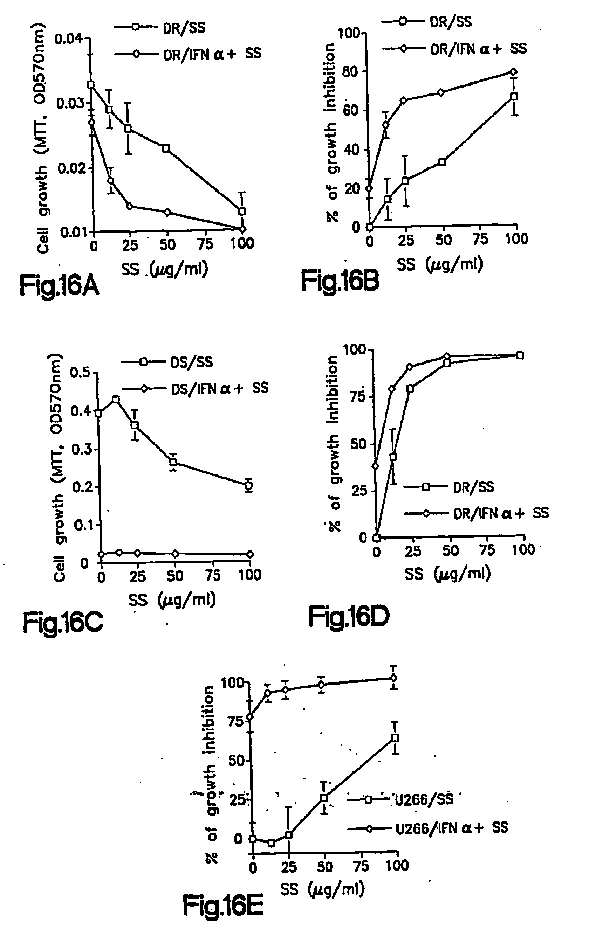

FIG. 16. A. Cell growth to DR cells cultured in the absence or presence of various amounts of sodium stibogluconate (SS) and/or IFN-alpha for three days. B. Percentage of growth inhibition of DR cells calculated from data presented in A. C. Cell growth to DS cells cultured in the absence or presence of various amounts of sodium stibogluconate and/or IFN-alpha for three days. D. Percentage of growth inhibition of DR cells in the absence or presence of various amounts of sodium stibogluconate and/or IFN-alpha for six days. E. Percentage of growth inhibition of U266 cells by IFN-alpha and various amounts of sodium stibogluconate in day six cultures.

FIG. 17. Percentage of growth inhibition of WM9 (A), DU145 (B), MDA231 (C) and WiT49-N1 (D) in the absence or presence of various amounts of sodium stibogluconate (SS) and/or IFN-alpha in day 6 cultures.

FIG. 18. Percentage of growth inhibition of WM9 cells in the absence or presence of various amounts of SS, IFN-alpha and IFN-beta in day 6 cultures.

FIG. 19. Percentage of control growth plots demonstrating the synergy between sodium stibogluconate (SS) and IFN-alpha (A) or IFN-beta (B) in WM9 cells.

FIG. 20. Flow cytometry plots for U266 cells cultured for three days in the absence (A) or the presence of IFN-alpha (B), sodium stibogluconate (SS) (C), or both (D) (X-axis shows staining with Annexin. V FITC, Y-axis shows staining with propium iodide).

FIG. 21. A. SDS-PAGE gel of total cell lysate of DR cells stimulated by IFN-alpha for various time points in the absence or presence of sodium stibogluconate (SS). B. SDS-PAGE gel of total cell lysate of human cancer cell lines WM9, WM35, WiT49-N1, and DU145 stimulated by IFN-alpha for five hours in the absence or presence of sodium stibogluconate.

FIG. 22. Effect of sodium stibogluconate, IFN-alpha, or both on tumor volume in WM9 and DU145 tumors in nude mice over time.

FIG. 23. Comparison of body weights of nude mice bearing WM9 xenographs and a control group.

Fig 24. Differential growth responses of Renca and WM9 cells to SSG in vitro. Renca (A) and WM9 (B) cells were cultured in the absence or presence of various amounts of SSG for 6 days. Viable cells were then quantified by MTT assays. Data represent mean + s.d. of triplicate samples.

Fig 25. SSG and SSG/IL-2 combination treatments inhibit Renca tumor growth in Balb/c mice. Renca cells were inoculated (106 cells/site, s.c.) into Balb/c mice. Mice with 4-day established Renca tumors were then untreated (Control) or treated with IL-2 (105 IU/daily, i.p.), SSG (12 mg/daily, i.m.) or the combination of the two agents. Renca tumor volumes (mean + s.d., n = 8) in these mice were recorded as indicated. The treatment durations of the agents are indicated by the arrows.

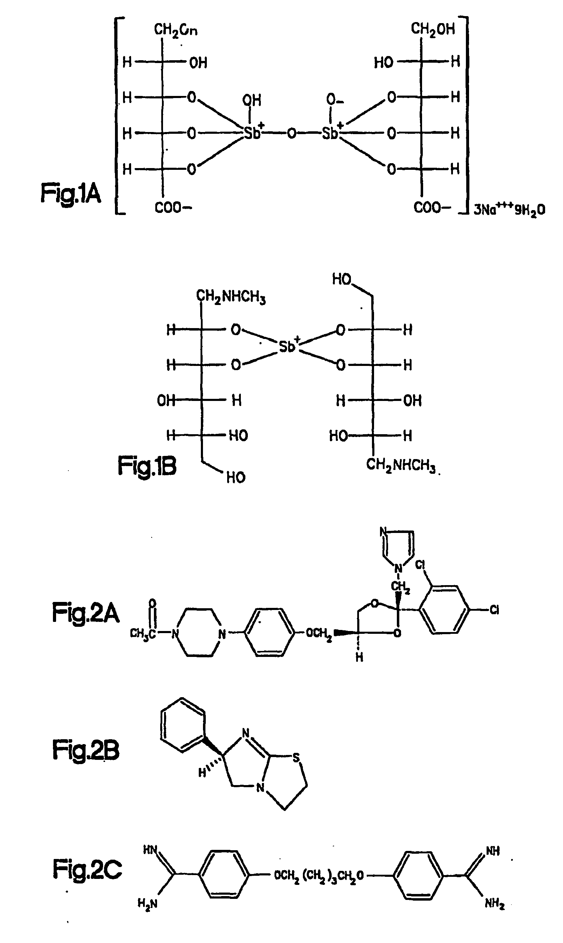

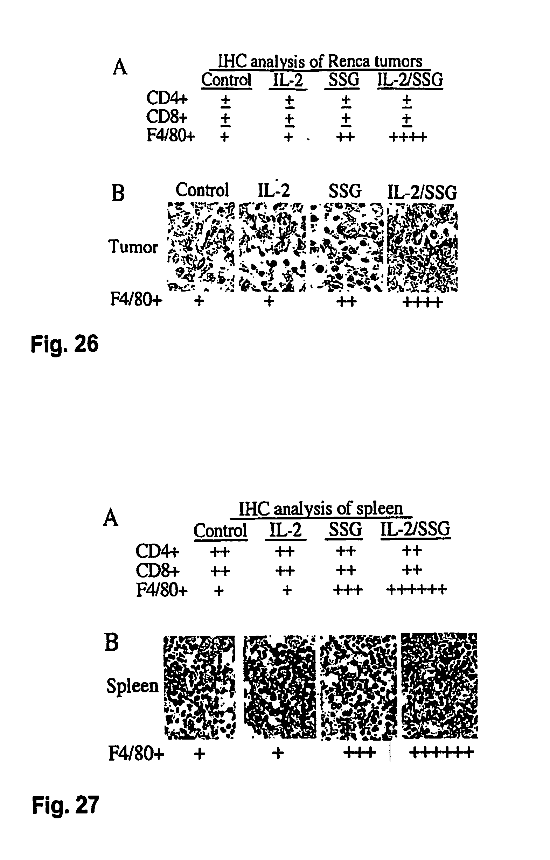

Fig 26. SSG and SSG/IL-2 combination treatments increase Renca tumor-infiltrating Mϕ in Balb/c mice. A, Relative numbers of T lymphoid cells and Mϕ in Renca tumors from the differentially treated Balb/c mice (Fig 2) as quantified by immunohistochemistry. Tissue sections of tumors harvested from the mice at the end of the treatments were stained by anti-CD4, anti-CD8 or anti-F4/80 mAb. The CD4+, CD8+ and F4/80+ cells in the tumors from the treated mice were scored (fold increase) by comparing to the basal levels in the tumors of the control mice. B, Representative views (40 x) of F4/80 + cells in Renca tumor sections from the differentially treated mice.

Fig 27. SSG and SSG/IL-2 combination treatments increase spleen Mϕ in Balb/c mice. A, Relative numbers of T cells and Mϕ in Spleen from the differentially treated Balb/c mice (Fig 2) as quantified by immunohistochemistry. Tissue sections of spleen harvested from the mice at the end of the treatments were stained by anti-CD4, anti-CD8 or anti-F4/80 mAb. The CD4+, CD8+ and F4/80+ cells in the spleen from the treated mice were scored (folds) by comparing to the basal levels in the spleen of the control mice. B, Representative views (20 x) of F4/80+ cells in spleen from the differentially treated mice.

Fig 28. SSG augments IFN-gamma secretion by Jurkat cells in vitro. Jurkat cells were cultured in the absence or presence of various amounts of SSG for 16 hrs. The amounts of IFN-gamma in culture supernatants of Jurkat T cells were quantified by ELISA. Data represent mean + s.d. of triplicate samples.

Fig 29. Effects of IL-2/SSG combination treatment on Renca tumor growth in athymic Balb/c mice. Renca cells were inoculated (106 cells/site, s.c.) into athymic Balb/c mice (nu/nu). The mice with 4-day established Renca tumors were then untreated (Control) or treated with the combination of IL-2 (105 IU/daily, i.p.) and SSG (12 mg/daily, i.m.). Renca tumor volumes (mean + s.d., n = 8) in these mice were recorded as indicated. The treatment durations of the agents are indicated by the arrows.

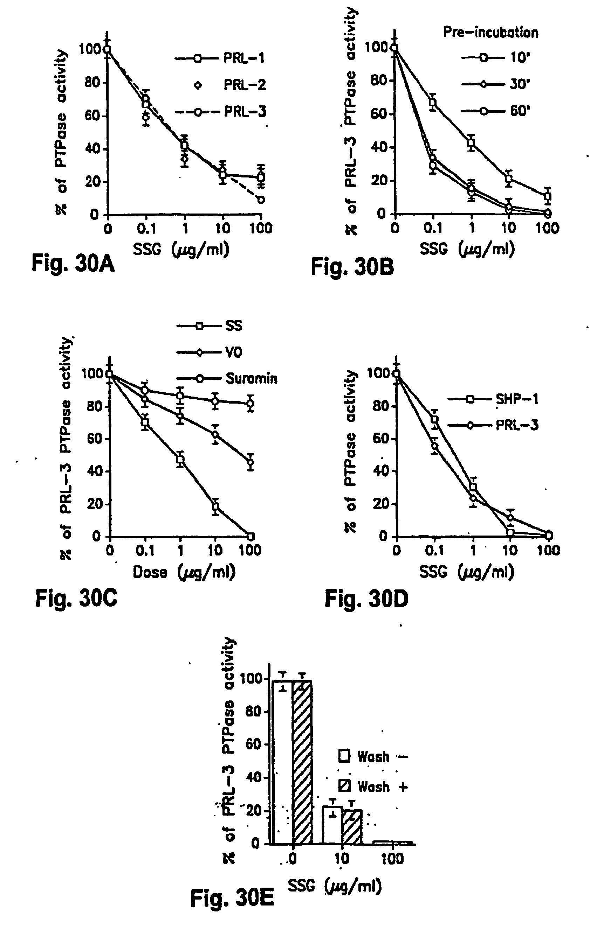

FIG. 30. A. Relative activities of recombinant PRL phosphatases in dephosphorylating a synthetic phosphotyrosine peptide in vitro in the presence or absence of sodium stibogluconate. B. Effects of differential pre-incubation times of sodium stibogluconate with recombinant PRL-3 on PRL-3 activity in dephosphorylating the peptide substrate. C. Relative activities of recombinant PRL-3 in dephosphorylating DiFMUP substrate in the absence or presence of various amounts of SSG, sodium orthovanandate (VO) or suramin. D. Relative activities of recombinant SHP-1 and PRL-3 in dephosphorylating DiFMUP in the absence or presence of SS. E. Relative phosphatase activities of PRL-3 bound to glutathione beads, pre-incubated with SSG for 10 minutes and then subjected no washing (Wash -) or a washing process (Wash +).

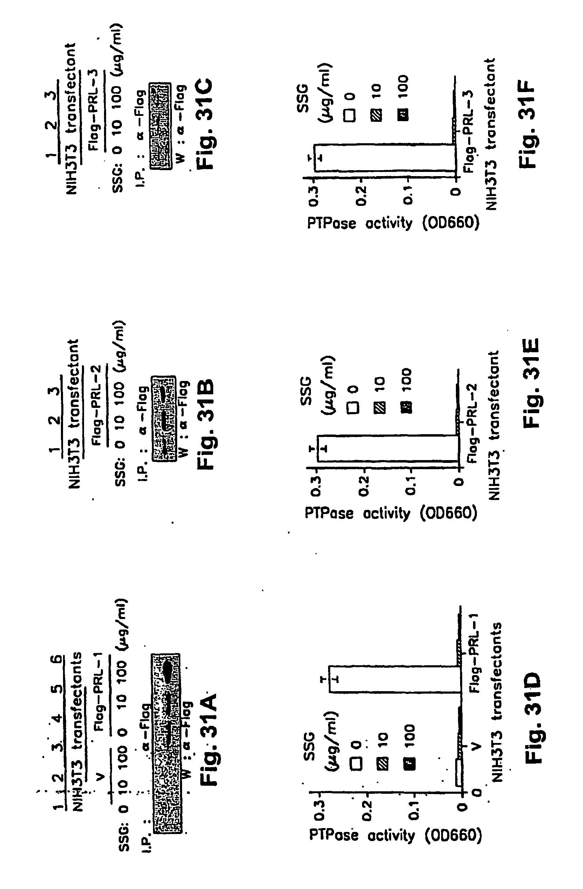

FIG. 31. A. PTPase activities of anti-Flag immunocomplexes from untreated (0) or sodium stibogluconate (SSG) treated (5 min) NIH3T3 transfectants of the control vector (V) or Flag-PRL-1 expression construct in in vitro PTPase assays. B. Relative amounts of Flag-PRL-1 in the immunocomplexes as detected by SDS-PAGE/Western blotting. C. PTPase activities of anti-Flag immunocomplexes from untreated or sodium stibogluconate-treated NIH3T3 transfectants of Flag-PRL-2. D. Relative amounts of Flag-PRL-2 in the immunocomplexes as determined by SDS-PAGE/Western blotting. E. PTPase activities of anti-Flag immunocomplexes from untreated or sodium stibogluconate-treated NIH3T3 transfectants of Flag-PRL-3. F. Relative amounts of Flag-PRL-3 in the immunocomplexes as determined by SDS-PAGE/Western blotting.

FIG. 32. A. Relative PTPase activity of anti-Flag immunocomplexes from Flag-PRL-2 transfectants untreated or treated with sodium stibogluconate (SSG) for 5 min, washed to remove cell-free drug, and then incubated for various times. B. Relative amounts of Flag-PRL-2 in the immunocomplexes as determined by SDS-PAGE/Western blotting.

FIG. 33. Expression of transcripts of PRLs in a panel of human cancer cell lines (A549, HEY, LoVo, Sk-N-SH, and DU145) and in PBMG from a healthy volunteer as determined by RT-PCR.

FIG. 34. Growth of human cancer cell lines A549 (A), HEY (B), LOVO (C), SK-N-SH (D), U251 (E) and DU145 (F) in day 6 culture in the absence or presence of SSG.

FIG. 35. A. Tumor volumes in mice inoculated with DU145 cells 2 days prior to subjecting to no treatment (Control) or treatment with sodium stibogluconate (SSG). B. Histology of DU145 cell inoculation site in control mice on day 25. C. Histology of DU145 cell inoculation site in SSG-treated mice on day 25. (DU145 tumors are indicated by arrows.)

FIG. 36. A. Growth of DU145 and DU145R cells in day 6 culture in the absence or presence of sodium stibogluconate (SSG). B. Sequences of PRL-1 cDNAs (around codon 86) from DU145 or DU145R cells. C. Position of S86 and R86 in PRL-1 protein. D. Activities of GST fusion proteins of PRL-1, PRL-1R86 (R86) and GST protein (control) in dephosphorylating a synthetic phosphotyrosine peptide substrate in PTPase assays in vitro. E. Relative PTPase activities of recombinant PRL-1 and PRL-1R86 (R86) in the absence or presence of sodium stibogluconate as determined by in vitro PTPase assays.

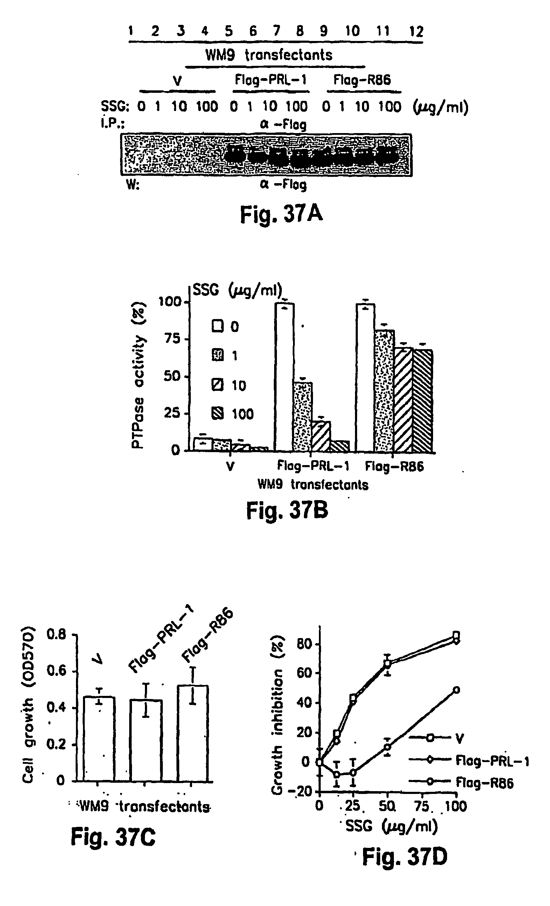

FIG. 37. A. SDS-PAGE/Western blotting analysis of anti-Flag immunocomplexes from untreated or sodium stibogluconate (SSG) treated WM9 cell transfectants of a control vector (V) or expression constructs of Flag-PRL-1 or Flag-PRL-1R86. B. Relative PTPase activities of the anti-Flag immunocomplexes as determined by in vitro PTPase assays (PTPase activity of the immunocomplex from untreated Flag-PRL-1 transfectant set as 100% value). C. Growth of the WM9 transfectants in the absence of sodium stibogluconate in day 6 culture. D. Relative growth inhibition of the WM9 transfectants in day 6 culture in the presence of various amounts of sodium stibogluconate.

FIG. 38. Relative SHP-1 and PRL-3 PTPase activity in the presence of meglumine antimonate in vitro.

FIG. 39. A. HPLC chromatograph of sodium stibogluconate separation showing fractions and Sb content in each fraction. B. Relative PTPase activity of recombinant SHP-1 in the presence of each sodium stibogluconate fraction.

FIG. 40. Relative PTPase activities of MKP (A), PTP1B (B), and GSTm8 (C) in the presence of levamisole, ketoconazole, and pentamidine with sodium stibogluconate (SS) serving as a model agent.

FIG. 41. Relative PTPase activities of SHP-1 (A), PTP1B (B), and NlKP1 (C) in the presence of ketokonazole and pentamidine with sodium stibogluconate (SS) serving as a model agent.

FIG. 42. A. Relative PTPase activities of PRL-1, PRL-2, and PRL-3 in the presence of varying amounts of pentamidine. B. Relative PTPase activities of PRL-1, PRL-2, and PRL-3 in the presence of varying amounts of ketoconazole. C. Relative PTPase activity of SHP-1 in the presence of pentamidine and ketoconazole.

FIG. 43. Percent growth inhibition of WM9 cells cultured in the presence of pentamidine (A) or ketoconazole (B) as single agents or in combination with IFN-alpha for 6 days.

### DETAILED DESCRIPTION OF THE INVENTION

As used herein, the following abbreviations have the following meanings:

"AML" is used herein to mean acute myeloid leukemia;

"ATRA" is used herein to mean All-trans-retinoic acid;

"GM-CSF" is used herein to mean granulocyte/macrophage colony stimulating factor;

"IFN-alpha" or "IFNα" are used herein to mean interferon-alpha;

"IFN-beta" or "IFNβ" are used herein to mean interferon-beta;

"IFN-gamma" or "IFNγ" are used herein to mean interferon-gamma;

"IL-2" is used herein to mean interleukin-2;

"IL-3" is used herein to mean interleukin-3;

"Jak2" is used herein to mean janus family kinase 2;

"Mϕ" is used herein to mean macrophage(s);

"NBT" is used herein to mean, nitroblue tetrazolium;

"PTPase" is used herein to mean protein tyrosine phosphatase;

"PTK" is used herein to mean protein tyrosine kinase;

"RCC" is used herein to mean renal cell carcinoma;

"SH2 is used herein to mean Src-homology 2 domain;

"SBP-1" is used herein to mean Src-homology protein tyrosine phosphatase;

"Stat1" is used herein to mean signal transducer and activator of transcription 1;

"Stat5" is used herein to mean signal transducer and activator of transcription 5;

"SS" and "SSG" is used herein to mean sodium stibogluconate.

"T cell activator" is used herein to mean a substance, molecule, or composition effective in eliciting the T cell effector functions disclosed herein, including the activation of tumor-infiltrating macrophages.

Disclosed herein are compositions and methods useful in inhibiting PTPase activity. The inventor has surprisingly and unexpectedly discovered that drugs effective in the treatment of leishmaniasis are potent protein tyrosine phosphatase inhibitors effective in the treatment of diseases associated with abnormally active protein tyrosine phosphatases, or otherwise implicating protein tyrosine phosphatase activity, such as cancer. Patients that are treated may include, but are not limited to, animals, which includes mammals, which in turn includes humans. The term leishmaniasis agent is used herein interchangeably with the phrase "compounds effective in the treatment of leishmaniasis." Classes of drugs effective in treating leishmaniasis include, but are not limited to, pentavalent antimonial compounds, imidazole compounds, and diamidine compounds. Moreover, pentavalent antimonial compounds, imidazole compounds, and diamidine compounds that are not leishmaniasis agents may be useful in inhibiting PTPase activity. A review of agents effective in treating leishmaniasis can be found at Steck, Prog. Drug Res. 18, 289 (1974). The term leishmaniasis agent or compound effective in treating leishmaniasis is intended to encompass drugs and compounds currently used to treat leishmaniasis either clinically and/or experimentally, as would be understood by one of ordinary skill in the art. Some specific examples of drugs effective in treating leishmaniasis include, but are not limited to the following compounds: allopurinol (e.g., Zyloric® from Glaxo Wellcome/Glaxo Smith Kline, Talol® from Saval, Zyloprim®), aminosidine (e.g., Gabbriomycin®), amphotericine/amphotericine B (e.g., Fungizone®, AmBisome®, Amphocin®, Amphocil®, Abelcet®), interferon (e.g., Actimmune®), itraconazole, ketokonazole (e.g., Nizoral®), levamisole (e.g., Ergamisol®), meglumine antimonate or glucantime (e.g., Glucantime®, Glucantim®), miltefosine (an alkylphospholipid), pammomycin (aminosidine) (e.g., Humatin®), pentamidine isothionate or isthionate pentamidine e.g., NebuPeat®, Pentacarinat®, Pentam®), pentamidine (e.g., Lomidine® from Rhone-Poulenc, May & Baker), sitamaquine/WR6026 (an 8-aminoquinoline), and sodium stibogluconate (e.g., Pentostam® from Glaxo Wellcome). The omission of any compound or compounds from the foregoing list should not be construed as an intention not to include such an agent within the scope of the term leishmaniasis agent. The term leishmaniasis agent is also intended to encompass drugs and compounds that have not yet been found to be effective in treating leishmaniasis, but may be found to be effective in the future.

Pentavalent antimonial compounds include, but are not limited to, compounds such as meglumine antimonate (glucantime), antimony dextran glucoside, antimony mannan, ethyl stibanine, urea stibamine, and sodium stibogluconate. Pentavalent antimonial compounds have been found to be potent PTPase inhibitors. Pentavalent antimonial compounds contain Sb(V). By way of example, sodium stibogluconate is a complex of Sb(V) and gluconic acid, and meglumine antimonate is a complex of Sb(V) and n-methyl-D-glucamine. The structures of sodium stibogluconate and meglumine antimonate have not been conclusively determined because these compositions often exist in polymeric forms. Hypothetical structures for sodium stibogluconate and meglumine antimonate are shown in FIGS. 1A and 1B respectively. Sodium stibogluconate has been used for decades in the treatment of leishmaniasis, a disease caused by the protozoa parasites residing in macrophages. While its pharmacological mechanism is poorly understood, there have been indications that the drug's therapeutic effect might be mediated via a cellular target(s): it kills intracellular leishmania but has no effect on the free living form (promastigotes) of the protozoa that lives in the intestine of sandflys and can grow in defined culture medium in vitro. Sodium stibogluconate is also known as sodium antimony gluconate, Stibanate, Dibanate, Stihek, Solustibostam, Solyusurmin, and Pentostam®. Methods for the synthesis of sodium stibogluconate are known by those of skill in the art.

A therapeutic composition for treating cancer comprising sodium stibogluconate as an anti-cancer agent and OL-2. An anti-cancer agent is an agent effective in the treatment of cancer. Other componuds of the composition may be other pentavalent antimonial compounds, imidazole compounds, or diamidine whereby compounds. The pentavalent antimonial compounds of the therapeutic composition may include, but are not limited to, meglumine antimonate, and biological equivalents of those compounds and imidazole compounds of the therapeutic composition may include, but are not limited to, ketoconazole, levamisole, and biological equivalents of those compounds and diamidine compound may be, but is not limited to, pentamidine and biological equivalents. The cancer that is treated may be, but is not limited to, lymphoma, multiple myeloma, leukemia, melanoma, prostate cancer, breast cancer, renal cancer, and bladder cancer. The therapeutic composition may be used to treat a patient with multiple cancers.

According to the invention a therapeutic composition for treating cancer comprising sodium stibogluconate and JL-2 is provided. The cancer that is treated may be, but is not limited to, lymphoma, multiple myeloma, leukemia, melanoma, prostate cancer, breast cancer, renal cancer, and bladder cancer. The therapeutic composition may be used to treat a patient with multiple cancers.

In addition, use of a therapeutic composition for treating a disease responsive to cytokine (JL-2) treatment comprising JL-2 as a cytokine and sodium stiboglukonate as a PTPase inhibitor. Many diseases including, but not limited to, an infectious disease, a disease associated with PTPase activity, immune deficiency, cancer, an infection, a viral infection, multiple sclerosis, hepatitis B, and hepatitis C are treated with cytokines. The use of a PTPase inhibitor along with a cytokine has been surprisingly and unexpectedly discovered to improve the effectiveness of the cytokine. PTPases may interfere with the operation of the co-administered cytokines rendering them ineffective. By inhibiting a PTPase that is interfering with the operation of a co-administered cytokine, the activity of a cytokine may be enhanced. In the composition used other PTPase inhibitor may be selected from the following classes of compounds. Other pentavalent antimonial compounds, imidazole compounds, or diamidine compounds. The PTPase inhibitor may be a biological equivalent of any of the compounds known to exist in these classes or discovered in the future, whereby the pentavalent antimonial compounds of the therapeutic composition may include, but are not limited to meglumine antimonate, and biological equivalents of those compounds and imidazole compounds of the therapeutic composition may include, but are not limited to, ketoconazole, levamisole, and biological equivalents of those compounds, and diamidine compound may be, but is not limited to, pentamidine and biological equivalents. The therapeutic composition may comprise mixtures or combinations of those compounds.

Use of a therapeutic composition for treating a disease responsive to JL-2 cytokine treatment comprising JL-2 as a cytokine and sodium stibogluconate as a leishmaniasis agent is provided. Many diseases including, but not limited to, an infectious disease, a disease associated with PTPase activity, immune deficiency, cancer, an infection, a viral infection, multiple sclerosis, hepatitis B, and hepatitis C are treated with cytokines. In the composition used other leishmaniasis agent may be, but is not limited to the following classes of compounds: other pentavalent antimonial compounds, imidazole compounds, or diamidine compounds. The leishmaniasis agent may be a biological equivalent of any compounds known to exist in these classes or discovered in the future, whereby leishmaniasis agents include, but are not limited to, allopurinol, aminosidine, amphotericine/amphotericine B, interferon, intraconazole, ketoconazole, levamisole, meglumine antimonate, miltefosine, paromomycin, pentamidine isothionate, pentamidine, sitamiquine/WR6026 and biological equivalents of those compounds. The therapeutic composition may comprise mixtures or combinations of those compounds.

Use of a therapeutic composition for treating a disease responsive to cytokine treatment comprising sodium stibogluconate and JL-2 as a cytokine is provided. The disease treated may include, but is not limited to, an infectious disease, a disease associated with PTPase activity, immune deficiency, cancer, an infection, a viral infection, multiple sclerosis, hepatitis B, and hepatitis C. The therapeutic composition may be used to treat a patient with multiple diseases.

Use of a therapeutic composition for treating cancer comprising administering to a patient an effective amount of sodium stibogluconate as an anti-cancer agent and JL-2 as is provided. In the composition used other anti-cancer agent may be selected from one of the following classes: other pentavalent antimonial compounds, imidazole compounds, or diamidine compounds. The anti-cancer agent may be a biological equivalent of any of the compounds known to exist in these classes or discovered in the future. The anti-cancer agent may comprise mixtures or combinations of those compounds, whereby the pentavalent antimonial compounds of the therapeutic composition may include, but are not limited meglumine antimonate, and biological equivalents of those compounds and imidazole compounds of the therapeutic composition may include, but are not limited to, ketoconazole, levamisole, and biological equivalents of those compounds and diamidine compound may be, but is not limited to, pentamidine and biological equivalents. The cancer that is treated may be, but is not limited to, lymphoma, multiple myeloma, leukemia, melanoma, prostate cancer, breast cancer, renal cancer, and bladder cancer. The method may be used to treat a patient with multiple cancers.

Use of a therapeutic composition for treating cancer comprising administering to a patient an effective amount of sodium stibogluconate as a leishmaniasis agent and JL-2 is provided. In the composition used other leishmaniasis agent may be selected from the following classes of compounds: other pentavalent antimonial compounds, imidazole compounds, or diamidine compounds, whereby leishmaniasis agents include, but are not limited to, allopurinol, aminosidine, amphotericine/amphotericine B, interferon, intraconazole, ketoconazole, levamisole, meglumine antimonate, miltefosine, paromomycin, pentamidine isothionate, pentamidine, sitamiquine/WR6026 and biological equivalents of those compounds. The cancer that is treated may be, but is not limited to, lymphoma, multiple myeloma, leukemia, melanoma, prostate cancer, breast cancer, renal cancer, and bladder cancer. The therapeutic composition may be used to treat a patient with multiple cancers. The therapeutic composition may comprise mixtures or combinations of leishmaniasis agents.

Another embodiment of the invention relates to the use of a therapeutic composition for treating cancer comprising administering to a patient an effective amount of sodium stibogluconate aud JL-2. The cancer that is treated may be, but is not limited to, lymphoma, multiple myeloma, leukemia, melanoma, prostate cancer, breast cancer, renal cancer, and bladder cancer. The method may be used to treat a patient with multiple cancers.

Another embodiment of the invention concerns the use of a therapeutic composition for treating a disease responsive to cytokine treatment comprising administering to a patient an effective amount of JL-2 as cytokine and sodium stibogluconate as a PTPase inhibitor. Diseases including, but not limited to, an infectious disease, a disease associated with PTPase activity, immune deficiency, cancer, an infection, a viral infection, multiple sclerosis, hepatitis B, and hepatitis C may be treated using this method. In the composition used other PTPase inhibitor may be selected from the following classes of compounds other pentavalent antimonial compounds, imidazole compounds, or diamidine compounds. The PTPase inhibitor may be a biological equivalent of any of the compounds known to exist in these classes or discovered in the future, whereby the pentavalent antimonial compounds of the therapeutic composition may include, but are not limited to meglumine antimonate, and biological equivalents of those compounds and the imidazole compounds of the therapeutic composition may include, but are not limited to, ketoconazole, levamisole, and biological equivalents of those compounds and the diamidine compound may be, but is not limited to, pentamidine and biological equivalents. The therapeutic composition may comprise mixtures or combinations of those compounds. Examples of cytokines include, but are not limited to, interferon-alpha, interferon-beta, interferon-gamma, and granulocyte/macrophage colony stimulating factor.

In addition, use of a therapeutic composition for treating a disease responsive to cytokine treatment comprising administering to a patient an effective amount of JL-2 as a cytokine and sodium stibogluconate as a leishmaniasis agent is provided. Diseases including, but not limited to, an infectious disease, a disease associated with PTPase activity, immune deficiency, cancer, an infection, a viral infection, multiple sclerosis, hepatitis B, and hepatitis C may be treated using this method. In the composition used the leishmaniasis agent may be, but is not limited to the following classes of compounds: othes pentavalent antimonial compounds, imidazole compounds, or diamidine compounds. The leishmaniasis agent may be a biological equivalent of any compounds known to exist in these classes or discovered in the future, whereby the leishmaniasis agents suitable for use by this use include, but are not limited to, allopurinol, aminosidine, amphotericine/amphotericine B, interferon, intraconazole, ketoconazole, levamisole, meglumine antimonate, miltefosine, paromomycin, pentamidine isothionate, pentamidine, sitamiquine/WR6026, and biological equivalents of those compounds. The therapeutic composition may comprise mixtures or combinations of those compounds.

According to the invention use of a therapeutic composition for treating a disease responsive to cytokine treatment comprising administering to a patient an effective amount of sodium stibogluconate JL-2, and as a cytokine is provided. The disease treated by this method may include, but is not limited to, an infectious disease, a disease associated with PTPase activity, immune deficiency, cancer, an infection, a viral infection, multiple sclerosis, hepatitis B, and hepatitis C. The therapeutic composition may be used to treat a patient with multiple diseases.

Another embodiment of the present invention relates to fractionating a compound comprising a mixture of compounds. In any of the above embodiments, if the compound provided or used as part of a therapeutic composition or use comprises a mixture of compounds, the mixture may be fractionated and one or more fractions may be eliminated. Compounds present in a mixture of compounds may comprise different molecular weight compounds (e.g., polymers), conformers, enantiomers, isomers, analogues, derivatives, unreacted precursors, alternative products, intermediates, or degradation products. As an example, sodium stibogluconate exists as a polymer of multiple species with molecular weights varying from 100 to 4,000 amu. Fractionation of a parent mixture of sodium stibogluconate by chromatography, or another suitable method, provides fractions with varying PTPase inhibitory activity. Elimination of fractions with relatively low or no PTPase inhibitory activity may increase the PTPase inhibitory activity of the overall solution. Further, toxicity associated with degradation or other products or components within a parent mixture may be reduced when fewer molecular species are present in the final mixture.

One unclaimed embodiment describes a method for treating a disease dependent upon substrate dephosphorylation comprising screening diseased cells for the presence of and mutations in PRL phosphatases. In some instances, simply determining that a certain type of phosphatase is present in a diseased cell may not provide enough information to select an effective phosphatase inhibitor. If a phosphatase was mutated, for example, resistance may be conferred on the mutated phosphatase against a particular phosphatase inhibitor that was very effective against the same type of non-mutated phosphatase. For example, if a cystein is substituted for an arginine at position 86 of PRL-1, the enzyme is significantly less resistant to inhibition by sodium stibogluconate. If a mutated phosphatase with conferred resistance is present in a diseased cell, knowledge of that mutation may be important in treating that disease. Thus, this embodiment of the invention provides a screening method for determining if a mutated PRL phosphatase is present in a diseased cell. One step comprises screening a sample of diseased cells to determine whether the cells contain PRL phosphatase. Another step comprises screening a PRL phosphatase for a mutation that confers resistance to PRL phosphatase inhibitors. Another step comprises administering to a patient a therapeutically effective amount of an inhibitor to the PRL phosphatase found in the cells. If the PRL phosphatase is found to be mutated, the PRL phosphatase inhibitor chosen to fight the disease may be different from the PRL phosphatase inhibitor that would be used for a non-mutated PRL phosphatase. These steps may-be performed in any order. A kit may be provided containing apparatus for performing the method of this embodiment. The kit apparatus may determine whether the sample contains a PRL phosphatase by methods known to one of skill in the art. The kit apparatus may determine whether the PRL phosphatase contains one or more mutations by methods known to one of skill in the art.

Another embodiment of the invention provides a therapeutic composition for treating cancer, comprising sodium stibogluconate as a PTPase inhibitor and JL-2 as a T cell activator. A T cell activator is any agent effective in causing, either directly or indirectly, T cells to execute their effector functions, including the induction of tumor-infiltrating macrophages. T cell activators and T cell effector functions are well known in the art and are described in Abbas et al., Cellular and Molecular Immunology, 4th Ed. 2000, and in Janeway et al., Immunobiology, 5th Ed., 2001. A T cell activator may be a protein, peptide, or organic or inorganic molecule. For example, bisphosphonates and phosphoantigens are well known in the art to be potent T cell activators. The T cell activator is JL-2, and by another unclaimed embodiment functional variants of JL-2 may be used. As used herein, a "functional variant" or "variant" of a peptide T cell activator is a peptide which contains one or more modifications to the primary amino acid sequence of a T cell activator peptide while retaining the immunostimulatory effect of the parental protein or peptide T cell activator. If a functional variant of a T cell activator peptide involves an amino acid substitution, conservative amino acid substitutions typically will be preferred, i.e., substitutions which retain a property of the original amino acid such as charge, hydrophobicity, conformation, etc. Examples of conservative substitutions of amino acids include substitutions made among amino acids within the following groups: (1) M, I, L, V; (2) F, Y, W; (3) K, R, H; (4) A, G; (5) S, T; (6) Q, N; and (7) E, D. Stimulation of T cells by the variant peptide T cell activator indicates that the variant peptide is a functional variant. IL-2 is a protein/peptide T cell activator that is well known to a person of skill in the art. FDA approved IL-2 formulations, such as proleukin (Chiron) are readily available commercially. In the composition used the PTPase inhibitor may be selected from the following classes of compounds other pentavalent antimonial compounds, imidazole compounds, or diamidine compounds. The PTPase inhibitor may be a biological equivalent of any of the compounds known to exist in these classes or discovered in the future. The therapeutic composition may comprise mixtures or combinations of those compounds. The pentavalent antimonial compounds of the therapeutic composition may include, but are not limited to meglumine antimonate, and biological equivalents of those compounds. The imidazole compounds of the therapeutic composition may include, but are not limited to, ketoconazole, levamisole, and biological equivalents of those compounds. The diamidine compound may be, but is not limited to, pentamidine and biological equivalents. The cancer that is treated may be, but is not limited to, lymphoma, multiple myeloma, leukemia, melanoma, prostate cancer, breast cancer, renal cancer, and bladder cancer. The therapeutic composition may be used to treat a patient with multiple cancers.

Another embodiment of the invention provides a therapeutic composition for treating cancer, comprising sodium stibogluconate as a leishmaniasis agent and JL-2 as a T cell activator. The term leishmaniasis agent is intended to encompass drugs and compounds currently used to treat leishmaniasis either clinically and/or experimentally, whereby the leishmaniasis agent may be within, but is not limited to, the following classes of compounds other pentavalent antimonial compounds, imidazole compounds, or diamidine compounds. Examples of leishmaniasis agents include, but are not limited to, allopurinol, aminosidine, amphotericine/amphotericine B, interferon, intraconazole, ketoconazole, levamisole, meglumine antimonate, miltefosine, paromomycin, pentamidine isothionate, pentamidine, sitamiquine/WR6026, and biological equivalents of those compounds. The cancer that is treated may be, but is not limited to, lymphoma, multiple myeloma, leukemia, melanoma, prostate cancer, breast cancer, renal cancer, and bladder cancer. The therapeutic composition may be used to treat a patient with multiple cancers. The therapeutic composition may comprise mixtures or combinations of leishmaniasis agents.

Another embodiment of the invention provides a therapeutic composition for treating cancer, comprising sodium stibogluconate and JL-2 as a T cell activator. The cancer that is treated may be, but is not limited to, lymphoma, multiple myeloma, leukemia, melanoma, prostate cancer, breast cancer, renal cancer, and bladder cancer. The therapeutic composition may be used to treat a patient with multiple cancers.

Another embodiment of the invention provides a therapeutic composition for reducing the toxicity of JL-2, comprising JL-2 as a T cell activator and sodium stibogluconate as a PTPase inhibitor. The use of a PTPase inhibitor along with a T cell activator has been surprisingly and unexpectedly discovered to significantly potentiate the therapeutic effectiveness of the T cell activator and to dramatically reduce its toxicity. In the composition used other PTPase inhibitor may be selected from the following classes of compounds: other pentavalent antimonial compounds, imidazole compounds, or diamidine compounds. The PTPase inhibitor may be a biological equivalent of any of the compounds known to exist in these classes or discovered in the future, whereby the pentavalent antimonial compounds of the therapeutic composition may include, but are not limited to meglumine antimonate, and biological equivalents of those compounds and the imidazole compounds of the therapeutic composition may include, but are not limited to, ketoconazole, levamisole, and biological equivalents of those compounds, and the diamidine compound may be, but is not limited to, pentamidine and biological equivalents. The therapeutic composition may comprise mixtures or combinations of those compounds.

Another embodiment of the invention provides a therapeutic composition for treating a disease responsive to cytokine treatment comprising sodium stibogluconate as a leishmaniasis agent and JL-2 as a T cell activator. Many diseases including, but not limited to, an infectious disease, a disease associated with PTPase activity, immune deficiency, cancer, an infection, a viral infection, multiple sclerosis, hepatitis B, and hepatitis C are treated with cytokines and may be amenable to treatment with the compositions of the instant invention. In the composition other leishmaniasis agent may be used, other pentavalent antimonial compounds, imidazole compounds, or diamidine compounds. The leishmaniasis agent may be a biological equivalent of any compounds known to exist in these classes or discovered in the future, whereby the leishmaniasis agents include, but are not limited to, allopurinol, aminosidine, amphotericine/amphotericine B, interferon, intraconazole, ketoconazole, levamisole, meglumine antimonate, miltefosine, paromomycin, pentamidine isothionate, pentamidine, sitamiquine/WR6026 and biological equivalents of those compounds. The therapeutic composition may comprise mixtures or combinations of those compounds.

Another embodiment of the invention provides a therapeutic composition for treating a disease responsive to cytokine treatment comprising sodium stibogluconate or a biological equivalent thereof, and JL-2 as a T cell activator. The disease treated may include, but is not limited to, an infectious disease, a disease associated with PTPase activity, immune deficiency, cancer, an infection, a viral infection, multiple sclerosis, hepatitis B, and hepatitis C. The therapeutic composition may be used to treat a patient with multiple diseases. The T cell activator used preferably induces tumor infiltrating macrophages.

Another embodiment of the invention provides use of a therapeutic composition for treating cancer comprising administering to a patient an effective amount of sodium stibogluconate as an anti-cancer agent and JL-2 as a T cell activator. Other compounds, as the composition may be other pentavalent antimonial compounds, imidazole compounds, or diamidine compounds. The anti-cancer agent may be a biological equivalent of any of the compounds known to exist in these classes or discovered in the future, whereby the anti-cancer agent may comprise mixtures or combinations of those compounds, and whereby the pentavalent antimonial compounds of the therapeutic composition may include, but are not limited to meglumine antimonate, and biological equivalents of those compounds and the imidazole compounds of the therapeutic composition may include, but are not limited to, ketoconazole, levamisole, and biological equivalents of those compounds and the diamidine compound may be, but is not limited to, pentamidine and biological equivalents. The anti-cancer agent may be a PTPase inhibitor. The cancer that is treated may be, but is not limited to, lymphoma, multiple myeloma, leukemia, melanoma, prostate cancer, breast cancer, renal cancer, and bladder cancer. The method may be used to treat a patient with multiple cancers.

Another embodiment of the invention provides the use of sodium stibogluconate and JL-2 for the preparation of a pharmaceutical composition for treating cancer The cancer that is treated may be, but is not limited to, lymphoma, multiple myeloma, leukemia, melanoma, prostate cancer, breast cancer, renal cancer, and bladder cancer. The therapeutic composition may be used to treat a patient with multiple cancers.

Another embodiment of the invention provides the use of sodium stibogluconate and JL-2 for the preparation of a pharmaceutical composition for treating cancer comprising effective amount of sodium stibogluconate and JL-2 as a T cell activator. The cancer that is treated may be, but is not limited to, lymphoma, multiple myeloma, leukemia, melanoma, prostate cancer, breast cancer, renal cancer, and bladder cancer. The method may be used to treat a patient with multiple cancers.

Another embodiment of the invention provides the use of sodium stibogluconate and JL-2 for the preparation of a pharmaceutical composition for treating a disease responsive to cytokine treatment comprising an effective amount of JL-2 as a T cell activator and sodium stibogluconate as a PTPase inhibitor. Diseases including, but not limited to, an infectious disease, a disease associated with PTPase activity, immune deficiency, cancer, an infection, a viral infection, multiple sclerosis, hepatitis B, and hepatitis C may be treated using this method. The T cell activator preferably induces tumor infiltrating macrophages. Other, PTPase inhibitor of the composition may be: other pentavalent antimonial compounds, imidazole compounds, or diamidine compounds. The PTPase inhibitor may be a biological equivalent of any of the compounds known to exist in these classes or discovered in the future, whereby the pentavalent antimonial compounds of the therapeutic composition may include, but are not limited to, meglumine antimonate, and biological equivalents of those compounds, and the imidazole compounds of the therapeutic composition may include, but are not limited to, ketoconazole, levamisole, and biological equivalents of those compounds and the diamidine compound may be, but is not limited to, pentamidine and biological equivalents. The therapeutic composition may comprise mixtures or combinations of those compounds.

Another embodiment of the invention provides the use of sodium stibogluconate and JL-2 for the preparation of a pharmaceutical composition for treating a disease responsive to cytokine treatment comprising an effective amount of a JL-2 as a T cell activator and sodium stibogluconate as a leishmaniasis agent. Diseases including, but not limited to, an infectious disease, a disease associated with PTPase activity, immune deficiency, cancer, an infection, a viral infection, multiple sclerosis, hepatitis B, and hepatitis C may be treated using this method. In the composition other leishmaniasis agent may be used, other pentavalent antimonial compounds, imidazole compounds, or diamidine compounds. The leishmaniasis agent may be a biological equivalent of any compounds known to exist in these classes or discovered in the future, whereby leishmaniasis agents suitable for use include, but are not limited to, allopurinol, aminosidine, amphotericine/amphotericine B, interferon, intraconazole, ketoconazole, levamisole, meglumine antimonate, miltefosine, paromomycin, pentamidine isothionate, pentamidine, sitamiquine/WR6026 and biological equivalents of those compounds. The therapeutic composition may comprise mixtures or combinations of those compounds.

Another embodiment of the invention provides the use of a therapeutic composition for treating a disease responsive to cytokine treatment comprising an effective amount of sodium stibogluconate and JL-2 as a T cell activator. The disease treated by this method may include, but is not limited to, an infectious disease, a disease associated with PTPase activity, immune deficiency, cancer, an infection, a viral infection, multiple sclerosis, hepatitis B, and hepatitis C. The method may be used to treat a patient with multiple diseases.

Another embodiment of the present invention relates to fractionating a compound comprising a mixture of compounds. In any of the above embodiments, if the compound provided or used as part of a therapeutic composition or use comprises a mixture of compounds, the mixture may be fractionated and one or more fractions may be eliminated. Compounds present in a mixture of compounds may comprise different molecular weight compounds (e.g., polymers), conformers, enantiomers, isomers, analogues, derivatives, unreacted precursors, alternative products, intermediates, or degradation products. As an example, sodium stibogluconate exists as a polymer of multiple species with molecular weights varying from 100 to 4,000 amu. Fractionation of a parent mixture of sodium stibogluconate by chromatography, or another suitable method, provides fractions with varying PTPase inhibitory activity. Elimination of fractions with relatively low or no PTPase inhibitory activity may increase the PTPase inhibitory activity of the overall solution. Further, toxicity associated with degradation or other products or components within a parent mixture may be reduced when fewer molecular species are present in the final mixture.

Another embodiment of the invention provides the use of sodium stibogluconate and JL-2 for the preparation of a pharmaceutical composition for reducing the toxicity of IL-2. Another embodimen relates to the use of sodium stibogluconate for the preparation of a pharmaceutical composition for reducing the toxicity of JL-2 in a subject undergoing IL-2 therapy. Another embodiment of the invention provides the use of sodium stibogluconate and JL-2 for the preparation of a pharmaceutical composition for potentiating the therapeutic efficacy of IL-2. Another embodiment relates to the use of sodium stibogluconate for the preparation of a pharmaceutical composition for potentiating the therapeutic efficacy of JL-2 in a subject undergoing IL-2 therapy.

The embodiments of the invention presented above provide for compositions and uses for the preparation of a pharmaceutical composition for the prophylactic and therapeutic treatment of diseases associated with protein tyrosine activity or abnormal activity thereof "Prophylactic" means the protection, in whole or in part, against a particular disease or a plurality of diseases. "Therapeutic" means the amelioration of the disease itself, and the protection, in whole or in part, against further disease. The uses comprise the administration of an inhibitor of a PTPase in an amount sufficient to treat a subject either prophylactically or therapeutically. The drugs disclosed herein include all biological equivalents (i.e. pharmaceutically acceptable salts, precursors, derivatives, and basic forms). "To mix", "mixing", or "mixture(s)" as used herein means mixing a substrate and an agonist: 1) prior to administration ("in vitro mixing"), 2) mixing by simultaneous and/or consecutive, but separate (i.e. separate intravenous lines) administration of substrate and agonist (angiogenic growth factor) to cause "in vivo mixing".

Preferably, the drug administered to a patient is a biological equivalent of the compounds disclosed herein, which are effective in inhibiting protein tyrosine phosphatases. A biological equivalent is a pharmaceutically acceptable analogue, precursor, derivative, or pharmaceutically acceptable salt of the compounds disclosed herein. One of ordinary skill in the art will appreciate that a precursor, which may also be referred to as a prodrug, must be one that can be converted to an active form of the drug in or around the site to be treated.

The drugs described herein or pharmaceutically acceptable salts can be administered in accordance with the present inventive method by any suitable route. Suitable routes of administration include systemic, such as orally or by injection, topical, intraocular, periocular, subconjunctival, subretinal, suprachoroidal and retrobulbar. The manner in which the drug is administered may be dependent, in part, upon whether the treatment is prophylactic or therapeutic.

One skilled in the art will appreciate that suitable methods of administering a therapeutic composition useful in the above listed embodiments are available. Although more than one route can be used to administer a particular therapeutic composition, a particular route can provide a more immediate and more effective reaction than another route. Accordingly, the described routes of administration are merely exemplary and are in no way limiting.

The particular dose administered to an animal, particularly a human, in accordance with the present invention should be sufficient to effect the desired response in the animal over a reasonable time frame. The therapeutic compositions disclosed herein may be administered to various subjects including, but not limited to animals, which includes mammals, which in turn includes humans. One skilled in the art will recognize that dosage will depend upon a variety of factors, including the strength of the particular therapeutic composition employed, the age, species, condition or disease state, and body weight of the animal. The size of the dose also will be determined by the route, timing and frequency of administration as well as the existence, nature, and extent of any adverse side effects that might accompany the administration of a particular therapeutic composition and the desired physiological effect. It will be appreciated by one of ordinary skill in the art that various conditions or disease states, in particular, chronic conditions or disease states may require prolonged treatment involving multiple administrations.

Suitable doses and dosage regimens can be determined by conventional range-finding techniques known to those of ordinary skill in the art. Generally, treatment is initiated with smaller dosages, which are less than the optimum dose of the compound. Thereafter, the dosage is increased by small increments until the optimum effect under the circumstances is reached.

The administration(s) may take place by any suitable technique, including, but not limited to, subcutaneous and parenteral administration. Examples of subcutaneous administration include intravenous, intra-arterial, intramuscular, and intraperitoneal. The dose and dosage regimen will depend mainly on whether the therapeutic composition is being administered for therapeutic or prophylactic purposes, separately or as a mixture, the type of biological damage and host, the history of the host, and the type of inhibitors or biologically active agent. The amount must be effective to achieve an enhanced therapeutic index. Humans are generally treated longer than mice and rats with a length proportional to the length of the disease process and drug effectiveness. Doses may be single doses or multiple doses over a period of several days. Therapeutic purpose is achieved, as defined herein, when the treated hosts or patients exhibit improvement against disease or infection, including but not limited to improved survival rate, more rapid recovery, or improvement or elimination of symptoms. If multiple doses are employed, as preferred, the frequency of administration will depend, for example, on the type of host and type of cancer, dosage amounts, etc. The practitioner may need to ascertain upon routine experimentation which route of administration and frequency of administration are most effective in any particular case.

Compositions for use in the embodiments disclosed above preferably comprise a pharmaceutically acceptable carrier, known as an excipient, and an amount of the therapeutic composition sufficient to treat the particular disease prophylactically or therapeutically. The carrier can be any of those conventionally used and is limited only by chemico-physical considerations, such as solubility and lack of reactivity with the compound, and by the route of administration. One of ordinary skill in the art will appreciate that, in addition to the following described pharmaceutical compositions, the therapeutic composition can be formulated as polymeric compositions, inclusion complexes, such as cyclodextrin inclusion complexes, liposomes, microspheres, microcapsules and the like (see, e.g., U.S. Pat. Nos. 4,997,652; 5,185,152; and 5,718,922 ).

The therapeutic composition can be formulated as a pharmaceutically acceptable acid addition salt. Examples of pharmaceutically acceptable acid addition salts for use in the pharmaceutical composition include those derived from mineral acids, such as hydrochloric, hydrobromic, phosphoric, metaphosphoric, nitric and sulfuric acids, and organic acids, such as tartaric, acetic, citric, malic, lactic, fumaric, benzoic, glycolic, gluconic, succinic, and arylsulphonic, for example p-toluenesulphonic, acids.

The pharmaceutically acceptable excipients described herein are well known to those who are skilled in the art and are readily available to the public. Preferably the pharmaceutically acceptable excipient is chemically inert to the therapeutic composition and has no detrimental side effects or toxicity under the conditions of use.

The embodiments described above can also involve the co-administration of other pharmaceutically active compounds. By "co-administration" is meant administration before, concurrently with, e.g., in combination with the therapeutic composition in the same formulation or in separate formulations, or after administration of a therapeutic composition as described above. For example, corticosteroids, e.g., prednisone, methylprednisolone, dexamethasone, or triamcinalone acetinide, or noncorticosteroid anti-inflammatory compounds, such as ibuprofen or flubiproben, can be co-administered. Similarly, vitamins and minerals, e.g., zinc, anti-oxidants, e.g., carotenoids (such as a xanthophyll carotenoid like zeaxanthin or lutein), and micronutrients can be co-administered. In addition, other types of inhibitors of the protein tyrosine phosphatase pathway can be co-administered.

The following examples and discussion are meant to further illustrate the functionality and use of the molecules and methods described above

### EXAMPLES

I. Sodium Stibogluconate is a Potent Inhibitor or Protein Tyrosine Phosphatases and Augments Responses in Hemopoietic Cell Lines.

Chemical reagents were screened by in vitro phosphatase assays to identify inhibitors of the SHP-1 phosphatase. Sodium stibogluconate was found to be a potent in vitro inhibitor of protein tyrosine phosphatases, including SHP-1, SHP-2, and PTP1B, but not the dual specificity phosphatase MKP1. SHP-1 phosphatase activity in vitro was almost completely inhibited by the sodium stibogluconate at 10 µg/ml, a concentration less than or equal to the peak serum level obtained in human beings treated for leishmaniasis. The inhibitory activity of the sodium stibogluconate against PTPases in vivo was indicated by an enhancement of tyrosine phosphorylation of distinct cellular proteins in Baf3 cells and by an augmentation of Baf3 proliferation induced by the hematopoietic growth factor IL-3. Importantly, sodium stibogluconate augmented the opposite effects of GM-CSF and IFN-alpha on TF-1 cell growth, suggesting broad activities of the drug in enhancing the signaling of various cytokines.

A. Materials and Methods

1. Chemicals and Reagents

Protein tyrosine phosphatase assay kits and GST fusion protein of protein tyrosine phosphatase 1B (PTP1B) were purchased from Upstate Biotechnology Inc. (Lake Placid, N.Y.). Suramin and potassium antimonyl tartrate was purchased from Sigma (St. Louis, Mo.). Sodium stibogluconate (its Sb content is 100 µg/ml and used to designate sodium stibogluconate concentration hereafter) was a gift from Dr. Xiaosu Hu (Sichuan Medical College, China). GST fusion proteins of SHP-1 (Yi et al.,Mol. Cell. Biol. 12, 836 (1992)) and SHP-2 (Frearson et al., Eur. J. Immunol. 26, 1539 (1996)) were prepared following protocols established in Burshtyn et al., J. Biol. Chem. 272, 13066 (1997). The GST fusion protein of SHP-1 cata was purified from DH5a bacteria transformed with a pGEX construct containing the coding region of the PTPase catalytic domain (amino acids 202 to 554) of murine SHP-1, derived by PCR from the murine SBP-1 cDNA. The GST fusion protein of mitogen-activated protein kinase phosphatase 1 (MKP1) was purified from DH5a bacteria transformed with a pGEX construct containing the coding region of MKP1 cDNA derived by RT-PCR using the following primers (MKP1/5, 5'ctggatcctgcgggggctgctgca- ggagcgc (SEQ ID NO: 1); MKP1/3, 5'aagtcgacgcagcttggggaggtggtgat) (SEQ ID NO: 2).

Murine IL-3 (Yang et al., Blood 91, 3746 (1998)), recombinant human GM-CSF (Thomassen et al., Clin. Immunol. 95, 85 (2000)) and recombinant human IFN-alpha (Uddin et al., Br. J. Haematol. 101, 446 (1998)) have been described previously. Antibodies against phosphotyrosine (anti-ptyr, 4G10, UBI), .beta.-actin (Amersham, Arlington Heights, Ill.), phosphotyrosine Stat5 (New England BioLab Inc, Beverly, Mass.) and Jak2 (Affinity BioReagents, Inc., Golden, Colo.) were purchased from commercial sources.

2. In Vitro Protein Tyrosine Phosphatase Assays.

In vitro PTPase activities were measured using the commercial protein tyrosine phosphatase assay kit (Upstate Biotechnology, Inc. ("UBI")) following established procedure known to one of skill in the art. This assay measures the in vitro dephosphorylation of a synthetic phosphotyrosine peptide of the sequence Arg-Arg-Leu-Ile-Glu-Asp-Ala-Gle-T- yr-Ala-Ala-Arg-Gly, wherein the tyrosine is phosphorylated (SEQ ID NO: 3). Briefly, 0.01 µg of GST/PTPase fusion proteins was incubated in 50 µl of Tris buffer (10 mM Tris, pH 7.4) containing different concentrations of inhibitors or chemicals (0-1,000 µg/ml) at 22.degree. C. for 10 minutes, followed by addition of 0.2 mM of the phosphotyrosine peptide and incubation at 22.degree. C. for 18 hours. 100 µl of Malachite Green solution was added and incubated for 5 minutes, and the absorbance at 660 nm was measured after 5 minutes.

To assess the reversibility of inhibition of SHP-1 by PTPase inhibitors, GST/SHP-1 fusion protein bound on glutathione beads were pre-incubated in cold Tris buffer or Tris buffer containing the PTPase inhibitors at 4° C. for 30 minutes. The beads were then either subjected to in vitro PTPase assays or washed 3 times in Tris buffer then subjected to in vitro PTPase assays.

3. Cells, Cell Culture and Cell Proliferation Assays.

The murine hematopoietic cell line Baf3 was maintained in RPMI 1640 medium supplemented with 10% fetal calf serum (FCS) and murine IL-3 (20 units/ml) as described previously in Damen et al., J. Biol. Chem. 270, 23402 (1995). Human myeloid cell line TF-1 was maintained in RPMI 1640 supplemented with 10% FCS and 40 ng/ml of recombinant human GM-CSF as described previously in Thomassen et al., Clin. Immunol. 95, 85 (2000). For cell proliferation assays, cells were washed in 10% FCS medium twice, resuspended in 10% FCS medium, incubated at 37° C. for 16 hours, and then cultured at 37° C. in 10% FCS medium containing various amounts of cytokines, sodium stibogluconate, or potassium antimonyl tartrate for 3-6 days as indicated. The cell numbers in proliferation assays were determined by an MTT assay or by microscopic cell counting as indicated.

4. Induction of Cellular Protein Phosphorylation and Western Blotting.

For induction of cellular protein phosphorylation by sodium stibogluconate or pervanadate, Baf3 cells were incubated in 0.1% FCS-RPMI 1640 medium at 37° C. for 16 hours. The cells were then washed twice in RPMI 1640 medium and incubated with sodium stibogluconate or pervanandate (0.1 mM) for various times prior to termination by lysing cells in cold lysis buffer (50 mM Tris, pH 7.4; 150 mM NaCl; 0.2 mM Na₃VO₄; 20 mm NaF; 1% Nonidet P-40; 2 mM PMSF; 20 µg/ml of Aprotinin and 1 mM of sodium molybdic acid). To determine the effect of sodium stibogluconate or potassium antimonyl tartrate on IL-3-induced Jak/Stat phosphorylation, Baf3 cells were deprived of the growth factor for 16 hours in 0.1% FCS RPMI 1640 medium and then incubated with or without sodium stibogluconate or potassium antimonyl tartrate for 10 minutes. IL-3 was next added to the cell suspension and incubated for various times. The Cells were then harvested and lysed in cold lysis buffer at 4° C. for 45 minutes. Total cell lysates (TCL) were separated in SDS-PAGE gels, blotted onto nitrocellulose membrane (Schleicher & Schuell), probed with specific antibodies and detected using an enhanced chemiluminescence kit (ECL, Amersham, Arlington Heights, Ill.).

B. Results

1. Sodium Stibogluconate Inhibits Protein Tyrosine Phosphatases In Vitro.

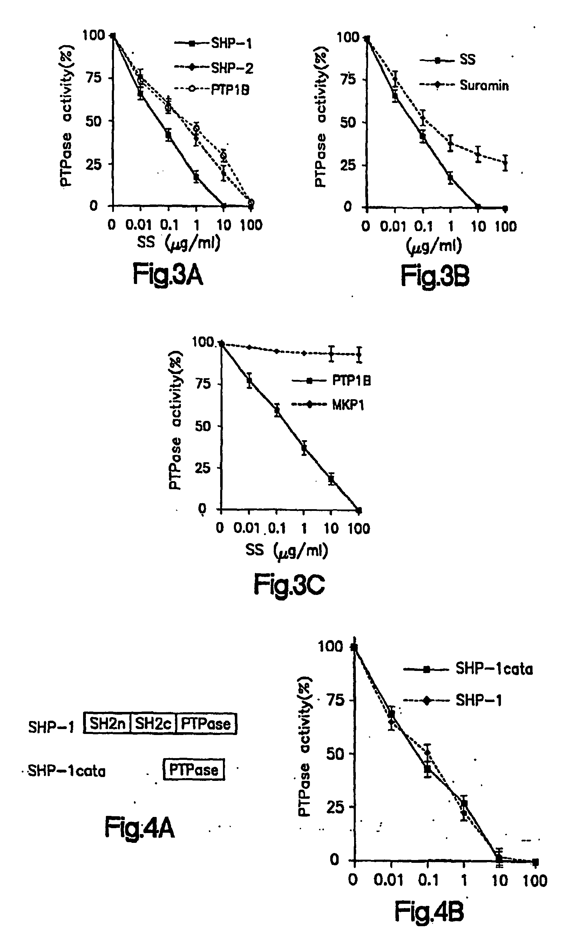

Through screening various chemical compounds by in vitro PTPase assays, sodium stibogluconate was identified as an inhibitor of PTPases. The dephosphorylation of a synthetic phosphotyrosine peptide by the GST/SHP-1 fusion protein was almost completely blocked (99%) by sodium stibogluconate at 10 µg/ml (FIG. 3A) (data represent the mean +/- SD values of triplicate samples). Sodium stibogluconate also inhibited SHP-2 and PTP1B.(FIG. 3A), however, approximately 10 fold higher concentrations of the drug (100 µg/ml) were required to achieve a similar degree (about 99%) of inhibition (FIG. 3A). Inhibition of SHP-1 by the known PTPase inhibitor Suramin was less effective under comparable conditions (FIG. 3B). The drug showed no obvious inhibitory activity against MKP1, a dual-specificity protein tyrosine phosphatase (FIG. 3C). Under the experimental conditions, the GST fusion proteins of SHP-1, SHP-2, PTP1B and MKP1 showed similar PTPase activities against the peptide substrate (660 nm absorbance approximately 0.6 above background (0.03)) in the absence of inhibitors.

2. Sodium Stibogluconate Targets the SHP-1 PTPase Catalytic Domain and Forms Stable Complexes with the Phosphatase In Vitro.

Substrate dephosphorylation is mediated by the PTPase catalytic domain, the activity of which is often regulated by flanking N-terminal and C-terminal regions. To define whether sodium stibogluconate inhibited PTPases through targeting the PTPase catalytic domain or via the flanking regulatory regions, the effect of sodium stibogluconate on the GST/SHP-1 fusion protein was compared with the GST/SHP-1cata fusion protein, which contains the PTPase catalytic domain but has the SH2 domains and the C-terminal region deleted (FIG. 4A). Sodium stibogluconate showed similar activities in inhibiting the two proteins in their dephosphorylation of the phosphotyrosine peptide substrate in vitro (FIG. 4B) (data represent the mean +/- SD values of triplicate samples), demonstrating that inhibition of SHP-1 PTPase activity by sodium stibogluconate does not require the SHP-1 SH2 domains and the C-terminal region. These results provide strong evidence that sodium stibogluconate directly targets the SUP-1 PTPase catalytic domain.

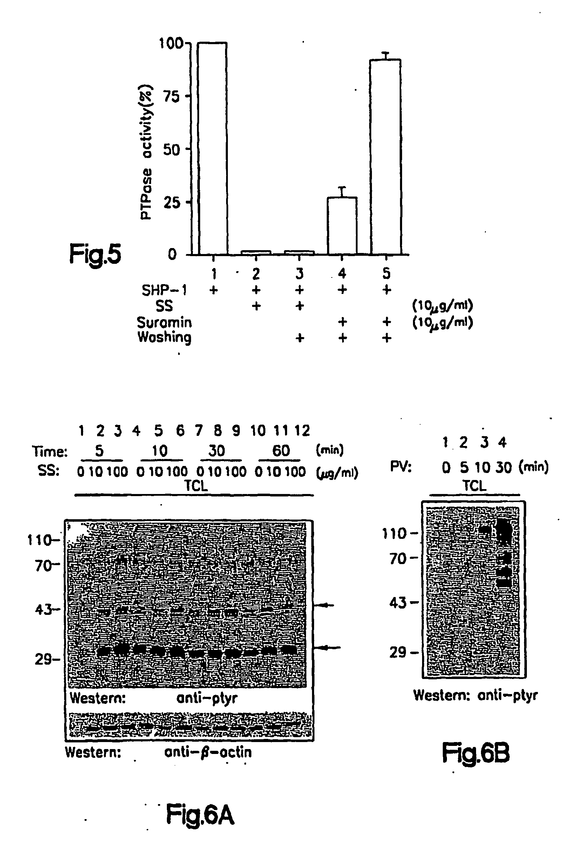

To determine whether the in vitro inhibition of SHP-1 PTPase by sodium stibogluconate is a reversible process, it was examined whether washing the GST-SHP-1 fusion protein pre-incubated with sodium stibogluconate could relieve the inhibition. The inhibition of the GST/SHP-1 fusion protein by sodium stibogluconate was not affected by washing (FIG. 5). In contrast, the inhibition of the phosphatase by Suramin was almost completely removed by the washing process (FIG. 5), consistent with a previous report (Zhang et al., J. Biol. Chem. 273, 12281 (1998)).

3. Sodium Stibogluconate Induces Tyrosinephosphorylation of Cellular Proteins and Augments IL-3-Induced Jak2/Stat5 Phosphorylation in Baf3 Cells.

The effect of sodium stibogluconate on cellular protein tyrosine phosphorylation in the murine IL-3-dependent cell line Baf3 was examined to determine whether sodium stibogluconate functions as a PTPase inhibitor in vivo. Treatment of Baf3 cells with sodium stibogluconate induced protein tyrosine phosphorylation (FIG. 6A) that was modest and transient in comparison with those induced by pervanadate (0.1 mM) (FIG. 6B). Increased tyrosine phosphorylation of cellular proteins of approximately 55 and 32 kDa was apparent in cells incubated with the drug for 5 minutes (FIG. 6, Lane 1-3). This induction of cellular protein tyrosine phosphorylation was dose-dependent with more marked induction occurring at the higher drug concentration (FIG. 6, comparing lane 2 and 3). Heightened phosphorylation of these proteins was also detected with prolonged treatment of 10, 30 or 60 minutes but at more modest levels (FIG. 6, Lane 4-12). This increased protein tyrosine phosphorylation was not due to variations in the protein samples as indicated by the similar amounts of P-actin protein in these samples (FIG. 6, lower panel). The drug showed no obvious effect on several other phosphotyrosine cellular proteins in the total cell lysate (TCL) samples (FIG. 6), suggesting certain specificity of the drug in induction of protein tyrosine phosphorylation. The identities of the 55 and 32 kDa proteins have not been determined. The weaker phosphorylation signal of the p32 band in lane 1 of FIG. 6 compared to that of lane 4, 7 and 10 was not consistently detected.

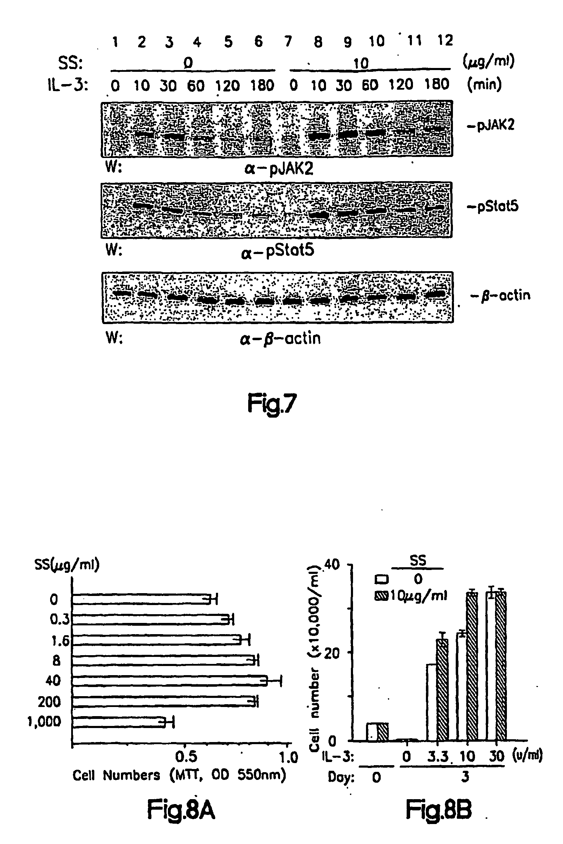

To determine whether sodium stibogluconate inhibits SHP-1 in vivo, the effect of the drug on IL-3-induced Jak2 tyrosine phosphorylation in Baf3 cells was examined (FIG. 7). Baf3 cells deprived of IL-3 were incubated with or without the drug for 10 minutes and then stimulated with IL-3 for various times. IL-3 induced tyrosine phosphorylation of Jak2 and Stat5 in Baf3 cells in the presence or absence of the drug. However, the phosphotyrosine levels of Jak2 and Stat5 in the presence of the drug were about twice of those in cells without drug treatment as determined by densitometry analysis (FIG. 7, comparing Lane 2-6 and Lane 8-12).

In cells unstimulated by IL-3, tyrosine phosphorylation of the two proteins was undetectable in the presence or absence of the drug (FIG. 7, Lane 1 and 7). Prolonged incubation with the drug alone at 37° C. for 16 hours also failed to induce Jak2/Stat5 tyrosine phosphorylation.

4. Sodium Stibogluconate Augments IL-3-induced Cell Proliferation of Baf3 Cells.

SHP-1 is known to down-regulate cytokine signaling as demonstrated by the hyperresponsiveness of SHP-1-deficient cells to various cytokines, including IL-3. The inhibitory activity of sodium stibogluconate against SHP-1 predicted that the drug would augment IL-3-induced proliferation of Baf3 cells. Indeed, IL-3-induced Baf3 proliferation was increased in the presence of sodium stibogluconate at 0.3 to 200 µg/ml with the maximal effect concentration about 40 µg/ml (FIG. 8A). At a higher concentration (1,000 µg/ml), the drug suppressed IL-3-induced Baf3 growth (FIG. 8A). This growth promoting activity of the drug was apparent at suboptimal (3.3 or 10 units/ml), but not optimal (30 unit/ml), amounts of IL-3 (FIG. 8B). In the absence of IL-3, sodium stibogluconate failed to support cell proliferation or maintain cell viability in day 3 culture (FIG. 8B).

5. Sodium Stibogluconate Augments the Opposite Effects of GM-CSF and IFN-Alpha on the Proliferation of TF-1 Cells.

The Jak/Stat signaling pathways transduce signals initiated by cytokines that often have opposite effects on cell growth. The human myeloid leukemia cell line TF-1 responds to both GM-CSF, which promotes proliferation, and IFN-alpha, which inhibits cell growth. To determine whether the effect of the PTPase inhibitor is unique for the IL-3-initiated signaling events or affects other cytokines, the growth responses of TF1 cells to GM-CSF and IFN-alpha in the presence or absence of sodium stibogluconate was examined.

Proliferation of TF-1 cells was induced by suboptimal concentrations of GM-CSF (5-40 ng/ml) in a dose-dependent manner (FIG. 9A) (data represent the mean +/- SD values of triplicate samples). This proliferation of TF-1 cells was augmented in the presence of sodium stibogluconate at 50 µg/ml (FIG. 9A). No viable cells were detected in the cultures lacking GM-CSF in the presence or absence of the drug (FIG. 9A). These results demonstrated that sodium stibogluconate augmented the growth promoting activity of GM-CSF in TF-1 cells but could not substitute the growth factor for maintaining cell viability or promoting growth under the experimental conditions.

In the presence of IFN-alpha, GM-CSF-induced proliferation of TF-1 cells was suppressed (FIG. 9B). Further reduction of GM-CSF-induced cell growth was detected in cultures containing both IFN-alpha and sodium stibogluconate (50 µg/ml) (FIG. 9B and C), indicating that the growth inhibition activity of IFN-alpha was enhanced in the presence of the drug. Since the enhanced growth inhibition of IFN-alpha by the drug occurred in the presence of GM-CSF, it indicated the dominance of the synergy between IFN-alpha and the drug over the activity of the drug in augmenting GM-CSF mitogenic signaling under the experimental conditions.

As shown in FIG. 9D, the activity of sodium stibogluconate in augmenting GM-CSF-induced TF-1 proliferation was dose-dependent, with the optimal activity at 50 µg/ml. On the other hand, more dramatic growth inhibition in the presence of IFN-alpha occurred at higher concentrations of the drug (FIG. 9E). Since the drug at low doses (12.5-50 µg/ml) showed no negative effect on GM-CSF-induced cell growth, its effect at such doses in augmenting IFN-alpha-induced growth inhibition was likely resulted from specific enhancement of IFN-alpha signaling. On the other hand, non-specific toxicity of drug at higher doses in combination with IFN-alpha might have contributed to the more dramatic growth inhibition.

6. The Sb(III) Form of Potassium Antimonyl Tartrate Lacks Inhibitory Activity Against PTPases.

Sodium stibogluconate is of Sb(V) form and transforms inside cells into Sb(III) form that can affect leishmania growth. The activity of potassium antimonyl tartrate of SB(III) form in inhibiting PTPases in vitro and in vivo was determined.

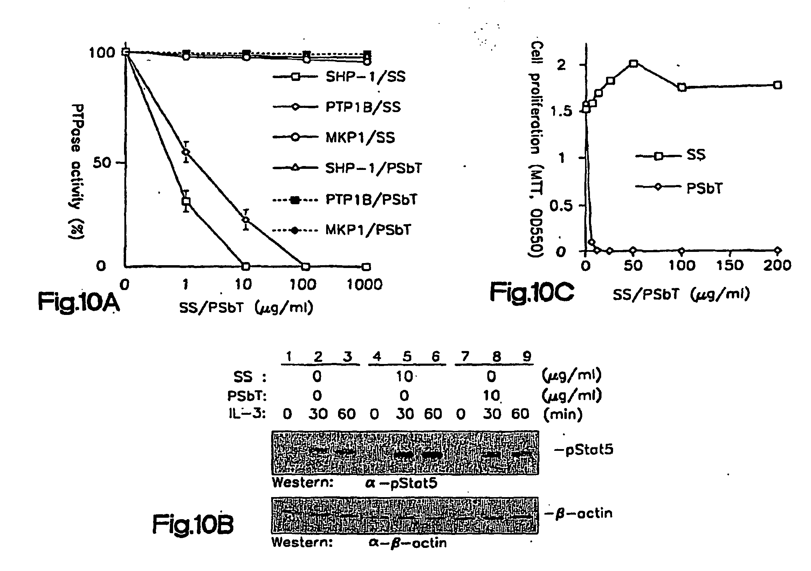

Unlike sodium stibogluconate, potassium antimonyl tartrate at 1-1,000 µg/ml showed no detectable inhibition of PTPases SHP-1 and PTP1B in vitro (FIG. 10A). It also failed to enhance IL-3-induced Stat5 phosphorylation (FIG. 10B) or IL-3-induced proliferation of Baf3 cells (FIG. 10C), indicating its lack of inhibitory activity against PTPases in vivo (data represent the mean +/- SD values of triplicate samples). Interestingly, it showed marked toxicity against Baf3 cells. The results together indicate that only the Sb(V) form acts as a PTPase inhibitor that is inactivated when transformed into the Sb(III) form.

C. Discussion

These data demonstrate that sodium stibogluconate is a potent inhibitor of protein tyrosine phosphatases in vitro and in vivo. Sodium stibogluconate inhibited the dephosphorylation of a synthetic phosphotyrosine peptide substrate by protein tyrosine phosphatases (SHP-1, SHP-2 and PTP1B) in in vitro PTPase assays (FIG. 3). The dephosphorylation of pNPP (p-nitrophenyl phosphate, Sigma) by these PTPases in vitro was also similarly inhibited by the drug. The inhibitory activity of the drug against PTPases in vivo was indicated by the rapid induction of protein tyrosine phosphorylation of the two yet-unidentified cellular proteins of 56 and 32 kDa in Baf3 cells (FIG. 6). Interestingly, proteins of similar molecular weights had been found to be hyperphosphorylated in SHP-1 deficient cells in previous studies (Yang et al., Blood 91, 3746 (1998)). Induced cellular protein tyrosine phosphorylation was less dramatic with prolonged drug incubation (FIG. 6), suggesting that the drug may be unstable under the experimental conditions or that the drug may sequentially inactivate PTPases with opposite effects on the phosphorylation of the cellular proteins. In this regard, it is interesting that PTPases were inhibited by the Sb(V) form of sodium stibogluconate which is known to transform in cells to the Sb(III) form that failed to show PTPase inhibitory - activity (FIG. 10). The intracellular transformation therefore could result in inactivation of the PTPase inhibitor and may account for the drug's modest and transient induction of tyrosine phosphorylation and modest effect on cell proliferation. This may have a beneficial side as it may be related to the lower toxicity of the drug in comparison to other PTPase inhibitors that allows its clinical application.

The inhibitory activity of sodium stibogluconate against PTPases in vivo was further indicated by the augmentation of IL-3-induced Jak2/Stat5 phosphorylation and IL-3-induced proliferation of Baf3 cells. Previous experiments have shown that SHP-1 dephosphorylates the Jak family kinases to down regulate signaling initiated by cytokines (Jiao et al., Exp. Hematol. 25, 592 (1997)). Among the Jak kinases, IL-3 specifically activates the Jak2 kinase which phosphorylates the Stat5 protein to regulate gene expression. The observation that sodium stibogluconate augmented IL-3-induced Jak2/Stat5 tyrosine phosphorylation and IL-3-induced proliferation of Baf3 cells is therefore consistent with inhibition of SHP-1 by the drug in vivo. However, it remains possible that the effect of the drug on IL-3-induced Jak2/Stat5 phosphorylation and cell proliferation involves additional PTPases (e.g., the CD45 PTPase ) that participate in dephosphorylating the Jak kinases. Indeed, sodium stibogluconate augmented GM-CSF-induced Tyk2/Stat3 tyrosine phosphorylation in SHP-1-deficient cells. That the enhancement of IL-3-induced Jak2/Stat5 tyrosine phosphorylation by the drug was more dramatic in later time points to post IL-3 stimulation, indicating induction of extended period of phosphorylation by the drug. Such an effect of the drug suggests its targeting of PTPases recruited to Jak2/Stat5 at the later time points post IL-3 stimulation to inactivate the signaling molecules.

Inhibition of PTPases in vivo by sodium stibogluconate was also consistent with the observation that the drug augmented the opposite effects of GM-CSF and IFN-alpha on TF-1 cell proliferation (FIGS. 9 and 10). In particular, the observation suggested that the drug targeted PTPases that dephosphorylate shared signaling molecules (e.g., the Jak family kinases) utilized by both GM-CSF and IFN-alpha. Such a putative mechanism would explain the cytokine-dependent effects of the drug: its inhibition of PTPases leads to amplification of both mitogenic and growth inhibitory signals initiated by GM-CSF and IFN-alpha respectively. It also suggests that sodium stibogluconate may have broad activities in augmenting the signaling of various cytokines. It is worth noticing that SHP-1 has been shown in previous studies to down regulate the signaling of GM-CSF and IFN-alpha. It was reported that macrophages from SHP-1-deficient mice show approximately 2 folds increase of GM-CSF-induced cell growth in comparison to controls. This level of growth increase is similar to the increase of GM-CSF-induced TF-1 cell growth in the presence of sodium stibogluconate, consistent with inhibition of SHP-1 by the drug. In light of the pathogenic effect of SHP-1-deficient monocytes/macrophages in the fatal motheaten phenotype, it is possible that the apparently modest effect of the drug on GM-CSF-induced cell growth could have significant biological consequences in vivo.

These results also suggest that inhibition of PTPases by sodium stibogluconate at therapeutic concentrations to increase Jak/Stat phosphorylation and cellular responses to cytokines may be a major factor responsible for the pharmacological effect of the drug in the treatment of leishmaniasis. Among the cytokines that depend on Jak/Stat pathways for signal transduction, IFN-gamma plays an important role in eliminating intracellular leishmania. Moreover, impaired IFN-gamma signaling was detected in leishmania-infected macrophages and was associated with activation of SHP-1 by the parasite. Therefore, it could be postulated that sodium stibogluconate may augment IFN-gamma signaling in macrophages via inhibiting SHP-1 (and other PTPases) and contribute to the clearance of intracellular leishmania. Thus, anti-leishmania activity of sodium stibogluconate may derive both from augmenting cell signaling by Sb(V) and from parasite-killing by Sb(III) transformed from Sb(V) inside cells. Such a functional mechanism, nevertheless, is consistent with previous observations that modulation of host PTPases with specific inhibitors can effectively control the progression of leishmania infection by enhancing cytokine signaling in macrophages. In light of the observation that anti-leishmania drug sodium arsenite inhibits LPS-induced MAP kinase signaling in macrophages, modulation of cellular signaling could be a common mechanism of anti-leishmania drugs.

The mechanism through which sodium stibogluconate inhibits PTPases is likely by targeting the PTPase catalytic domain of the enzymes. The drug was effective in inhibiting both the wild type SHP-1 and the SBP-1 mutant containing the PTPase domain without the flanking N-terminal SH2 domains or the C-terminal region that regulate SHP-1 activity (FIG. 4). This mechanism is also consistent with the observation that the drug inhibited PTP1B, which, except for its PTPase catalytic domain, has no apparent structure similarity with SHP-1 and SHP-2. In this regard, it is not unexpected that the drug showed no obvious activity against MKP1 since the amino acid sequence and structure of the catalytic domain of dual specificity phosphatases are substantially different from those of the tyrosine specific PTPases. Such a mechanism also suggests that the drug may have inhibitory activities against all tyrosine specific PTPases that have the conserved PTPase catalytic domain. While these results indicated that the drug formed a stable complex with SBP-1 in vitro that was resistant to a washing process, it is not clear at present whether this was due to docking of the drug into a pocket structure in the PTPase domain or involved the formation of covalent bonds. In the former case, it is likely subtle differences in the putative pocket structure of PTPases may be responsible for the different sensitivities of the enzymes to the inhibitor in vitro. In addition, it also suggests the feasibility of developing chemical derivatives of the drug with more specific and potent activities against individual PTPases.

Demonstrated differential sensitivities of PTPases to the drug in vitro suggest similar differential sensitivities of PTPases in vivo, which may explain the dose-dependent effect of the drug on IL-3-induced cell proliferation and the known clinical side effect of the drug at higher dosages. Sodium stibogluconate augmented IL-3-induced Baf3 proliferation at therapeutic concentrations and suppressed cell growth at higher dosages. In clinical applications, sodium stibogluconate at therapeutic dosages was well tolerated, but is known at higher dosages to have side effects that include reversible nonspecific ECG changes and renal defects. Effects of the drug at higher dosages may be related to inhibition of PTPases that are only sensitive to the drug at higher concentrations.

II. Effects of Sodium Stibogluconate on Differentiation and Proliferation of Human Myeloid Leukemia Cell Lines In Vitro

To explore the potential of sodium stibogluconate as a drug in differentiation induction therapy in the treatment of AML, the effect of sodium stibogluconate on differentiation of various human AML cell lines in vitro was examined. The data demonstrated that sodium stibogluconate induces differentiation of AML cell lines NB4, HL-60, and U937 in a dose- and time-dependent manner. At optimal dosage, sodium stibogluconate induced irreversible differentiation of NB4 cells to a level similar to that induced by ATRA. Sodium stibogluconate-induced differentiation of HL-60 and U937 cells was at 60% and 50% respectively, which were augmented by GM-CSF to levels nearly equal or higher than those induced by ATRA in the two cell lines. These results indicate the potential of sodium stibogluconate, and probably other PTPase inhibitors, in AML treatment.

A. Materials and Methods

1. Reagents

All-trans-retinoic acid (ATRA), nitroblue tetrazolium (NBT), and 12-O-tetradecanoylphorbol-13-acetate (TPA) were purchased from Sigma (Saint Louis, Mo.). Sodium stibogluconate (Pathak et al., J. Immunol. 167, 3391 (2001)) and recombinant human GM-CSF (granulocyte/macrophage colony stimulating factor) (Thomassen et al., Clin. Immunol 95, 85 (2000)) have been described previously.

2. Cell Lines, Cell Culture, and Cell Proliferation Assay.

The NB4 cell line (Lanotte et al., Blood 77, 1080 (1991)) was a gift from Dr. Dan Lindner of the Cleveland Clinic Foundation. IL-60 and U937 cell lines were purchased from American Type Culture Collection (Rockville, Md.). These human AM cell lines were maintained in RPMI 1640 medium supplemented with 10% fetal calf serum (FCS). For cell proliferation assays, cells were cultured at 37° C. in 10% RCS medium containing various amounts of sodium stibogluconate for 6 days. The cell numbers in the cultures were determined by an MTT assay as described previously in Mosmann, J. Imunol. Methods 65, 55 (1983).

3. Studies of Induction of Differentiation

Differentiation of AML cell lines was assessed by their ability to produce superoxide as measured by reduction of NBT to formazan and by analysis of expression of CAD11b surface marker by flow cytometry. For NBT reduction, each cell suspension was mixed with an equal volume of solution containing 1 mg/ml of NBT (Sigma) and 2.5 µg/ml of TPA for 30 minutes at 37° C. After incubation, cells containing the purple formazan deposits and cells devoid of NBT-reducing activity (white cells) in each sample were determined by counting 200 cells under microscope. Data is expressed as a percentage based on the following ratio: purple cells/purple+white cells. For analysis of cell surface antigens, cells were exposed to phycoerythrin (PE)-conjugated murine anti-human CD116 (DAKO Corp., Carpinteria, Calif.). Analysis of fluorescence was performed on a FACScan flow cytometer (Beckton Dickinson, Mountain View, Calif.).

[0161] 4. Cell Cycle Analysis

[0162] The cell cycle was analyzed by flow cytometry after 3 days of culture of NB4 cells in the absence or presence of sodium stibogluconate (250 µg/ml) or ATRA (1 µM). Briefly, the cells were fixed in cold ethanol and incubated for 30 minutes at 4° C. in the dark with a solution of 50 mg/ml propidium iodide, 1 mg/ml RNase and 0.1% NP-40. Analysis was performed immediately after staining using the CELLFIT program (Becton Dickinson, Mountain View, Calif.).

5. Detection of Apoptotic Cells by Annexin V/Propidium Iodide Assay.

Annexin V staining of exposed membrane phospholipid phosphatidylserine (PS) was done using the Annexin V assay kit (Pharmingen, San Diego, Calif.). Briefly, NB4 cells were cultured in the 10% FCS RPMI 1640 medium in the absence or presence of sodium stibogluconate (250 µg/ml) or ATRA (1 µM for 3 days. Cells were then washed in PBS twice and stained in binding buffer (10 mM Hepes, pH 7.4; 140 mM NaCl; 2.5 mM CaCl₂) containing Annexin V-FITC and propidium iodide for 15 min. The reaction was stopped by adding 10 volumes of binding buffer and analyzed by FACS (Becton Dickinson Facsvantage).

B. Results

1. Sodium Stibogluconate Induced Differentiation of AML Cell Line NB4 in a Dose- and Time-Dependent Manner.

NB4 is a human AML cell line derived from an APL patient and can be induced to differentiate into granulocytes by ATRA. To explore the potential of sodium stibogluconate in differentiation induction therapy for AML, the activity of the drug was initially determined by inducing differentiation of NB4 cells into more mature granulocyte-like cells by NBT reduction assays and CD11b antigen expression.

2. Sodium Stibogluconate Induced NB4 Cell Differentiation in Dose- and Time-Dependent Manner as Indicated by the Increase of NBT Positive Cells in the Presence of the Drug.

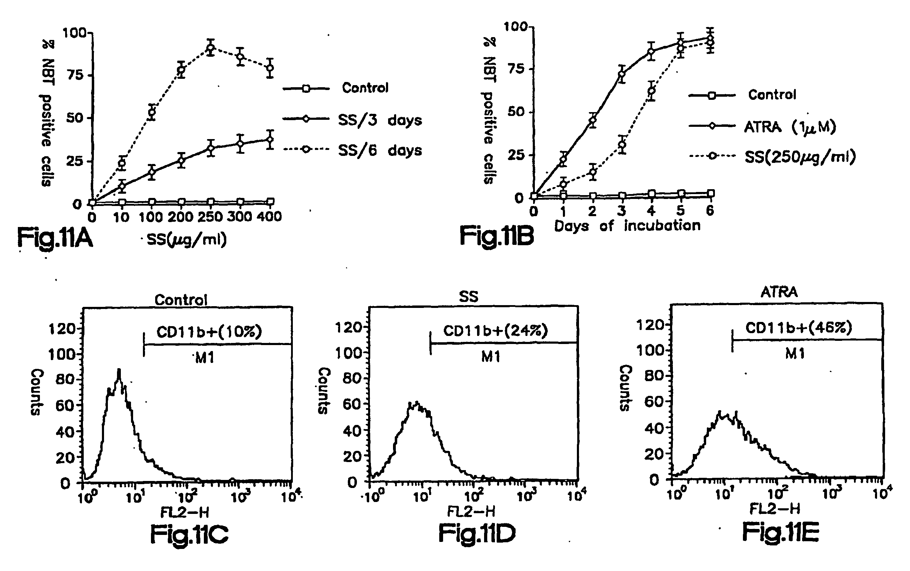

Sodium stibogluconate had a differentiation induction activity at all of the dosages (10 to 400 µg/ml) that were tested in day 3 or day 6 culture (FIG. 11A). The optimal dosage was at 250 µg/ml which induced 87% differentiation of NB4 cells cultured in the presence of sodium stibogluconate for 6 days (FIG. 11A). At this dosage, sodium stibogluconate-induced NB4 cell differentiation was detectable after cells were treated with the drug for the first 24 hours, increased further during the following days and reached 87% by day 6 (FIG. 11B). NB4 cells treated with ATRA (1 µM) for 6 days also reached a similar degree of cell differentiation under comparable conditions (FIG. 11B). Sodium stibogluconate-induced NB4 cell differentiation was further confirmed by the increase of CD11b positive cells from 10% in the control to 24% in NB4 cells cultured in the presence of sodium stibogluconate (250 µg/ml) for 3 days (FIG. 11C).

3. Sodium Stibogluconate-Induced NB4 Cell Differentiation Associates with Cell Growth Arrest at S Phase and Increased Cell Death.

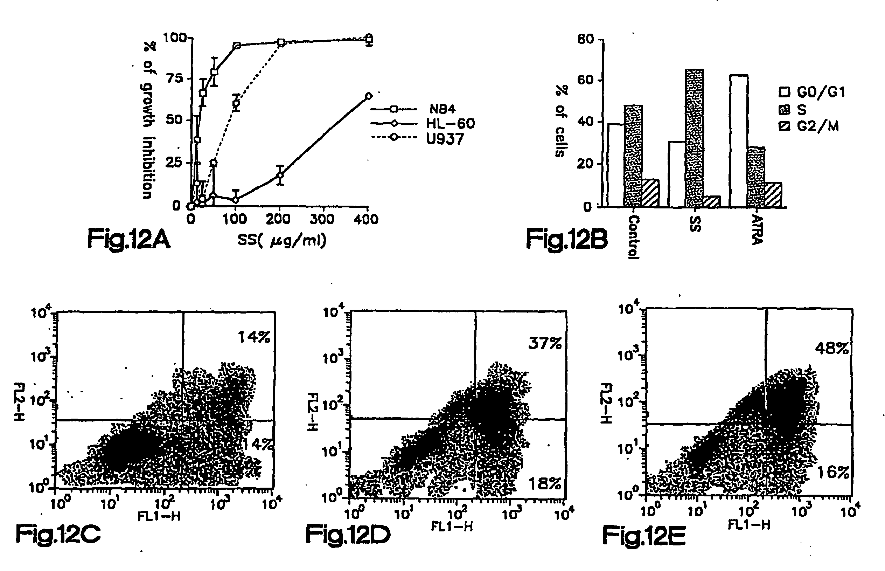

The effect of sodium stibogluconate on NB-4 cell growth by MTT assays was determined. Proliferation of NB4 cells was markedly inhibited in the presence of sodium stibogluconate at all the dosages that were examined (12.5-400 µg/ml) (FIG. 12A). Cell DNA content analysis (FIG. 12) showed a significant increase of cells at S phase in the NB4 cells treated with sodium stibogluconate (250 µg/ml) for 3 days (FIG. 12B). In contrast, NB4 cells cultured in the presence of ATRA (1 µM) for 3 days were arrested at GI phase (FIG. 12B), consistent with a previous report (Idres et al., Cancer Res. 61, 700 (2001)). A substantial population of NB4 cells cultured in the presence of sodium stibogluconate (250 µg/ml) for 6 days was stained positive by Annexin V, suggesting that the cells were dying through apoptosis (FIG. 12C). These results demonstrated that sodium stibogluconate induced NB4 cell growth arrest at S phase and had a cytotoxic effect against the cells.

4. Sodium Stibogluconate-Induced NB4 Differentiation is Irreversible and Requires Continuous Exposure to the Drug for Optimal Induction.

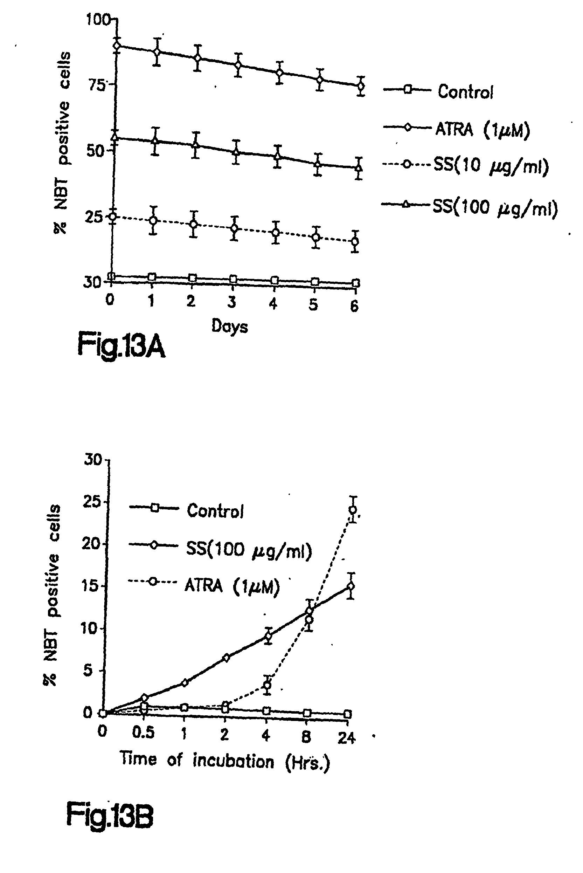

We next investigated whether sodium stibogluconate-induced NB4 differentiation would be reversed in the absence of the sodium stibogluconate. NB4 cells cultured in the presence of sodium stibogluconate (10 µg/ml or 100 µg/ml) for 6 days were washed and resuspended in medium without sodium stibogluconate. The cells were then cultured for 6 days with the numbers of NBT-positive cells determined daily. As shown in FIG. 13A, the percentage of NBT-positive cells remained largely consistent during the 6 day period, demonstrating that sodium stibogluconate-induced NB4 differentiation was not reversed in the absence of sodium stibogluconate. Under comparable conditions, ATRA-induced NB4 differentiation showed a similar characteristic as previously reported (Lanotte et al., Blood 95, 85 (1991)).

To determine whether induction of NB4 cell differentiation requires long term exposure to sodium stibogluconate, NB4 cells were cultured in the presence of sodium stibogluconate (100 µg/ml) for 0.5 to 24 hours, then washed and cultured in medium without sodium stibogluconate for 6 days prior to NBT staining. A linear increase of NBT-positive cells was detected in NB4 cells exposed to sodium stibogluconate for 0.5 to 24 hours with maximal increase (16%) at 24 hours (FIG. 13B). Thus, NB4 cell differentiation was inducible following short exposure to sodium stibogluconate. However, the 16% NBT-positive cells induced by exposing to sodium stibogluconate for 24 hours was substantially less than the 52% level in NB4 cells cultured in the presence of sodium stibogluconate (100 µg/ml) for 6 days (FIG. 11A). Since the percentage of differentiated cells in the culture was directly related to the length of exposure time to sodium stibogluconate (FIG. 11B), the results together indicated that optimal induction of NB4 cell differentiation by sodium stibogluconate requires continuous drug exposure. Similarly, NB4 cell differentiation induced by short exposure to the ATRA (FIG. 13B) was modest in comparison to that of long term exposure (FIG. 11B).

5. Sodium Stibogluconate Induces Differentiation of HL-60 and U93 7 Cell Lines.

To investigate whether the differentiation induction activity of sodium stibogluconate was unique to NB4 cells, the effect of sodium stibogluconate in AML cell lines HL-60 and U937 was examined. HL-60 and U937 cells were cultured in the absence or presence of various amounts of sodium stibogluconate for different times. The percentage of NBT-positive cells in the culture was determined as an indicator of cell differentiation.

Sodium stibogluconate induced differentiation of HL-60 and U937 cells in a dose-and time-dependent manner (FIG. 14). The optimal dosage of sodium stibogluconate in inducing differentiation of HL-60 and U937 cells was 400 µg/ml under the experimental conditions in day 6 culture (FIG. 14A and 14C). At this dosage, the sodium stibogluconate-induced differentiation (approximately 60%) of HL-60 and U937 cells was less than that induced by ATRA (90% for HL60 and 72% for U937) in day 6 culture (FIG. 14B and 14D). Similar to NB4 cells, the percentage of differentiated cells of HL-60 and U937 increased proportionally with prolonged culture in the presence of sodium stibogluconate (FIG. 14B and 14D), indicating a requirement of continuous drug exposure for optimal differentiation induction. The PTPase inhibitor also showed a growth inhibition activity against the two AML cell lines. At the optimal dosage (400 µg/ml) of the drug for differentiation induction in the two cell lines, sodium stibogluconate achieved 97% growth inhibition of U937 cells and 63% inhibition of HL-60 cells in day 6 cultures (FIG. 12A).

6. Sodium Stibogluconate-Induced Differentiation of HL-60 and U937 is Augmented by GM-CSF.

The effect of sodium stibogluconate in combination with GM-CSF in inducing differentiation of HL-60 and U937 cells was determined. HL-60 and U937 cells were cultured in the presence of sodium stibogluconate (400 µ/ml), GM-CSF (25 ng/ml) or both for 1-6 days with the percentage of NBT-positive cells determined daily.

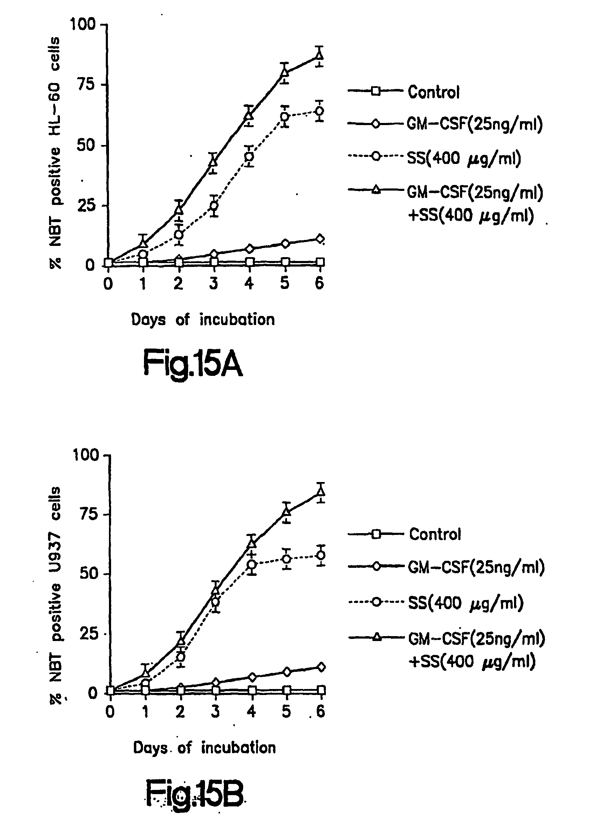

Sodium stibogluconate-induced differentiation of HL-60 and U937 was augmented by GM-CSF to levels nearly equal or higher than those induced by ATRA (FIG. 15). Consistent with previous results reported at James et al., Leukemia 11, 1017 (1997), GM-CSF alone showed a minor effect on HL-60 (FIG. 15A) and U937 (FIG. 15B) differentiation, with maximal increase of NBT-positive cells (8-10%) at day 6. Interestingly, the percentage ofNBT-positive cells in HL-60 cultured in the presence of GM-CSF and sodium stibogluconate both was increased to 83% comparing to 60% with sodium stibogluconate alone (FIG. 15A) or 90% with ATRA alone (FIG. 14B). More dramatically, the combination of GM-CSF and sodium stibogluconate in U937 cells induced 80% cell differentiation, which was higher than that of sodium stibogluconate alone (55%) (FIG. 15B) or ATRA alone (73%) (FIG. 14D). In contrast, GM-CSF alone showed no detectable effect on NB-4 cell differentiation, consistent with a previous report, and failed to augment sodium stibogluconate-induced NB4 cell differentiation under comparable conditions.

C. Discussion

These data demonstrate that sodium stibogluconate, a drug previously used for leishmaniasis and found to be a PTPase inhibitor, induces differentiation of AML cell lines NB4, HL-60 and U937 in vitro. These data showed that sodium stibogluconate induces granulocyte-like maturation of NB4, HL-60 and U937 cells as indicated by the increase of NBT-positive cells and by the increased expression of CD11b surface marker (NB4). This differentiation induction activity of the drug was detectable at low dosage of the drug following relatively short exposure. With prolonged exposure at optimal dosages, sodium stibogluconate induces differentiation levels of NB4 cells comparable to those induced by ATRA. High levels of differentiation of HL-60 and U937 cells similar to those induced by ATRA were also achieved by optimal dosage of sodium stibogluconate in combination with GM-CSF. The data further demonstrate that sodium stibogluconate-induced differentiation is irreversible and associates with growth arrest and cell death via, probably, apoptosis. These results demonstrated a marked differentiation induction activity of the drug in AML cell lines in vitro and indicated sodium stibogluconate as a candidate in differentiation induction therapy in AML treatment.

These results suggested that sodium stibogluconate may be effective in inducing differentiation of AML cells of different FAB classes. This is indicated by its differentiation induction activity in the AML cell lines that represent M3 (NB4 and HL-60) and M5 (U937) subclasses. It is supported by its effect in inducing differentiation of human AML cell line AML-3, which represents the M2 subclass. Because sodium stibogluconate is a PTPase inhibitor, it is expected that sodium stibogluconate induces differentiation via directly targeting a PTPase or PTPases in AML cells. Such a mechanism apparently functions independently of the PML/RAR-alpha chimeric protein, a major target of ATRA that is degraded in ATRA-treated NB4 cells. This is evident as sodium stibogluconate had no detectable effect on the expression levels of PML/RAR-alpha chimeric protein in NB4 cells and did not synergize with ATRA in differentiation induction. This distinct mechanism of sodium stibogluconate in differentiation induction suggests that sodium stibogluconate may be particularly useful in AML cases unresponsive or developed resistance to ATRA treatment.

It is likely that the key sodium stibogluconate target in AML differentiation is among the PTPases that are relatively insensitive to the drug. This is based on the observation made above of differential sensitivities of PTPases to the inhibitor, with complete inhibition of sensitive PTPases (e.g., SHP-1) by sodium stibogluconate at 10 µg/ml and a similar inhibition of insensitive PTPases at more than 100 µg/ml. And it is supported by the data presented here that the optimal dosage of sodium stibogluconate in inducing AML cell differentiation is at levels more than 100 µg/ml. In this regard, the involvement of amplification and overexpression of HePTP in AML is interesting and suggests the PTPase as a candidate target of the drug. Characterization ofPTPase expression profiles of sodium stibogluconate-sensitive and sodium stibogluconate-resistant AML cell lines will help to identify the putative PTPase target in AML differentiation.

The optimal dosage of sodium stibogluconate for inducing differentiation of NB4 and HL-60IU937 cells is 250 µg/ml and 400 µg/ml respectively. The standard dosage for leishmaniasis treatment is 10-20 mg/kg/day resulting in 10 µg/ml or more serum levels. However, higher drug dosages may be clinically achievable and tolerated since doses as high as 80-143 mg/kg had been used in leishmaniasis treatment. Nevertheless, even standard dosage of sodium stibogluconate may have certain therapeutic benefit as the drug at lower dosages (e.g., 10 µg/ml) showed differentiation induction activity in AML cells (FIG. 9).

The observation that GM-CSF augments sodium stibogluconate-induced differentiation of HL-60 and U937 suggested the potential clinical use of the two reagents in combination in AML treatment (FIG. 15). Such an interaction between sodium stibogluconate and GM-CSF is not unexpected given the activity of the drug in augmenting GM-CSF signaling and the biological effect of the cytokine on myeloid cells. However, combined usage of sodium stibogluconate and GM-CSF may only benefit a subgroup of AML cases as a positive interaction between the two reagents in differentiation induction was not detected in NB4 cells, which were not responsive to the cytokine. Moreover, sodium stibogluconate may also interact with other cytokines in differentiation induction of AML cells. GM-CSF and IFNs were reported to potentiate differentiation of AML cells. Like GM-CSF, the two cytokines signal through the Jak/Stat pathway that could be augmented by sodium stibogluconate.

III. PTPase Inhibitor Sodium Stibogluconate Inhibits the Growth of Human Cancer Cell Lines In Vitro in Synergy with IFNs

To explore the potential of sodium stibogluconate as an anti-tumor drug, its effect on the growth of various human cancer cell lines in vitro was examined. The data demonstrate that sodium stibogluconate, used alone or in combination with IFN-alpha and 1FN-beta, was effective in inhibiting the in vitro growth of different human cell lines of lymphoma, multiple myeloma, leukemia, melanoma, prostate cancer, breast cancer, renal cancer and bladder cancer. Moreover, this anti-cancer activity of sodium stibogluconate was related to the enhancement of tyrosine phosphorylation of specific cellular proteins and the induction of cell apoptosis. The effectiveness of sodium stibogluconate in overcoming IFN-resistance of cancer cells was indicated by the near complete killing by sodium stibogluconate alone or in combination with IFN-alpha of cancer cell lines that showed only partial growth inhibition in response to the cytokine. The broad in vitro anti-cancer activity of sodium stibogluconate indicates its potential as a novel anti-cancer drug as a single agent or in combination with IFN-alpha/-beta. Moreover, the ability of the drug to augment Jak/Stat signaling via targeting Jak/Stat PTPase(s) suggests it will be effective in other therapies of hematopoietic growth factors and cytokines that signal through the Jak/Stat pathway.

A. Materials and Methods

1. Reagents.

Recombinant human IFN-alpha (IFN-alpha-2b, specific activity 2x10⁸ units/mg protein, Schering Plough) and sodium stibogluconate have been described previously (Phatak et al., J. Immunol. 167, 3391 (2001)). Recombinant human IFN-beta (specific activity 2x10⁸ U/mg protein) was obtained from Aeres-Serono (Rockland, Mass.). Antibodies for phosphotyrosine (Upstate Biotechnology, Lake Placid, N.Y.), phosphotyrosine Stat1 and Stat1 (New England BioLab Inc., Beverly, Mass.), SHP-1 and SHP-2 (Santa Cruz Biotechnology, Santa Cruz, Calif.), and .beta.-actin (Pharmacia, Arlington Heights, Ill.) were purchased from commercial sources as indicated.

2. Cells, Cell Culture and Cell Proliferation Assays.

Human cell lines were maintained in RPMI 1640 or DMEM medium supplemented with 10% fetal calf serum (FCS) at 37° C. DS and DR (Fan Dong, the Cleveland Clinic Foundation (CCF)), U266, DU145 and C42 (Alex Almasan, CCF), Peer (John Winfield, University of North Carolina), H9 (ATCC), WM9 and WM35 (Ernest Borden, CCF), MDA231 and MDA435 (Graham Casey, CCF), WiT49-N1 (Bryan Williams, CCF), RC45 and 5637 (S. K. Bandyopadhyay, CCF) were employed in the studies.

For cell proliferation assays, cells were grown in 10% FCS culture medium containing various amounts of IFNs and/or sodium stibogluconate in 96 well plates and cultured at 37° C. for 3 or 6 days as indicated. The numbers of viable cells in proliferation assays were determined by MTT assays as described in Phatak et al., J. Immunol. 167, 3391 (2001).

3. Drug Interaction Analysis.

Median effect analysis (Chou et al., Adv. Enzyme Regul. 22, 27 (1984)), which provides the most general form of studying the interactions between drugs, was utilized to analyze the interaction between sodium stibogluconate and IFN-alpha or IFN-beta. Dose response curves were generated for each drug alone, and also the combinations. Median effect plots were generated for IFNs alone, sodium stibogluconate alone, and the combination. The combination index (CI) was determined and plotted vs. fraction affected (FA). Data were analyzed in both modes, mutually exclusive and mutually nonexclusive. The interaction between two mutually nonexclusive drugs is described by the Equation CI=D₁/Dₓ₁+D₂/Dₓ₂+D₁D₂/Dₓ₁Dₓ₂, where Dₓ₁ and Dₓ₂ are the doses of drug 1 and drug 2 that are required to inhibit growth x %. D₁ and D₂ in combination also inhibit growth x % (i.e. drug 1 and drug 2 are isoeffective). When CI<1, drugs are synergistic, when CI=1, drugs are additive, and when CI>1, drugs are antagonistic.

4. Detection of Apoptotic Cells by Annexin V/Propidium Iodide Assay.

Annexin V staining of exposed membrane phospholipid phosphatidylserine (PS) was done using the Annexin V assay kit (Pharmingen, San Diego, Calif.). Briefly, U266 or WM9 cells were cultured in the 10% FCS RPMI 1640 medium in the absence or presence of sodium stibogluconate, IFN-alpha or both for 3 days. Cells were then washed in PBS twice and stained in binding buffer (10 mM Hepes, pH 7.4; 140 mM NaCl; 2.5 mM CaCl₂) containing Annexin V-FITC and propidium iodide for 15 min. The reaction was stopped by adding 10 volumes of binding buffer and analyzed by FACS (Becton Dickinson Facsvantage) or fluorescent microscopy.

5. Induction of Stat1 Tyrosine Phosphorylation by IFN-Alpha and/or Sodium Stibogluconate.

For induction of Stat1 tyrosine phosphorylation by IFN-alpha in the absence or presence of sodium stibogluconate, cells grown in 10% FCS RPMI 1640 medium at 37° C. were stimulated with IFN-alpha (50 u/ml) for various time points and treated with or without sodium stibogluconate for 5 minutes prior to termination by lysing the cells in cold lysis buffer (1% NP-40; 50 mM Tris, pH 7.4; 100 mM NaCl; 1 mM EDTA, 10% glycerol, 10 mM sodium molybdic acid and 4 mM AEBSF).

6. Cell Lysate Preparation, SDS-PAGE and Western Blotting.

Cell lysates were prepared by lysing cells in cold lysis buffer for 30 min and cleared by centrifuging at 14,000 rpm at 4° C. for 15 min. For SDS-PAGE, cell lysates were mixed with equal volume of 2X SDS-PAGE sample buffer, heated at 90° C. for 5 min and separated in 10% SDS-PAGE gels. Cellular proteins in SDS-PAGE gels were transferred to nitrocellulose membrane (Schleicher & Schuell, Keene, N.H.), blocked in 5% milk, probed with antibodies and detected by using an enhanced chemiluminescence kit (ECL, Amersham, Arlington Heights, Ill.).

B. Results

1. Sodium Stibogluconate Inhibits the In Vitro Growth of Human Cell Lines of Hematopoietic Malignancies and Augments IFN-Alpha-Induced Cell Growth Inhibition.

Sodium stibogluconate markedly augmented IFN-alpha-induced growth inhibition of the IFN-alpha-resistant lymphoma cell line DR. DR and DS cell lines were derived from the parental human lymphoma cell line Daudi and were resistant or sensitive to IFN-alpha respectively. Consistent with their sensitivity to IFN-alpha, DS cells cultured in the presence of IFN-alpha (1,000 u/ml) were almost completely eliminated by day 3 (FIG. 16C). In contrast, IFN-alpha treatment resulted in only 19% growth inhibition of the DR cells (FIG. 16A and B). Importantly, this IFN-alpha-induced DR cell growth inhibition was increased to 46-69% in the presence of various amounts of sodium stibogluconate (FIGS. 16A and B). Augmentation of IFN-alpha-induced growth inhibition by sodium stibogluconate was also observed in prolonged culture of DR cells for 6 days (FIG. 16D), in which the 39% of IFN-alpha-induced growth inhibition was increased to 80% and 92% in the presence of sodium stibogluconate at 12.5 µg/ml and 25 µg/ml respectively. Interestingly, the PTPase inhibitor by itself showed a marked activity against DR cells at higher dosages: it almost completely eliminated proliferation of DR cells (95-99%) in the day 6 culture at 50 µg/ml and 100 µg/ml as a single agent (FIG. 16D). Sodium stibogluconate by itself showed a modest activity against the DS cells (FIG. 16C).

This initial observation of marked growth inhibition of DR cells by sodium stibogluconate alone or in combination with IFN-alpha prompted a determination of its effect against other cell lines of human hematopoietic malignancies. U266 is cell line of human multiple myeloma, a disease currently treated with IFN-alpha. Again, augmentation of IFN-alpha-induced cell growth inhibition of U266 cells was detected with a substantial growth inhibition activity of the drug by itself (FIG. 16E). Various degrees of augmentation of IFN-alpha growth inhibition activity by sodium stibogluconate were also observed in other cell lines ofT-lymphoma (H9) and T-ALL (Peer) (Table 1).

2. Sodium Stibogluconate Inhibits the In Vitro Growth of Human Cell Lines of Non-Hematopoietic Malignancies and Augments IFN-Alpha-Induced Growth Inhibition.

The effect of sodium stibogluconate in augmenting IFN-alpha-induced growth inhibition and in causing growth inhibition by itself in cell lines of human hematopoietic malignancies suggested potential activity of the drug against non-hematopoietic cancer cells as the drug has inhibitory activity against PTPases (e.g., PTP1B and SHP-2) that express in various non-hematopoietic tissues.

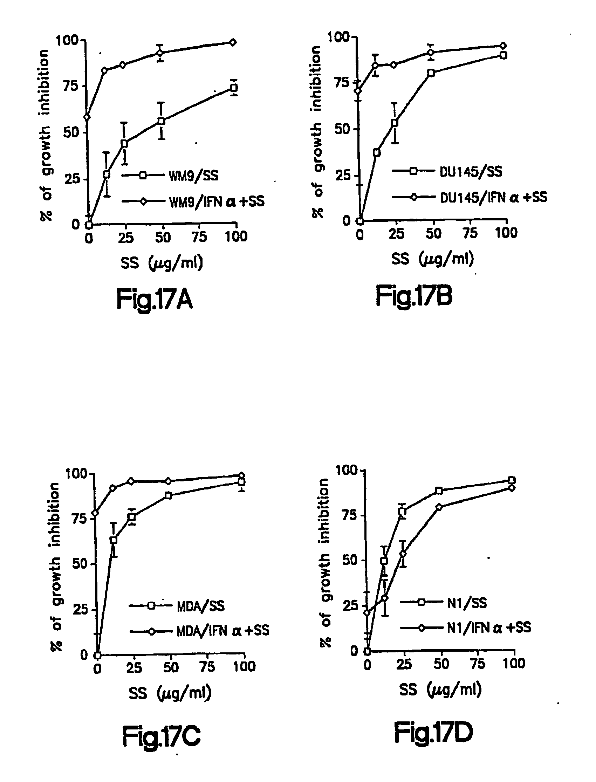

Several solid tumor cell lines were found to be sensitive to the PTPase inhibitor alone or in combination with IFN-alpha. IFN-alpha-induced growth inhibition of WM9 (melanoma), MDA231 (breast cancer) and DU145 (prostate cancer) was augmented by sodium stibogluconate (FIG. 17A, B and C). Like the DR lymphoma cell line, these tumor cell lines were sensitive to the PTPase inhibitor as a single agent, which at 50 µg/ml and 100 µg/ml dosages killed all cells in day 6 culture (FIG. 17). The Wilms tumor cell line WiT49-N1 was also sensitive to sodium stibogluconate although its growth inhibition activity was not enhanced by IFN-alpha (FIG. 17D).

Further studies of the drug in additional cell lines demonstrated that sensitivity to sodium stibogluconate was not tumor type-specific but unique to individual cell lines. In contrast to the sensitive WM9 melanoma cell line, the WM35 melanoma cell line was minimally affected by sodium stibogluconate (Table 1). Unlike the DU145 prostate cancer cell line, the C42 prostate cancer cell line was highly resistant to the inhibitor (Table 1). Growth responses of several other human tumor cell lines to IFN-alpha and/or sodium stibogluconate were also determined (Table 1).

3. Sodium Stibogluconate Augments IFN-Alpha- and IFN-Beta-Induced Growth Inhibition of WM9 Cells in a Synergistic Manner.

To further investigate whether augmentation of IFN-alpha-induced cell growth inhibition by sodium stibogluconate was unique to this drug combination, the effect of the drug on IFN-alpha- or IFN-beta-induced growth inhibition of the WM9 cell line of human melanoma, which is currently treated by the cytokines, was compared.

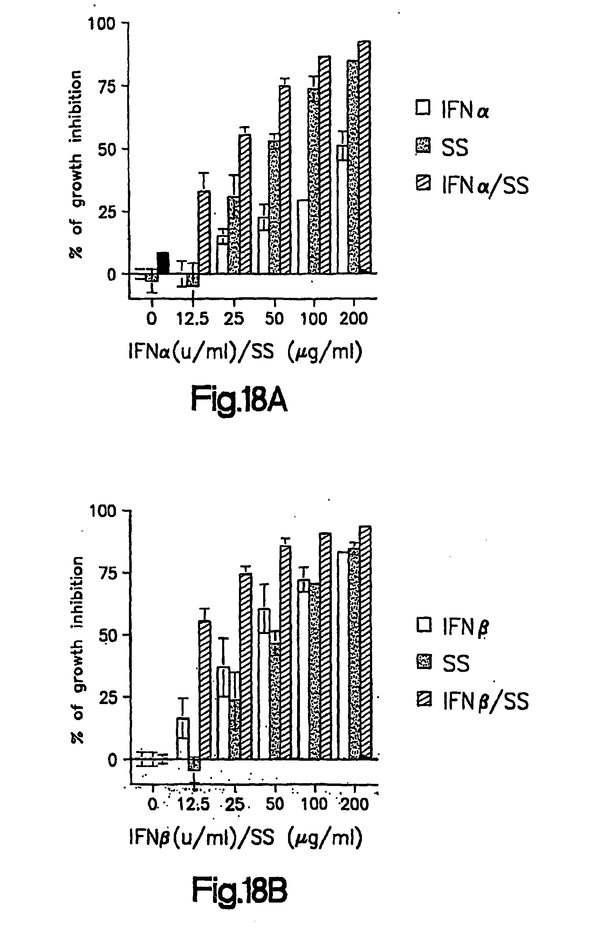

The growth of WM9 cells was suppressed by IFN-alpha (FIG. 18A) and, more potently, by IFN-beta (FIG. 18B). In the presence of sodium stibogluconate, IFN-alpha- and IFN-beta-induced growth inhibition was greatly enhanced (FIG. 18). This augmentation of IFN-alpha/-beta-induced growth inhibition by sodium stibogluconate was most dramatic at lower dosage levels of sodium stibogluconate (12.5-50 µg/ml) and the IFNs (12.5-50 units/ml) but was also detectable in the higher dosage range (FIG. 18). Thus, sodium stibogluconate was effective in augmenting the growth inhibition activity of IFN-alpha and IFN-beta against WM9 cells.

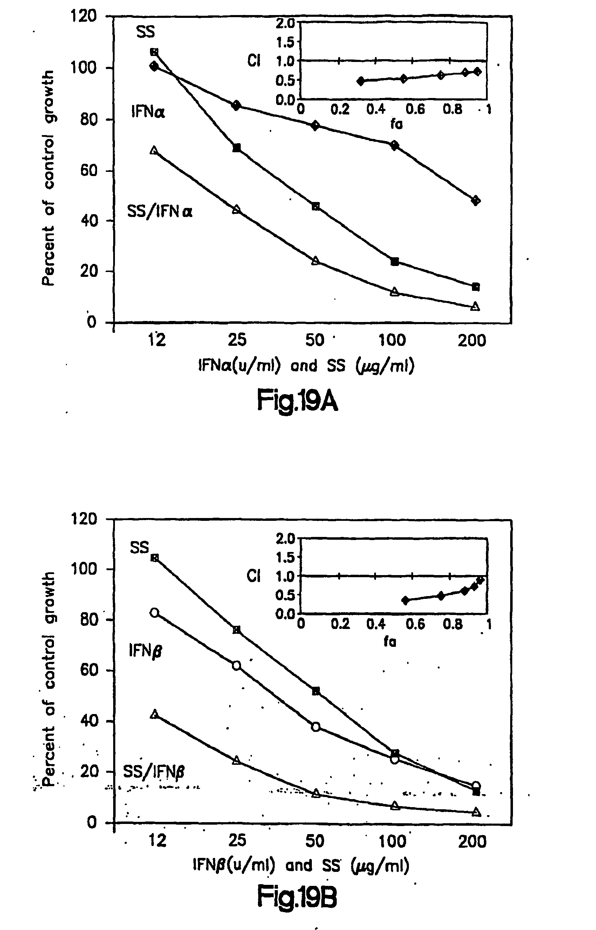

To determine the nature of the drug interaction in the IFN-alpha/sodium stibogluconate and IFN-beta/sodium stibogluconate combinations, data represented by FIG. 18 were subject to median effect analysis to derive combination index (CI) values that define drug interaction as synergy (CI<1), additivity (CI=1) or antagonism (CI>1). The results, calculated in both modes of mutually exclusive and nonexclusive, demonstrate that the drug interaction in the combinations of IFN-alpha/sodium stibogluconate (FIG. 19A) and IFN-beta/sodium stibogluconate (FIG. 19B) are synergistic at all doses tested, characterized by a CI value less than 1. Since the growth inhibition of DR, DU145 and MDA231 cells achieved by the combination of sodium stibogluconate and IFN-alpha was similar to that of the WM9 cells (FIG. 17), the results also suggested a synergistic interaction for the two agents in those cell lines.

4. Growth Inhibition of Human Cancer Cell Lines by Sodium Stibogluconate Associates with Induction of Apoptosis.

The marked growth inhibition of tumor cell lines by sodium stibogluconate alone and/or in combination with IFN-alpha indicated induction of cell death by the PTPase inhibitor. Therefore the numbers of apoptotic cells of U266 and WM9 cell lines grown in the presence of sodium stibogluconate, IFN-alpha or both, were determined.

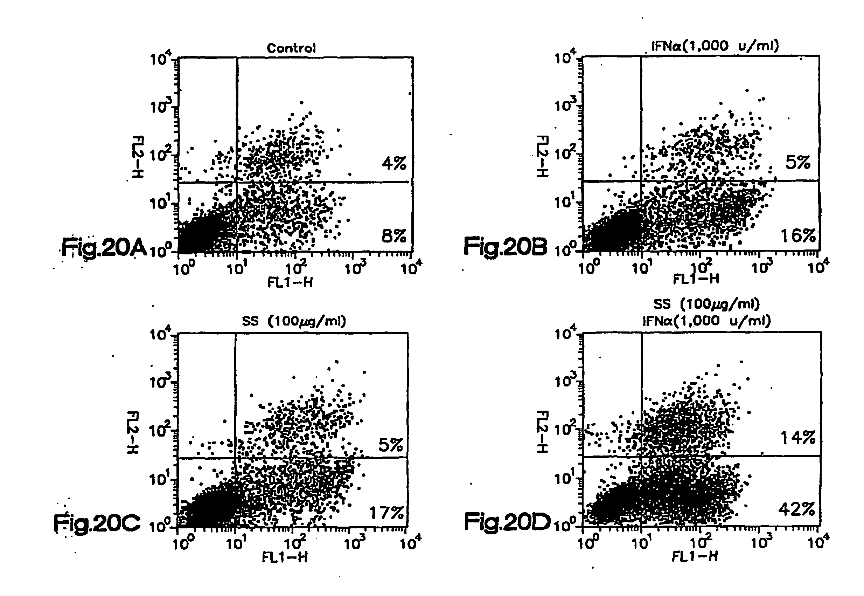

Increased apoptosis of U266 cells was detected in the presence of sodium stibogluconate alone and, more dramatically, of the inhibitor and IFN-alpha both (FIG. 20). In the presence of sodium stibogluconate (100 µg/ml), the percentage of apoptotic cells was increased to 17% (FIG. 20C) from 8% (FIG. 20A). IFN-alpha (1000 units/ml) induced 16% apoptosis (FIG. 20B). When both sodium stibogluconate and IFN-alpha were present, the number of apoptotic cells increased to 42% (FIG. 20D). Evaluated by fluorescent microscopy, WM9 cells in the presence of sodium stibogluconate, IFN-alpha or both were increased to 11%, 15% or 31% respectively from 5% (control). Thus, growth inhibition of these tumor cell lines by sodium stibogluconate and IFN-alpha was mediated at least in part by inducing apoptosis.

5. Augmentation of IFN-Alpha-Induced Cell Growth Inhibition by Sodium Stibogluconate Correlates with Enhanced Stat1 Tyrosine Phosphorylation.

To investigate the signaling mechanism of sodium stibogluconate in augmenting 1FN-alpha-induced cell growth inhibition, the effect of sodium stibogluconate on IFN-alpha-induced tyrosine phosphorylation of stat1, which clearly mediates the anticellular effects of the cytokine, was determined.

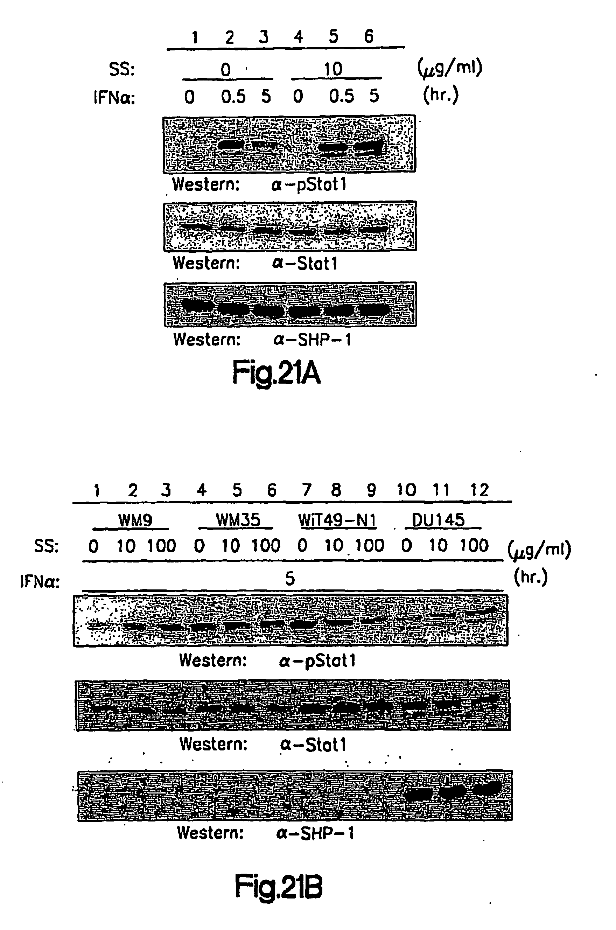

IFN-alpha-induced Stat1 tyrosine phosphorylation was enhanced in the presence of sodium stibogluconate in cell lines (DR, WM9 and DU145) in which a synergy of IFN-alpha and sodium stibogluconate in growth inhibition was detected (FIGS. 16 and 17). In the absence of sodium stibogluconate, Stat1 tyrosine phosphorylation in DR cells was induced by IFN-alpha within 30 min and decreased by 5 hours post-stimulation (FIG. 21A, lanes 1-3). In the presence of sodium stibogluconate (10 µg/ml), Stat1 tyrosine phosphorylation at 30 min post-stimulation was approximately two folds greater than control (FIG. 21A, lanes 2 and 5) and remained elevated for 5 hours (FIG. 21A). Enhanced Stat1 tyrosine phosphorylation at 5 hours post-stimulation by IFN-alpha was also detected in WM9 and DU145 cell lines cultured in the presence of sodium stibogluconate (FIG. 21B). In contrast, sodium stibogluconate failed to enhance IFN-alpha-induced Stat1 tyrosine phosphorylation in WM35 and WiT49-N1 cell lines (FIG. 21B) in which no antiproliferative synergy between IFN-alpha and sodium stibogluconate was detected (Table 1 and FIG. 17D). In the absence of IFN-alpha, sodium stibogluconate failed to induce Stat1 tyrosine phosphorylation by itself in DR cells (FIG. 21A). IFN-alpha-induced Stat1 tyrosine phosphorylation in WiT49-N1 cells was not increased in the presence of sodium stibogluconate (FIG. 21B).

To assess the involvement of SHP-1, which is known to regulate Jak/Stat phosphorylation in hematopoietic cells, the expression of the PTPase in the tumor cell lines (FIG. 21) was determined. As expected, SHP-1 protein was easily detected in DR cells (FIG. 21A). However, SHP-1 protein was undetectable in the two melanoma cell lines although it was present in the Wilms tumor cell line (WiT49-N1) and the prostate cell line (DU145) (FIG. 21B). Thus, the enhancement of IEN-alpha-induced Stat1 tyrosine phosphorylation in WM9 cells occurred in the absence of SHP-1 and may be mediated by other PTPases sensitive to the PTPase inhibitor.

C. Discussion

Resistance of cancer cells to IFN-alpha and IFN-beta is a major problem that limits the clinical application of these cytokines in anti-cancer therapies. Although the mechanism of IFN-resistance of cancer cells is not fully understood, reduced IFN signaling is often detected in cancer cells and believed to be an important factor. Therapeutic reagents that augment IFN signaling may help to overcome such resistance in cancer cells but have not been reported yet.

These data provide evidence that sodium stibogluconate augments IFN signaling and can overcome IFN-resistance in various human cancer cell lines. Augmentation of IFN-alpha signaling by the drug was clearly demonstrated by its enhancement of WN-alpha-induced Stat1 phosphorylation. This activity was detectable at its therapeutic concentration (10-20 µg/ml), a concentration that is clinically well tolerated. Moreover, the activity of the drug in augmenting of IFN-alpha signaling was effective in overcoming IFN-resistance as it was accompanied by augmentation of IFN-alpha-induced growth inhibition of various human cancer cell lines.

The drug at 25-100 µg/ml was extremely effective at overcoming IFN-resistance of cell lines that were only partially inhibited by IFN-alpha as a single agent. This was well-illustrated by the complete elimination of WM-9 melanoma cells by the drug and IFN-alpha in combination while the two agents individually achieved only 75% and 58% growth inhibition respectively. Similarly, the drug at 25 µg/ml combined with IFN-alpha achieved near complete elimination of MDA231 breast cancer cells compared to 65% and 79% growth inhibition by the two agents individually. This in vitro anti-cancer activity of the drug alone or in combination with IFN-alpha was shown to involve induction of apoptosis in WM9 cell and U266 cells. Although the standard dosage for leishmaniasis treatment is 10-20 mg/kg/day resulting in 10 µg/ml or more serum levels, higher drug dosages may be clinically achievable and tolerated. Doses as high as 850 mg/kg/day have been used in leishmaniasis treatment.

The finding that sodium stibogluconate also augmented IFN-beta-induced growth inhibition suggested that the drug may improve the efficacy of IEN-beta therapies in the treatment of cancer as well as several other diseases (e.g., hepatitis B and multiple sclerosis) that are currently treated with the cytokine. Moreover, it provided additional evidence that among the targets of the PTPase inhibitor are Jak/Stat PTPases which down regulate cytokine signaling by dephosphorylating Jak/Stat proteins, a hypothesis based on the previous finding of drug augmentation of cell responses to IL-3 and GM-CSF that signal through the Jak/Stat pathway like the IFNs. PTPase SHP-1 and CD45 are known to down-regulate Jak/Stat tyrosine phosphorylation in hematopoietic cells. As the expression of SBP-1 (FIG. 21B, lanes 1-3) and CD45 was not detectable in WM9 cells in which IFN-alpha-induced Stat1 phosphorylation was augmented by the drug, the results indicate the existence of other Stat1-regulatory PTPase(s) as the drug target in these cells. But the data does not exclude the involvement of SHP-1 or CD45 as drug targets in hematopoietic cells. This mechanism of the drug targeting Jak/Stat PTPase(s) predicts that the PTPase inhibitor will have a similar activity in augmenting the signaling of other cytokines signaling through the Jak/Stat pathway. Many cytokines signaling through Jak/Stat pathway (e.g., IL-2, IL-4, and IL-12) have been used in anti-cancer therapies, which may be improved in combination with the PTPase inhibitor.

The interaction of sodium stibogluconate with IFN-alpha and IFN-beta in growth inhibition of WM9 melanoma cells was clearly synergistic. Such a synergy between the drug and IFNs is consistent with the augmentation of IFN-induced Stat1 phosphorylation by the PTPase inhibitor. Although several other drugs have been shown to synergize with IFNs, sodium stibogluconate is the first one that works through targeting molecules in the IFN signaling pathway.

The data demonstrate that sodium stibogluconate has marked growth inhibitory activity against human cancer cell lines in vitro. This activity was most dramatic at higher dosages (25-100 µg/ml) with a substantial activity detectable at therapeutic concentration. For instance, sodium stibogluconate at 100 µg/ml achieved complete or near complete killing of cells in day 6 culture of the DR, DU145, MDA231 and WiT49-N1 cell lines. Induction of cell apoptosis may play a role in the killing of the cancer cells as indicated by the increased apoptosis of WM9 and U266 cells in the presence of sodium stibogluconate at 100 µg/ml. Unlike the synergy of the drug at therapeutic concentration with IFNs that was mediated via targeting Jak/Stat PTPases to augment IFN-induced Jak/Stat phosphorylation and signaling, this activity is likely mediated by other PTPases independent of the Jak/Stat pathway as indicated by the failure of sodium stibogluconate alone to induce Stat1 phosphorylation at 10 pg/ml (FIG. 21A, lane 4) or at higher concentration.

The sensitivity of certain human cancer cell lines to sodium stibogluconate by itself suggests potential effectiveness of sodium stibogluconate as a single agent in cancer treatment. In this regard, the finding that drug sensitivity is unique to individual cancer cell lines instead of tumor type-specific underscores the importance of identification of markers of drug-sensitivity and -resistance in cancer cells. It is likely that drug-resistance may be due to the absence of target PTPases or PTPase substrates in drug-resistant cells which have adapted to grow without these molecules.

IV. Sodium Stibogluconate Synergizes with IFN-Alpha to Eradicate Human Melanoma WM9 Tumors and Markedly Suppress Human Prostate Carcinoma DV145 Tumors In Vivo in Nude Mice.

To address whether sodium stibogluconate has anti-cancer activity in vivo at a dosage that is clinically achievable and tolerated, the efficacy of sodium stibogluconate, as a single agent or in combination with IFN-alpha, against human melanoma WM9 and human prostate carcinoma DU145 xenografts in nude mice, was determined.

A. Methods

WM9 and DU145 cell lines were chosen for the study based on the following considerations: 1) the two cell lines were found in studies described above to be sensitive to sodium stibogluconate as a single agent or in combination with IFN-alpha (FIGS. 17A and B); 2) both cell lines are known to be tumorigenic in nude mice, 3) the cell lines represent human malignancies that are major health threats with no effective treatment; 4) IFN-alpha is used in the treatment of melanoma and prostate cancer with modest outcome, which may be significantly improved by combinational therapy with sodium stibogluconate that synergizes with the cytokine.

Nude mice bearing WM9 or DU145 xenografts were treated with IFN-alpha (500,000 U, s.c., daily), sodium stibogluconate (12 mg Sb, s.c., daily) or both. The amount of IFN-alpha used for the treatment is comparable to the dosages used in similar studies. The dosage of sodium stibogluconate corresponds to approximately 440 mg Sb/kg body weight (average mouse body weight 27 g), substantially higher than the standard therapeutic dose of 20 mg Sb/kg and the high dose (143 mg Sb/kg) that was clinically used by accident without serious toxicity. This dose of sodium stibogluconate was based on a previous observation in a pilot study that mice could tolerate daily dose of 20 mg Sb (approximately 700-800 mg Sb/kg). An observation that the effect of sodium stibogluconate in inhibiting the growth of the cancer cell lines in vitro was dose-dependent with complete or near complete killing of the cancer cells at 100 pg Sb/ml (or 100 ug Sb/kg) was also considered. In light of the relatively rapid rate of clearance of the drug in vivo, 440 mg Sb/kg dosage was used to ensure the detection of the effectiveness of the drug for this initial study.

For each of the cell lines, each of 16 mice received subcutaneous injection at the chest area of 3 x 10⁶ cells/site (WM9) or 2 x 10⁶ cells/site (DU145), two sites/mouse, on day 0. Mice were separated into four groups of four to receive treatment, injected into the thigh area starting on day 2. Tumor size was measured with a caliper to determine the two perpendicular diameters of each tumor. Tumor volume was calculated using the method of the NCI (length x width 2 in millimeters/2=volume in cubic millimeters).

B. Results

1. Sodium Stibogluconate as a Single Agent has a Marked Anti-Tumor Activity In Vivo and Synergizes with IFN-Alpha to Eradicate Xenografts of Human Melanoma WM9 in Nude Mice.

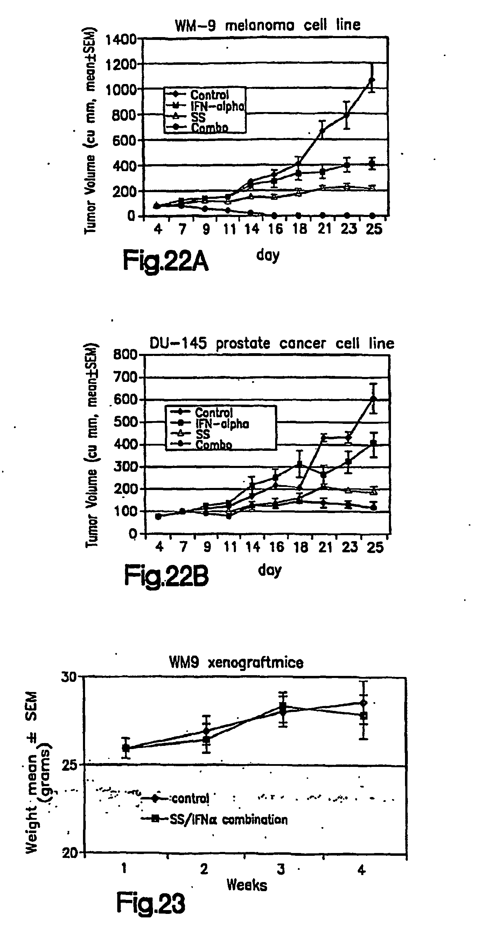

To test the anti-tumor effects of sodium stibogluconate and its synergy with IFN-alpha in vivo, the effect of sodium stibogluconate, IFN-alpha and their combination against xenografts of human WM9 melanoma in nude mice was determined. WM9 cells were inoculated into nude mice that were then subjected to no treatment (control) or treatment for 23 days with single agents or their combination starting on day 2 following inoculation. Tumor volume of WM9 xenografts in the mice was determined during the treatment course as indicators of efficacy of the treatment (FIG. 22A).

WM9 cells in nude mice formed tumors that showed continuous growth in a time dependent manner in the absence of any treatment. Treatment with IFN-alpha alone significantly suppressed WM9 tumor growth in the mice and resulted in an average tumor volume approximately 40% of the control group by the end of the treatment course (FIG. 22A, day 25 data). Interestingly, treatment with sodium stibogluconate alone caused a dramatic tumor growth suppression (tumor volume about 20% of the controls on day 25), superior to that of IFN-alpha treatment under the experimental conditions. Most strikingly, treatment with the combination of sodium stibogluconate and IFN-alpha led to a gradual shrinkage of WIND tumors which were virtually invisible by day 18 (FIG. 22A). This absence of visible tumor in this group of mice continued until the end of the treatment course by day 25. Two mice of this group were observed for additional 8 weeks without treatment. No visible tumor was detected in these mice at the inoculation sites during this additional observation period. Thus the combinational treatment eradicated the pre-formed WM9 tumors in the nude mice.

Statistical analysis of the data demonstrated that the differences of tumor volumes between the groups on day 25 were highly significant (t test: control vs. sodium stibogluconate, IFN-alpha and sodium stibogluconate/IFN-alpha, p<0.01; sodium stibogluconate vs. IFN-alpha, p<0.01; sodium stibogluconate vs. sodium stibogluconate/IFN-alpha, p<0.01). Combinational analysis indicates that the interaction between sodium stibogluconate and IFN-alpha is synergistic.

2. Sodium Stibogluconate Markedly Suppresses the Growth of Xenografts of Human Prostate Carcinoma DU145 in Nude Mice.

To test the anti-tumor effects of sodium stibogluconate and its synergy with IFN-alpha in vivo, the effect of sodium stibogluconate, IFN-alpha and their combination against xenografts of human DU145 melanoma in nude mice was determined. As shown in FIG. 22B, inoculation of DU145 cells in nude mice resulted in formation of tumors that was not significantly suppressed by IFN-alpha monotherapy during the greater part of the treatment duration, consistent with a previous study. Modest anti-tumor activity of the cytokines was detected by the end of the treatment course with the average tumor volume approximately 70% of the control on day 25. In contrast, sodium stibogluconate as a single agent markedly suppressed DU145 tumor growth and resulted in an average tumor volume of approximately 30% of the control by day 25. This anti-tumor activity of sodium stibogluconate was further augmented when the drug was used in combination with IFN-alpha (average tumor volume, 18% of control on day 25). These results together demonstrated that sodium stibogluconate has a marked anti-tumor activity against DU145 xenografts in nude mice and that the drug interacts with IFN-alpha to achieve a striking growth inhibition of DU145 xenografts in nude mice.

3. The Effective Dosage of Sodium Stibogluconate Against WM9 and DU145 Xenografts is Well Tolerated in Nude Mice.

As discussed above, the dosage of sodium stibogluconate used for the treatment of nude mice was 12 mg Sb/mouse, s.c., daily (or approximately 440 mg/kg body weight). This dosage is much higher than the standard dose for leishmaniasis (20 mg Sb/kg, daily). As an initial step to assess the toxicity of such a high dosage of sodium stibogluconate in nude mice, the effect of sodium stibogluconate on the viability and body weights of WM9 xenografts nude mice during the 25 day period of the study was determined.

All of the 16 mice inoculated with WM9 cells survived until the end of the study (day 25) regardless their treatment (control, sodium stibogluconate, IFN-alpha or both, 4 mice/group). The average body weight of the mice subjected to combinational treatment with sodium stibogluconate and IFN-alpha showed no significant difference from that of the control group mice (FIG. 23) or those of the sodium stibogluconate- or IFN-alpha-treatment group during the study period. In addition, no obvious difference was noticed among the 4 groups of mice in their general appearance, feeding or activity. Dissection of two mice from each group of the mice revealed no apparent abnormality of the internal organs. Two mice of the combinational treatment group were observed for additional 8 weeks without treatment. These mice showed no visually obvious abnormality during the period, indicating that the treatment caused no serious long-term side effect.

C. Discussion

These results demonstrate that sodium stibogluconate, as a single agent, showed a significant activity, higher than that of IFN-alpha, against the two types of tumors in vivo. Moreover, sodium stibogluconate synergized with IFN-alpha to eradicate the WM9 tumors in the nude mice with the combinational treatment for 16 days. Sodium stibogluconate was also found to synergize with IFN-alpha to achieve striking growth inhibition of the DU-145 tumors superior to those of the two drugs used alone.

Additionally, the responses of the two tumor cell lines to sodium stibogluconate and/or IFN-alpha in vivo correlated with their responses in vitro (comparing the results in FIGS. 17A and B, and FIG. 22), i.e., the WM9 cell line was more sensitive to the combination treatment of sodium stibogluconate and IFN-alpha in vivo than the DU145 cell line, similar to the above in vitro results. Further, sodium stibogluconate, at the dosage used in the study (12 mg Sb, daily of 440 mg Sb/kg daily), was well tolerated with no serious side effects.

Several conclusions were drawn from this data: (1) Sodium stibogluconate has a marked and broad anti-tumor activity in vivo as a single agent at a dosage that may be clinically achievable and tolerated. (2) The demonstrated synergy between sodium stibogluconate and cytokines, specifically IFN-alpha in vivo indicates that combinational usage of sodium stibogluconate may significantly improve the current IFN-alpha therapies in cancer treatment. (3) Because sodium stibogluconate targets PTPases and, therefore, functions via a mechanism distinct from those of current anticancer therapies, the drug may be useful as an alternative therapeutic for cancers non-responsive or resistant to conventional anti-cancer therapies. (4) The correlation between in vitro and in vivo responses of cancer cell lines to sodium stibogluconate or sodium stibogluconate/IFN-alpha indicates that other human cancer cell lines sensitive to these agents in vitro, as shown by studies described above, will be responsive to these agents in vivo as well. This further suggests that the human malignancies represented by the sensitive cell lines (e.g., human breast cancer cell line MDA231 and multiple myeloma cell line U266) may benefit from sodium stibogluconate/IFN-alpha combinational therapies. (5) Because the nude mice study verified that the synergy between sodium stibogluconate and IFN-alpha as detected in vitro also occurs in vivo, the in vitro synergy of sodium stibogluconate with other cytokines (e.g., IFNP), as detected in the studies described above, may similarly exist in vivo; therefore, sodium stibogluconate may be a useful adjuvant in IFN-alpha therapy for viral or autoimmune diseases (e.g. hepatitis C and multiple sclerosis).

V. Sodium Stibogluconate Interacts with IL-2 in Anti-Renca Tumor Action Via a T Cell-Dependent Mechanism in Connection With Induction of Tumor-Infiltrating Macrophages

IL-2 therapy induces 10-20% response rates in advanced renal cell carcinoma (RCC) via activating immune cells, in which protein tyrosine phosphatase SHP-1 is a key negative regulator. Based on our recent finding that sodium stibogluconate (SSG) inhibits SHP-1, the anti-RCC potential and action mechanism of SSG and SSG/IL-2 combination were investigated in a murine renal cancer model (Renca). SSG in Balb/c mice induced 61% growth inhibition of Renca tumors coincident with an increase (2-fold) in tumor-infiltrating macrophages (Mϕ) but failed to inhibit Renca cell proliferation in culture. SSG/IL-2 combination was more effective in inhibiting tumor growth (91%) and in inducing tumor-infiltrating Mϕ (4-fold) whereas the cytokine alone showed little effects. Involvement of T cells was indicated by the lack of activity of the combination treatment on Renca tumor growth in athymic nude mice. Although SSG or SSG/IL-2 treatment did not increase tumor-infiltrating T cells in Balb/c mice, SSG increased in vitro T cell secretion of IFN-gamma capable of activating tumoricidal activity of Mϕ. Spleen Mϕ increases were detected in the mice treated with SSG (3-fold) or SSG/IL-2 combination (6-fold) and indicate a systemic Mϕ expansion, which is a prominent feature in mice with genetic SHP-1 deficiency. The SSG and SSG/IL-2 combination treatments were tolerated in the mice. These results together demonstrate an anti-Renca tumor activity of SSG that is heightened in combination with IL-2 and functions via a T-cell-dependent mechanism in connection with expansion/activation of Mϕ, suggesting that SSG might improve anti-RCC efficacy of IL-2 therapy by enhancing anti-tumor immunity.

A. Materials and Methods

1. Reagents, cells, cell culture and cell growth inhibition assays

Renca (Murphy, G. P., and W. J. Hrushesky. 1973. A murine renal cell carcinoma. J Natl Cancer Inst 50:1013), Jurkat (Gillis, S., and J. Watson. 1980. Biochemical and biological characterization of lymphocyte regulatory molecules. V. Identification of an interleukin 2-producing human leukemia T cell line. J Exp Med 152:1709) and WM9 (Forsberg, K., I. Valyi-Nagy, C. H. Heldin, M. Herlyn, and B. Westermark. 1993. Platelet-derived growth factor (PDGF) in oncogenesis: development of a vascular connective tissue stroma in xenotransplanted human melanoma producing PDGF-BB. Proc Nat1 Acad Sci U S A 90:393) cell lines were obtained from a colleague at the Cleveland Clinic Foundation (CCF) and cultured in RPMI 1640 medium supplemented with 10% fetal calf serum (FCS). Recombinant IL-2 (Proleukin, 22 million IU/1.3 mg, Chiron, Emeryville, CA) was purchased from the CCF pharmacy. SSG has been described previously (Yi, T., M. K. Pathak, D. J. Lindner, M. E. Ketterer, C. Farver, and E. C. Borden. 2002. Anticancer activity of sodium stibogluconate in synergy with IFNs. J Immunol 169:5978). For cell growth inhibition assays, cells were cultured in absence or presence of various amounts of SSG for 6 days with viable cells quantified by MTT assays as described elsewhere (Pathak, M. K., and T. Yi. 2001. Sodium stibogluconate is a potent inhibitor of protein tyrosine phosphatases and augments cytokine responses in hemopoietic cell lines. J Immunol 167:3391).

2. Animal studies

Balb/c and athymic nude Balb/c mice (10 weeks old, female, Taconic Farms, Germantown, NY) were inoculated (s.c.) at the flanks with Renca cells (106 cells/site). Four days post-inoculation, the mice were subject to no treatment (Control) or treatment with IL-2 (105 IU/daily for 5 days, i.p.), SSG (12 mg/daily, i.m. at hip regions) or the combination of the two agents for two weeks. The IL-2 dose was comparable to those used in previous studies for assessing murine anti-Renca tumor immunity (Sonouchi, K., T. A. Hamilton, C. S. Tannenbaum, R. R. Tubbs, R. Bukowski, and J. H. Finke. 1994. Chemokine gene expression in the murine renal cell carcinoma, RENCA, following treatment in vivo with interferon-alpha and interleukin-2. Am J Pathol 144:747). The dose of SSG was similar to the effective daily dose of the drug for the treatment of murine leishmaniasis (Murray, H. W., J. D. Berman, and S. D. Wright. 1988. Immunochemotherapy for intracellular Leishmania donovani infection: gamma interferon plus pentavalent antimony. J Infect Dis 157:973). Tumor volume was measured during the study period and calculated using the formula for a prolate spheroid (V= 4/3 x a2b) (Lindner, D. J., E. C. Borden, and D. V. Kalvakolanu. 1997. Synergistic antitumor effects of a combination of interferons and retinoic acid on human tumor cells in vitro and in vivo. Clin Cancer Res 3:931). Student's t test was used for assessing the significance of tumor volume differences among differential treatment groups. Mouse viability (daily) and body weights (weekly) were also recorded during the study period. At the end of the study, Renca tumors and major internal organs (heart, kidney, liver, lung and spleen) were harvested for histology and immunohistochemistry analysis.

3. Histology and immunohistochemistry

Major internal organs and Renca tumors harvested from mice were fixed in 10% formalin or snap frozen in liquid nitrogen. H & E-stained tissue sections of the fixed samples were prepared and evaluated by microscopy as described previously (Yi, T., M. K. Pathak, D. J. Lindner, M. E. Ketterer, C. Farver, and E. C. Borden. 2002. Anticancer activity of sodium stibogluconate in synergy with IFNs. J Immunol 169:5978). Preparation of frozen tissue sections and immunohistochemistry were performed following established procedures (Joliat, M. J., P. A. Lang, B. L. Lyons, L. Burzenski, M. A. Lynes, T. Yi, J. P. Sundberg, and L. D. Shultz. 2002. Absence of CD5 dramatically reduces progression of pulmonary inflammatory lesions in SHP-1 protein-tyrosine phosphatase-deficient 'viable motheaten' mice. J Autoimmun 18:105). The antibodies used for imnzunohistochemistry were anti-CD4 (rat mAb, clone GK1.5, BD Biosciences, Franklin Lakes, NJ), anti-CD8 (rat mAb, clone 53-6.7.5, BD Biosciences, Franklin Lakes, NJ), anti-F4/80 (rat mAb, clone A3-1, Serotec, Oxford, UK) and anti-Asialo GM1 (rabbit polyclonal, Cedarlane, Hornby, Canada). The sections were counterstained with Mayer's hematoxylin prior to microscopic examination. Tissue sections of 2 mice/group were evaluated. The number of immune cells was semi-quantified based on the following scheme: +, 0-1 positive cells/40 x field; +, 2-5 positive cells/40 x field; ++, 6-10 positive cells/40 x field, etc.

4. In vitro effects of SSG on immune cells

Jurkat cells were cultured in the absence or presence of various amounts of SSG for 16 hours. The cells and culture medium supernatants were separated by centrifugation (1,000 g, 10 min). The amounts of IFN-gamma in the culture medium supernatants were quantified using an ELISA kit (R.D. system, Minneapolis, MN) following the manufacturer's protocol.

B. Results

1. SSG inhibits Renca tumor growth in Balb/c mice but not Renca cell proliferation in culture

To investigate a potential anti-RCC activity of SSG that functions via an immune mechanism, the effects of SSG on Renca tumor growth in Balb/c mice were determined. Renca, derived from a spontaneous kidney tumor in Balb/c mice, was chosen based on its tumorigenecity in this strain of immune competent mice (Murphy, G. P., and W. J. Hrushesky. 1973. A murine renal cell carcinoma. J Natl Cancer Inst 50:1013).

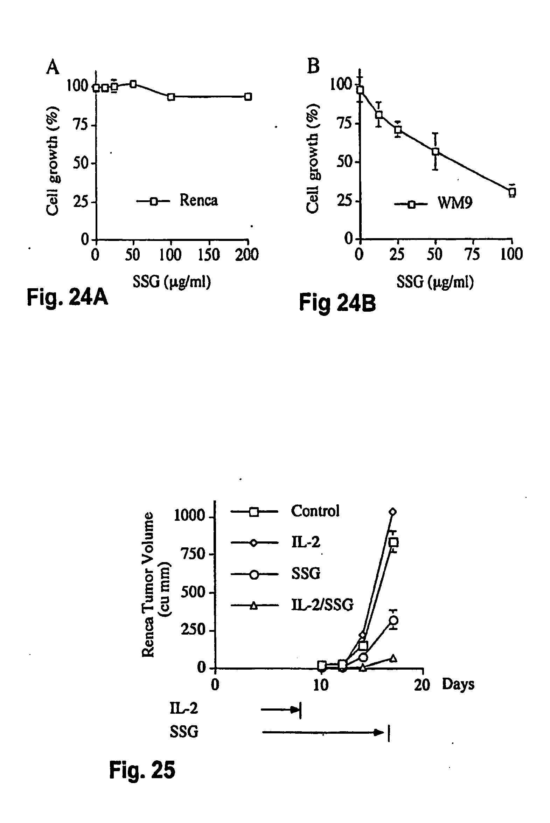

The effects of SSG on Renca cell growth in culture were initially examined to determine whether SSG could directly inhibit Renca cell growth in the absence of immune cells. Renca cells cultured in the absence or presence of SSG (6.25 - 200 µg/ml) for 6 days showed similar growth (Fig 24A) while the growth of WM9 melanoma cells was inhibited by SSG in a dose-dependent manner under comparable conditions (Fig 24B) as reported previously (Yi, T., M. K. Pathak, D. J. Lindner, M. E. Ketterer, C. Farver, and E. C. Borden. 2002. Anticancer activity of sodium stibogluconate in synergy with IFNs. J Immunol 169:5978).

The effects of SSG on Renca tumor growth in vivo were assessed by treating Balb/c mice bearing 4-day-established Renca tumors with SSG, which was administered daily at its effective dosage for murine Leishmaniasis (Murray, H. W., J. D. Berman, and S. D. Wright. 1988. Immunochemotherapy for intracellular Leishmania donovani infection: gamma interferon plus pentavalent antimony. J Infect Dis 157:973) for a two-week period. At the end of the treatment period, Renca tumors in the SSG treated mice were significantly (p < 0.01) smaller (39%) than those (100%) in the untreated control (Fig 25). The treatment was tolerated well: all mice in the treatment group survived at the end of treatment (data not shown).

Thus SSG as a single agent induced significant Renca tumor growth inhibition in Balb/c mice and was well tolerated. This anti-Renca tumor action of SSG probably did not result from a direct inhibition of Renca tumor growth since the drug did not obviously affect Renca cell growth in culture. These results together demonstrated an anti-Renca tumor activity of SSG, which appeared to function via an indirect mechanism that might involve anti-tumor immunity.

2. SSG/EL-2 combination induces more effective Renca tumor growth inhibition than single agents

A putative anti-tumor immune mechanism for SSG in anti-Renca tumor action suggests that Renca tumor growth might be inhibited more effectively by SSG in combination with IL-2, which is known to activate anti-tumor immune cells (Rosenberg, S. A. 2000. Interleukin-2 and the development of immunotherapy for the treatment of patients with cancer. Cancer J Sci Am 2000:S2). Since the cytokine is an approved treatment induces low response rates in advanced RCC (Margolin, K. A. 2000. Interleukin-2 in the treatment of renal cancer. Semin Oncol 27:194), evidence that SSG interacts with IL-2 in Renca tumor growth inhibition would also provide pre-clinical proof of concept regarding the potential of SSG to improve the efficacy of IL-2 therapy. Effects of SSG/IL-2 combination on Renca tumor growth were therefore determined.

Balb/c mice bearing 4-day-established Renca tumors were treated with SSG/IL-2 combination or with IL-2 alone in comparison to untreated control and mice subjected to SSG treatment. SSG/IL-2 combination treatment for two weeks induced 90% of Renca tumor growth inhibition vs 61% induced by SSG (p < 0.01) (Fig 25). IL-2 as a single agent under the experimental conditions failed to inhibit Renca tumor growth (Fig 25), consistent with previous reports (Sonouchi, K., T. A. Hamilton, C. S. Tannenbaum, R. R. Tubbs, R. Bukowski, and J. H. Finke. 1994. Chemokine gene expression in the murine renal cell carcinoma, RENCA, following treatment in vivo with interferon-alpha and interleukin-2. Am J Pathol 144:747; Samlowski, W. E., R. Petersen, S. Cuzzocrea, H. Macarthur, D. Burton, J. R. McGregor, and D. Salvemini. 2003. A nonpeptidyl mimic of superoxide dismutase, M40403, inhibits dose-limiting hypotension associated with interleukin-2 and increases its antitumor effects. Nat Med 9:750). The combination was tolerated as indicated by the survival of all of the treated mice at the end of the treatment period and the comparable body weights of untreated and treated mice that showed no histopathologic changes in their major organs (data not shown).

These results demonstrated that SSG/IL-2 combination induced more effective Renca tumor growth inhibition than the agents individually, consistent with an immune mechanism of SSG anti-Renca tumor action. The capacity of SSG to interact with IL-2 in anti-Renca tumor action and the tolerance of the combination treatment in mice suggest the possibility of SSG/IL-2 combination therapy as an improved treatment for advanced RCC.

3. SSG and SSG/IL-2 treatments induces tumor-infiltrating Mϕ and systemic Mϕ expansion

To further define the action mechanism of SSG and SSG/IL-2 combination in Renca tumor growth inhibition, the effects of these treatments on Renca tumor-infiltrating immune cells were investigated. T, NK and Mϕ lineage cells are important anti-tumor effectors (Rosenberg, S. A. 2001. Progress in human tumour immunology and immunotherapy. Nature 411:380). The relative numbers of these immune cells in Renca tumors from mice differentially treated with SSG, IL-2 or the combination were quantified by immunohistochemistry.

T lymphocytes (CD4+ or CD8+) were present at low numbers in Renca tumors as reported previously and showed little difference among tumors from the differentially treated mice (Fig 26A) whereas NK cells were undetectable in the tumors (data not shown) under the experimental conditions. Although tumor-infiltrating Mϕ (F4/80+) was at comparable levels in control and IL-2-treated mice, interestingly, it showed a modest increase (∼ 2-fold) in SSG-treated mice and a more marked increase (∼ 4-fold) in SSG/IL-2-treated mice (Fig 26AB).

To assess whether the increased Mϕ in Renca tumors from SSG and SSG/IL-2-treated mice is a tumor-specific event or a part of a systemic Mϕ expansion, the relative numbers of Mϕ in spleen of the differentially treated mice were also quantified by immunohistochemistry. Spleen Mϕ (F4/80+) numbers were significantly increased (∼ 3-fold) in SSG-treated mice and markedly heightened (∼ 6-fold) in mice treated by SSG/IL-2 combination in comparison to the background levels of M∼ in control or IL-2-treatment groups (Fig 27AB). Spleens of the differentially treated mice showed similar levels of CD4+/CD8+ cells (Fig 27A).

These results demonstrated that SSG treatment induced M∼ infiltration in Renca tumors and an apparently systemic Mϕ expansion, which were amplified by co-administering IL-2. In contrast, SSG or SSG/IL-2 treatment had little effect on the numbers of tumor-infiltrating T cells or NK cells. This selective induction of tumor-infiltrating Mϕ and systemic Mϕ expansion by SSG and SSG/IL-2 provides histological evidence supporting an immune mechanism for the anti-Renca tumor action of the treatments and implicates Mϕ as potential direct anti-Renca tumor effector cells.

4. SSG/IL-2 anti-Renca tumor action requires the presence of T cells

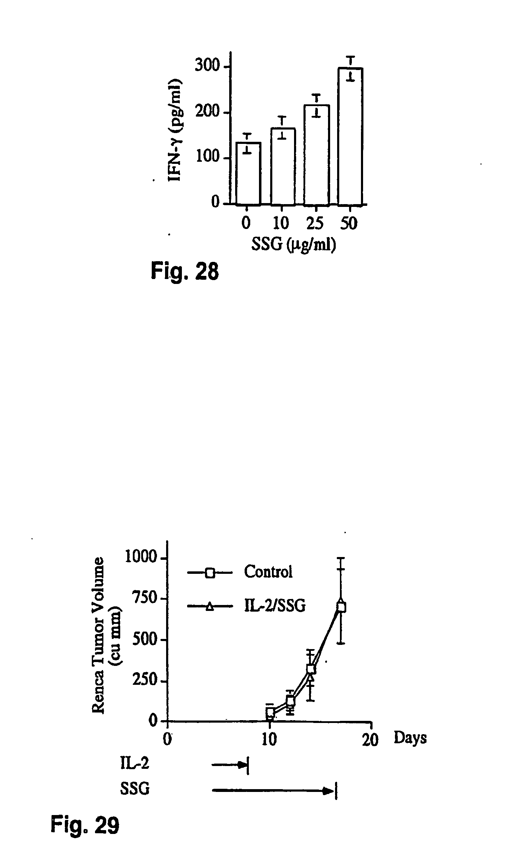

The putative mechanism that Mϕ acts as direct anti-Renca tumor effector cells does not exclude an involvement of T cells, which might be activated by SSG or SSG/IL-2 to secret cytokines required for inducing tumoricidal activity of Mϕ. Indeed, Jurkat T cells treated with SSG were found to secret increased amounts of IFN-gamma (Fig 28), which could activate Mϕ in anti-tumor action (Samlowski, W. E., R. Petersen, S. Cuzzocrea, H. Macarthur, D. Burton, J. R. McGregor, and D. Salvemini. 2003. A nonpeptidyl mimic of superoxide dismutase, M40403, inhibits dose-limiting hypotension associated with interleukin-2 and increases its antitumor effects. Nat Med 9:750, Qin, Z., J. Schwartzkopff, F. Pradera, T. Kammertoens, B. Seliger, H. Pircher, and T. Blankenstein. 2003. A critical requirement of interferon gamma-mediated angiostasis for tumor rejection by CD8+ T cells. Cancer Res 63:4095). The anti-Rencatumor efficacy of SSG/IL-2 combination was studied in athymic mice lacking T cells to assess the role of T cells in the anti-Renca tumor action of the therapy.

Athymic nude Balb/c mice bearing 4-day-established Renca tumors were untreated or treated with SSG/IL-2 combination for 2 weeks. Renca tumors grew in a comparable manner in both groups of mice during the treatment period (Fig 29), demonstrating a lack of growth inhibitory activity of the SSG/IL-2 treatment on Renca tumors in the athymic mice under the experimental conditions. Immunohistochemical analysis of Renca tumors and spleens from the control and SSG/IL-2-treated mice revealed an increase of Mϕ in tumors (2-fold) and spleen (3-fold) from the SSG/IL-2-treated mice in comparison to those of control (data not shown).

These results demonstrate that SSG/IL-2-induced Renca tumor growth inhibition requires the presence of T cells, providing genetic evidence supporting an anti-tumor immune mechanism for the treatment.

C. Discussion

The results presented herein demonstrate for the first time a significant anti-Renca tumor activity of SSG that is mediated via an immune mechanism and augmented in the presence of IL-2.

An immune mechanism of SSG anti-Renca tumor action is supported by several lines of evidence. It is suggested by the initial observation that SSG induced Renca tumor growth inhibition in Balb/c mice but failed to inhibit Renca cell proliferation in culture, which argues against a direct anti-Renca tumor mechanism via SSG cytotoxicity. It is indicated further by the histological evidence that SSG induced tumor-infiltrating Mϕ coincident with systemic Mϕ expansion. It is supported strongly by the genetic evidence of T cell requirement for the anti-Renca tumor action of SSG in combination with IL-2. We showed in previous studies an anti-melanoma tumor activity of SSG that is likely resulted from a direct action of SSG on melanoma tumor cells since SSG induces marked growth inhibition of the melanoma cells in culture and inhibited melanoma tumor growth in immune-deficient nude mice (Yi, T., M. K. Pathak, D. J. Lindner, M. E. Ketterer, C. Farver, and E. C. Borden. 2002. Anticancer activity of sodium stibogluconate in synergy with IFNs. J Immunol 169:5978). The reported capacity of SSG to interact with recombinant human IFN-alpha in anti-melanoma tumor action in mouse models could also be explained via an immunity-independent mechanism based on the synergy of SSG/IFN-alpha in direct growth inhibition of the melanoma cells in culture in the absence of immune cells (Yi, T., M. K. Pathak, D. J. Lindner, M. E. Ketterer, C. Farver, and E. C. Borden. 2002. Anticancer activity of sodium stibogluconate in synergy with IFNs. J Immunol 169:5978). Involvement of IFN-activated immunity in the reported anti-melanoma tumor action in mice could be excluded because the species-specificity of the recombinant human IFN-alpha, which is inactive on mouse immune cells but would inhibit the growth of the melanoma cells of human origin (Yi, T., M. K. Pathak, D. J. Lindner, M. E. Ketterer, C. Farver, and E. C. Borden. 2002. Anticancer activity of sodium stibogluconate in synergy with IFNs. J Immunol 169:5978). Our current study thus revealed a novel immune-mediated anti-tumor action for SSG.

This finding of an immune-mediated anti-tumor action of SSG has significant clinical implications in addition to providing insights into mechanism of action for the drug. It indicates a broader SSG application as a potential anti-cancer therapeutic that might be beneficial in patients with tumors sensitive as well as insensitive to direct growth inhibition by SSG. Moreover, the dual anti-tumor actions of SSG via direct tumor-growth inhibition and anti-tumor immunity also suggest that SSG might be most effective when used in immune competent patients with tumors sensitive to direct SSG growth inhibition. This concept might aid the selection of cancer patients for optimal efficacy of SSG-based therapy and could be verified in future pre-clinical studies. In addition, the immunie-mediated anti-tumor action of SSG suggests the potential of SSG to be used in combination with other immune activation agents, including IL-2 that was investigated in this study.

SSG/IL-2 combination has been demonstrated in this study to be more effective in anti-Renca tumor action in comparison to single agents. The superior anti-Renca tumor action of SSG/IL-2 combination and the tolerance of the treatment in mice provide pre-clinical proof of concept evidence that SSG might have potential for improving the efficacy of IL-2 anti-RCC therapy and warrant its clinical evaluation in the future. In this regard, it is worth noticing that the dose of IL-2 used in our anti-Renca tumor experiment was approximately 25% of the reported maximal tolerated dose (MTD) in mice (Samlowski, W. E., R. Petersen, S. Cuzzocrea, H. Macarthur, D. Burton, J. R. McGregor, and D. Salvemini. 2003. A nonpeptidyl mimic of superoxide dismutase, M40403, inhibits dose-limiting hypotension associated with interleukin-2 and increases its antitumor effects. Nat Med 9:750). Tolerance of SSG with IL-2 at MTD in mice was also observed in a preliminary experiment (unpublished data). It is therefore possible that more striking anti-Renca tumor action might be achievable using optimized SSG/IL-2 combination therapy that could be defined through differential dosing and/or treatment schedule. The demonstrated capacity of SSG to interact with IL-2 without obviously increasing IL-2 toxicity, which is mediated through IL-2 activated T cells that induce a capillary leaking syndrome (Rosenberg, S. A. 2000. Interleukin-2 and the development of immunotherapy for the treatment of patients with cancer. Cancer J Sci Am 2000:S2), might be related to our observation that SSG anti-Renca tumor activity is likely mediated in part through Mϕ as discussed below and thus does not depend solely on increasing IL-2-induced T cell activation.

An important finding of our study is that SSG induces tumor-infiltrating Mϕ and a marked systemic Mϕ expansion, which were amplified by IL-2. In addition to providing histological evidence supporting an immune mechanism for SSG anti-Renca tumor action, this SSG activity is also a potential indication of in vivo inhibition of SHP-1 in SSG-treated mice since systemic Mϕ expansion is a key feature of mice with genetic SHP-1-deficiencies (Green, M. C., and L. D. Shultz. 1975. Motheaten, an immunodeficient mutant of the mouse. I. Genetics and pathology. J Hered 66:250; Shultz, L. D., D. R Coman, C. L. Bailey, W. G. Beamer, and C. L. Sidman. 1984. "Viable motheaten," a new allele at the motheaten locus. I. Pathology. Am J Pathol 116:179; Shultz, L. D., P. A. Schweitzer, T. V. Rajan, T. Yi, J. N. Ihle, R. J. Matthews, M. L. Thomas, and D. R Beier. 1993. Mutations at the murine motheaten locus are within the hematopoietic cell protein-tyrosine phosphatase (Hcph) gene. Cell 73:1445). Moreover, it implicates Mϕ as direct anti-tumor effector cells of the drug. Such a putative role for Mϕ is consistent with the apparent lack of effects of SSG on the levels of tumor-infiltrating T cells and supported by previous reports of Mϕ as important immune cells with tumoricidal activity (Samlowski, W. E., R. Petersen, S. Cuzzocrea, H. Macarthur, D. Burton, J. R McGregor, and D. Salvemini. 2003. A nonpeptidyl mimic of superoxide dismutase, M40403, inhibits dose-limiting hypotension associated with interleukin-2 and increases its antitumor effects. Nat Med 9:750, Masztalerz, A., N. Van Rooijen, W. Den Otter, and L. A. Everse. 2003. Mechanisms of macrophage cytotoxicity in IL-2 and IL-12 mediated tumour regression. Cancer Immunol Immunother 52:235). Significantly, it also provides a rational explanation for the SSG/IL-2 interaction in anti-Renca tumor action. Since SSG-induced tumor-infiltrating Mϕ was augmented by IL-2, the heightened anti-Renca tumor effects of SSG/IL-2 combination might be resulted from a converging action of the two agents on Mϕ that directly attacks the tumor cells. However, it is not clear at present how IL-2 augments SSG-induced tumor-infiltrating Mϕ. Although the IL-2 receptor is expressed on monocytes (Espinoza-Delgado, I., M. C. Bosco, T. Musso, G. L. Gusella, D. L. Longo, and L. Varesio. 1995. Interleukin-2 and human monocyte activation. J Leukoc Biol 57:13) that differentiate into Mϕ, our observation that the effect of IL-2 on tumor-infiltrating Mϕ was T cell-dependent argues against a direct role for IL-2-induced monocyte differentiation. The involvement of cytokines from IL-2-activated T cells in the process is a more likely alternative mechanism.

In addition to revealing a putative role for Mϕ in SSG anti-Renca tumor action, our results also implicate the involvement of T cells that are known to play a key role in anti-tumor immunity (Rosenberg, S. A. 2001. Progress in human tumour immunology and immunotherapy. Nature 411:380). T cells are apparently required for the capacity of IL-2 to augment SSG induction of tumor-infiltrating Mϕ and systemic Mϕ expansion. This is indicated by the observation that the levels of tumor-infiltrating Mϕ and -spleen Mϕ expansion in SSG/IL-2-treated athymic mice were similar to those induced by SSG alone in the T cell-competent Balb/c mice. The importance of T cells is further underscored by the lack of Renca tumor growth inhibition in the presence of the modest increase of tumor-infiltrating Mϕ in the SSG/IL-2-treated athymic mice. Taking into consideration the low number of tumor-infiltrating T cells and the capacity of SSG to induce T cell secretion of IFN-gamma capable of activating Mϕ (Samlowski, W. E., R. Petersen, S. Cuzzocrea, H. Macarthur, D. Burton, J. R. McGregor, and D. Salvemini. 2003. A nonpeptidyl mimic of superoxide dismutase, M40403, inhibits dose-limiting hypotension associated with interleukin-2 and increases its antitumor effects. Nat Med 9:750, Qin, Z., J. Schwartzkopff, F. Pradera, T. Kammertoens, B. Seliger, H. Pircher, and T. Blankenstein. 2003. A critical requirement of interferon gamma-mediated angiostasis for tumor rejection by CD8+ T cells. Cancer Res 63:4095), the results together suggest that T cells might mediate SSG/IL-2 anti-Renca tumor action through secreting cytokines to induce and activate tumor-infiltrating Mϕ.

Our finding that SSG exerts anti-Renca tumor activity via an immune mechanism is also significant in several other aspects. It provides evidence that strengthens a hypothetic immune mechanism of SSG in anti-Leishmania action. In particular, the observed systemic Mϕ expansion in SSG-treated mice suggests the presence of such a pharmacological effect during SSG anti-Leishmaniasis therapy that might have been overlooked so far. Given the differential activities of SSG against the free-living promastigotes and intracellular amastigotes (Berman, J. D., and D. J. Wyler. 1980. An in vitro model for investigation of chemotherapeutic agents in leishmaniasis. J. Infect. Dis. 142:83), it raises the possibility that several other compounds (Berman, J. D. 1988. Chemotherapy for leishmaniasis: biochemical mechanisms, clinical efficacy, and future strategies. Rev Infect Dis 10:560) with similar anti-leishmania characteristics might also have potential anti-cancer activity through immune action and need to be re-evaluated accordingly. Taking into consideration of the tolerance of SSG and its apparent capacity to activate immune cells via inhibiting SHP-1, it indicates that refined inhibitors of the phosphatase could be developed as safe immune activators for anti-cancer therapy and other immune therapies.

V. Sodium Stibogluconate Inhibits PRL Family PTPases IN Anti-Cancer Action

The data collected demonstrates that sodium stibogluconate is a potent inhibitor of recombinant and intracellular PRLs and that sodium stibogluconate at a nontoxic dose has growth inhibitory activity in vitro and in mouse models against cancer cell lines that express the PRLs. The data suggested that sodium stibogluconate inactivation of PRLs is one of the key mechanisms of its anti-cancer activity because a mutated form of PRL-1 in sodium stibogluconate-resistant cancer cells is insensitive to sodium stibogluconate inhibition and confers resistance to sodium stibogluconate-induced growth inhibition when ectopically expressed in sodium stibogluconate-responsive cancer cells. These results suggested the potential of sodium stibogluconate as an anti-cancer drug and provide novel insights for developing PTPase inhibitors as targeted therapeutics.

A. Materials and Methods

1. Reagents

Sodium stibogluconate, suramin, sodium orthovanadate and GST fusion protein of SHP-1 have been described previously (Pathak et al., J. Imumol. 167, 3391 (2001)). Meglumine antimonate was obtained from Aventis. cDNAs of human PRL-1, PRL-2 and PRL-3 coding region were derived by RT-PCR from H9 cells (Safai, et al., Lancet 1, 1438 (1984)) and inserted in frame into the pGEX vector. cDNA of PRL-1R86 was generated by recombinant DNA technique using PRL-1 cDNA as template following established procedures (Jiao, et al., Mol. Cell. Biol. 16, 6985 (1996)). GST fusion proteins of the PRL phosphatases were prepared from DH5a bacteria transformed with the pGEX fusion protein constructs as described previously (Yi, et al., Mol. Cell. Biol. 12, 836 (1992)). cDNAs encoding the PRLs tagged at the N-termini with the Flag epitope (Castrucci, et al., J. Virol. 66, 4647 (1992)) were generated via recombinant DNA technique, sequenced to confirm their identities and cloned into the pBabepuro vector (Yang, et al., Blood 91, 3746 (1998)). Anti-Flag monoclonal antibody (M2, Sigma) was purchased from a commercial source. A synthetic phosphotyrosine peptide (Arg-Arg-Leu-Ile-Glu-Asp-Ala-Gle-Tyr-Al- a-Ala-Arg-Gly (SEQ ID NO: 3), wherein the tyrosine is phosphorylated; UBI) and DiFMUP (6, 8-difluoro-4-methylumbelliferyl phosphate, Molecular Probes) were purchased as substrates for PTPase assays.

2. In Vitro PTPase Assays and Immunocomplex PTPase Assays

In vitro PTPase assays were used to determine the effects of compounds on recombinant PTPases, following established procedures using a synthetic phosphotyrosine peptide or DiFMUP as the substrate (Pathak et al., J. Imumol. 167, 3391 (2001)). Briefly, individual PTPases (0.1 µg/reaction) in 50 µL of PTPase buffer (50 mM Tris, pH 7.4) were incubated at 22.degree. C. for 10 minutes or as indicated in the absence or presence of inhibitory compounds. Substrates (0.2 mM phosphotyrosine peptide) were then added and allowed to react at 22.degree. C. for 18 hrs. PTPase activity of individual reactions was measured by adding 100 µL of malachite green solution (UBI) and then quantifying the amounts of free phosphate cleaved by the PTPase from the peptide substrate by spectrometry (OD660 nm). PTPase assays using DiFMUP as a substrate were conducted following a previously described procedure (Matter, et al., Biochem. Biophys. Res. Comm. 283, 1061 (2001)). Relative PTPase activities were calculated based on the formula: (PTPase activity in the presence of an inhibitory compound/PTPase activity in the absence of the compound).times.100%. To assess the reversibility of PTPase inhibition, GST fusion proteins of the PTPases bound on glutathione beads (Pharmacia) were pre-incubated with cold Tris buffer (50 mM Tris, pH 7.0) or Tris buffer containing the inhibitor at 4.degree. C. for 30 minutes. The beads were then washed three times in cold Tris buffer or not washed prior to subjecting to in vitro PTPase assays.

Immunocomplex PTPase assays were performed to assess the effects of sodium stibogluconate on intracellular PTPases. Cells were untreated or treated with sodium stibogluconate for 5 minutes, washed with fresh medium and then lysed in cold lysis buffer (50 mM Tris, pH 7.4; 150 mM NaCl; 1% NP40; 2 mM PMSF; 20 ,g/ml of Aprotinin). The lysates were incubated with an anti-Flag antibody in immunoprecipitation assays. The immunocomplexes were collected with protein G sepharose beads (Pharmacia) and washed in cold lysis buffer for 4 times. Approximately 90% of the contents of individual samples were split into 3 comparable portions and each was then incubated in 50 µL of PTPase buffer (50 mM Tris, pH 7.4; 0.2 mM phosphotyrosine peptide) at 22.degree. C. for 18 hrs. 100 µL of malachite green solution (UBI) was added to each reaction prior to measurement of OD660 to quantify the amounts of free phosphate cleaved by the PTPases from the peptide substrate (Pathak et al., J. hnumol. 167, 3391 (2001)). The remaining 10% contents of individual samples were analyzed by SDS-PAGE/Western blotting to quantify the relative amounts of the phosphatase proteins. To assess the duration of sodium stibogluconate effects on the activities of intracellular PTPases, Flag-PRL-2 transfected cells were untreated or treated with sodium stibogluconate for 5 minutes at 37° C., washed twice with culture medium to remove cell-free drug and then incubated in fresh culture medium at 37° C. for 24-72 hours prior to termination by lysing the cells in cold lysis buffer. Flag-PRL-2 were immunoprecipitated from the lysates and subjected to PTPase assays and SDS-PAGE/Western blotting.

3. Cells, Cell Culture, Cell Growth Assays, and Transfection.

NIH3T3 (Yi, et al., Blood 85, 87 (1995)), WM9 (Forsberg, et al., Proc. Nat. Acad Sci. USA 90, 393 (1993)), DU145 (Mickey, et al., Cancer Res. 37, 4049 (1977)), LoVo (Drewinko, et al., Cancer Res. 36, 467 (1976)), HEY (Buick, et al., Cancer Res. 45, 3668 (1985)), U251 (Yoshida, et al., Cancer 50, 410 (1982)), A549 (Giard, et al., J. Natl. Cancer Inst. 51,1417 (1973)) and SK-N-SH (Helson, et al., Cancer Res. 35, 2594 (1975)) cell lines have been described and were cultured in RPMI 1640 supplemented with 10% fetal calf serum (FCS). For measurement of sodium stibogluconate effects on cell growth in vitro, cells were cultured in the absence (-) or presence (+) of various amounts of sodium stibogluconate for 6 days with viable cells quantified by MTT assays (Mosmann, J. Imunol. Methods 65, 55 (1983)). Percentages of growth inhibition by sodium stibogluconate were calculated (+/- x %).

The effects of sodium stibogluconate on intracellular PTPases were assessed using NIH3T3 or WM9 transfectants. NIH3T3 or WM9 cells were transfected with the pBabepuro vector (V) or pBabepuro expression constructs of Flag-tagged PRLs using Lipofectamine (BRL) following the manufacturer's procedures. Transfectants were selected in the presence of puromycine (0.5 µg/ml) for two weeks and expanded in culture without puromycine prior to their usage in measuring the effects of sodium stibogluconate on the PTPase activities of intracellular Flag-PRLs or to determine cell growth in culture in the absence or presence of sodium stibogluconate.

4. Animal Studies.

Athymic nude mice (nu/nu, NCR), 4 weeks old (Taconic), were inoculated (s.c.) in the flanks with DU145 cells (3 x 10⁶ cells/site) on day 0. Starting on day 2, the mice were subjected to no treatment (Control) or treatment with sodium stibogluconate (12 mg, s.c., daily, i.m., at the hip area). The dosage of sodium stibogluconate used in the study was similar to the effective daily dose of sodium stibogluconate for the treatment of murine leishmaniasis (Murray, et al. 1988). Tumor volume was measured and calculated using the formula for a prolate spheroid (V=4/3 7 a^{2b}) (Lindner, et al. 1997). Hematoxylin+Eosin (H.E.) stained tissue sections of internal organs and tumor inoculation sites tissues of the mice were prepared and subjected microscopic evaluation.

5. Isolation and Characterization of Sodium Stibogluconate-Resistant DU145 Colonies.

DU145 cells were cultured in the presence of sodium stibogluconate (100 µg/ml) in 48 well plates for 3 weeks. Cells from a well containing a single colony were transferred to flasks, cultured in sodium stibogluconate-free medium for 3 weeks and used as DU145R cells for further characterization. Growth of DU145R cells in the absence or presence of sodium stibogluconate in day 6 culture was determined by MTT assays. cDNAs of the coding region of PRLs were derived by RT-PCR from DU145 and DU145R cells and sequenced using primers described below.

6. Detection of the Expression of PRL Phosphatatases by RT-PCR Analysis.

Expression of the transcripts of PRLs in peripheral blood mononuclear cells (PBMC) from a healthy volunteer and in cancer cells lines were detected by RT-PCR with specific primer pairs for individual PRLs as listed below or for GAPDH. RT-PCR products were separated in agarose gels and visualized by ethidium bromide staining with their identities confirmed by restriction endonuclease mapping. The sequence of primer pairs are: huPRL-3/5, 5'-TAGGATCCCGGGAGGCGCCATGGCTCGGATGA-3' (SEQ ID NO: 4); huPRL-3/3, 5'-GAGTCGACCATAACGCAGCACCGGGTCTTGTG-3' (SEQ ID NO: 5); huPRL-2/5, 5'-TAGGATCCCCATAATGAACCGTCCAGCCCCTGT-3' (SEQ ID NO: 6); huPRL-2/3, 5'-GAGTCGACCTGAACACAGCAATGCCCATTGGT-3' (SEQ ID NO: 7); huPRL-1/5, 5'-TAGGATCCCCAACATGGCTCGAATGAACCGCCC-3' (SEQ ID NO: 8); huPRL-1/3, 5'-GAGTCGACTTGAATGCAACAGTTGTTTCTATG-3' (SEQ ID NO: 9).

B. Results

1. Sodium Stibogluconate Inhibits Recombinant PRL Phosphatases In Vitro.

To assess whether sodium stibogluconate is an inhibitor of PRL phosphatases, its effects on the PTPase activity of recombinant PRLs were evaluated by in vitro PTPase assays.

PTPase activity of recombinant PRL-1, PRL-2 and PRL-3 in dephosphorylating a synthetic phosphotyrosine peptide substrate was decreased in the presence of sodium stibogluconate in a dose-dependent manner with sodium stibogluconate at 100 µg/ml resulted in 80-90% of inhibition of the PTPases (FIG. 30A). These effects of sodium stibogluconate were detected under the condition that the PRLs were pre-incubated with the drug for 10 minutes prior to the initiation of PTPase assays by addition of substrate to the reactions. Because the three phosphatases were inhibited in a similar manner by sodium stibogluconate, PRL-3 was selected to further investigate the effect of prolonged pre-incubation with sodium stibogluconate on its phosphatase activity. Pre-incubation of PRL-3 with sodium stibogluconate for 30 or 60 minutes resulted in more dramatic inhibition with nearly complete inactivation of PRL-3 occurring at sodium stibogluconate concentration of 10 µg/ml (FIG. 30B). Inhibition of PRL-3 by sodium stibogluconate was also detected using an alternative substrate (DiFMUP) while the known phosphatase inhibitors sodium orthovanadate (Burke et al., Biopolymers 47, 225 (1998)) and suramin (Zhang, et al., J. Biol. Chem. 273, 12281 (1998)) were less effective than sodium stibogluconate under comparable conditions (FIG. 30C). Sodium stibogluconate induced similar inhibition of recombinant PRL-3 and recombinant SBP-1 (FIG. 30D). Inhibition of PRL-3 by sodium stibogluconate was not relieved by a washing process (FIG. 30E) effective in removing the inhibition of SHP-1 by reversible inhibitor suramin (Pathak et al., J. Imumol. 167, 3391 (2001)).

These results demonstrated that sodium stibogluconate was a potent and irreversible inhibitor of recombinant PRL phosphatases in vitro.

2. Sodium Stibogluconate Inactivates Intracellular PRLs in NIH3T3 Transfectants.

The effects of sodium stibogluconate on intracellular PRL phosphatases were next investigated to determine whether sodium stibogluconate is an inhibitor of PRLs in vivo.

An expression construct of Flag-tagged PRL-1 or control vector was transfected into NIH3T3 cells which were then treated without or with sodium stibogluconate and used for immunoprecipitation assays with a monoclonal anti-Flag antibody. The immunocomplexes were analyzed by SDS-PAGE/Western blotting and PTPase assays. A Flag-tagged protein with a molecular weight approximately 22 kDa as expected for Flag-PRL-1 was detected in the immunocomplexes from untreated or sodium stibogluconate-treated Flag-PRL-1 transfectants but not in those from the control cells (FIG. 31A). Immunocomplexes from untreated Flag-PRL-1 transfectants showed a markedly higher PTPase activity (about 23 folds) over that of control transfectants (FIG. 31B). In contrast, immunocomplexes from sodium stibogluconate-treated Flag-PRL-1 transfectants had little PTPase activities that were at levels similar to those of the control cells (FIG. 31B). Such a lack of PTPase activity was also evident in the immunocomplexes from sodium stibogluconate-treated NIH3T3 transfectants of Flag-PRL-2 (FIG. 31D) or Flag-PRL-3 (FIG. 31F) although Flag-tagged PRLs were present at similar levels in the immunocomplexes from the untreated or sodium stibogluconate-treated cells (FIG. 31C and E).

These results demonstrated that sodium stibogluconate treatment inactivated intracellular PRLs in the transfectants, indicating that sodium stibogluconate is an effective inhibitor of the phosphatases in vivo.

3. Sodium Stibogluconate Induces Prolonged PRL-2 Inactivation in NIH3T3 Transfectants.

In light of the observation that sodium stibogluconate inactivates intracellular PRLs, the issue of the duration of sodium stibogluconate-induced inactivation of PRLs was addressed. Because sodium stibogluconate was equally effective against each of the PRLs (FIG. 31), the duration of sodium stibogluconate-induced inaction of a single PRL in NIH3T3 transfectants was determined.

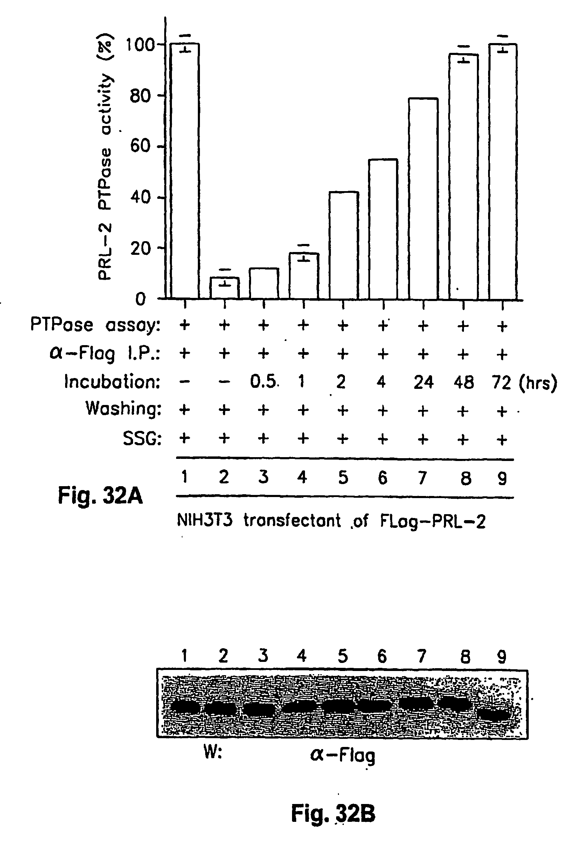

Flag-PRL-2 transfectants were briefly treated with sodium stibogluconate for 5 minutes, washed to remove cell-free drug and then incubated for various times prior to termination by cell lysis. Anti-Flag immunocomplexes from the cells were analyzed by SDS-PAGE/Western blotting and PTPase assays. The amounts of Flag-PRL-2 proteins in the immunocomplexes were at similar levels as quantified by probing with an anti-Flag antibody (FIG. 32B). Immunocomplexes from cells treated with sodium stibogluconate showed a markedly reduced PTPase activity in comparison to that from the control (comparing lanes 1 and 2 of FIG. 32A), consistent with inactivation of PRL-2 by sodium stibogluconate treatment. Immunocomplexes from cells incubated for different times following sodium stibogluconate-treatment and cell washing showed a gradual increase of PTPase activity in a time-dependent manner above the level of sodium stibogluconate-treated cells (FIG. 32A, lanes 3-9). PTPase activity of the immunocomplexes from cells incubated for 24 hours (FIG. 32A, lane 7) was 78% of the untreated cells. Immunocomplexes from cells incubated for 48-72 hours (FIG. 32A, lanes 8 and 9) showed PTPase activities similar to that of the untreated cells.

These results demonstrated that a brief sodium stibogluconate treatment had a prolonged effect on intracellular PRL-2 activity that required at least 24 hours for its full removal in NIH3T3 transfectants.

4. Sodium Stibogluconate Inhibits the In Vitro Growth of Human Cancer Cell Lines that Express PRL Phosphatases.

Given the demonstrated oncogenic activity of the PRL phosphatases and the association of PRL-3 over-expression with metastasis of colon cancer, it was thought that sodium stibogluconate might inactivate PRLs in human cancer cells and hence have anti-cancer activity. The expression levels of PRLs in a panel of human cancer cell lines and the effects of sodium stibogluconate on in vitro growth of the cell lines was determined.

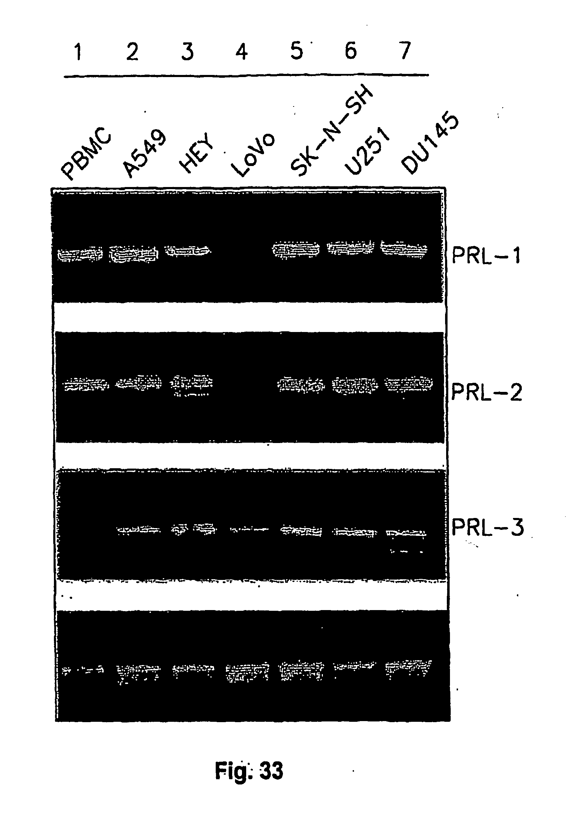

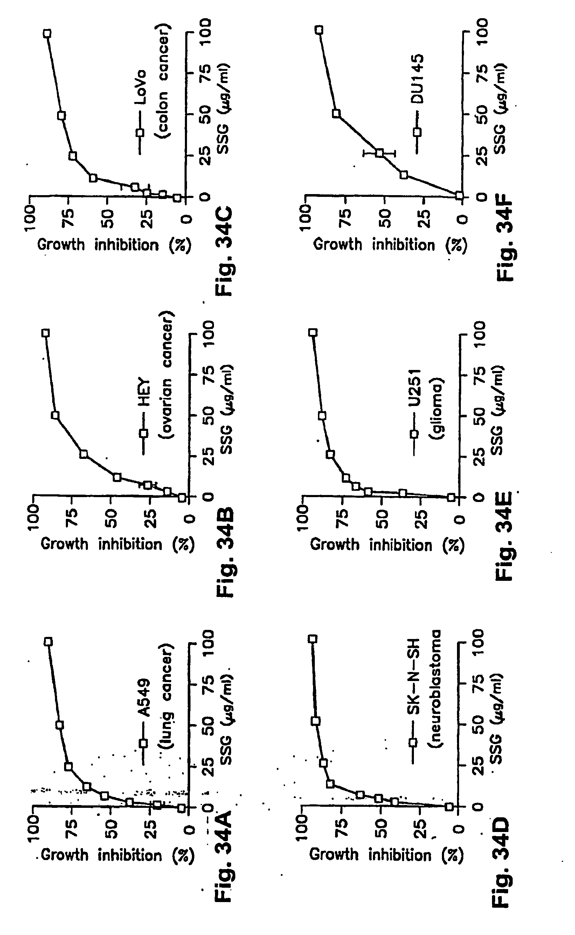

Expression of PRLs was detected in cell lines of human lung cancer (A549), ovarian cancer (Hey), colon cancer (LoVo), neuroblastoma (SK-N-SH), glioma (U251) and prostate cancer (DU145) (FIG. 33). In vitro growth of the cell lines was inhibited in the presence of sodium stibogluconate in a dose-dependent manner (FIG. 34). Sodium stibogluconate at 100 µg/ml resulted in near-complete cell killing of the cell lines under the experimental conditions while the drug at lower doses also showed significant growth suppression effects against these cell lines (FIG. 34). Among the cell lines, SK-N-SH and U251 cells were most sensitive to the drug, which at 25 µg/ml resulted in approximately 80% growth inhibition (FIG. 34). This dose of sodium stibogluconate caused about 50% growth inhibition in the less sensitive DU145 cells and about 60-76% growth inhibition in the remaining cell lines (FIG. 34).

These results demonstrated an in vitro growth inhibitory activity of sodium stibogluconate against various human cancer cell lines that expressed the PRL phosphatases at different levels.

5. Sodium Stibogluconate at a Nontoxic Dose Inhibits the Growth of DU]45 Tumors in Nude Mice.

To further assess the anti-cancer activity of sodium stibogluconate in vivo, the effects of sodium stibogluconate on the growth of DU145 tumors in nude mice were determined.

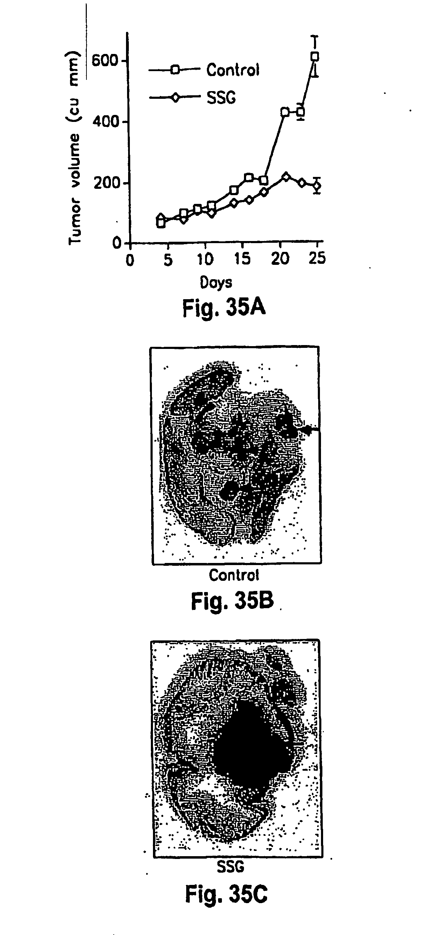

Nude mice were inoculated with DU145 cells sub-cutaneously at the shoulder area. Two days after inoculation when tumors were visible, the mice were subjected to no treatment (control) or sodium stibogluconate treatment (daily injection of 440 mg/kg, intermuscular at the hip area). DU145 tumors showed aggressive growth in control mice (FIG. 35A) (data represent mean+SEM (n=88)), consistent with a previous report (Mickey, et al., Cancer Res. 37, 4049 (1977)). Sodium stibogluconate treatment inhibited the growth of DU145 tumors which were approximately 30% in comparison to the tumor volume in the control mice (FIG. 35A). Histologic evaluation of the inoculation sites at the end of the treatment course revealed the presence of clusters of small tumors in the sodium stibogluconate-treated mice (FIG. 35C) in contrast to the single and much larger tumor in the controls (FIG. 35B). All mice in both groups survived until the end of the study. Histology of their major organs was unremarkable.

These results demonstrated a growth inhibitory effect of sodium stibogluconate against DU145 tumors in mice associated with no obvious toxicity and provide evidence that sodium stibogluconate at a non-toxic dose has anti-tumor activity in vivo.

6. Sodium Stibogluconate-Resistant DU145R Cells Express a Mutated form of PRL-1 Phosphatase Insensitive to Sodium Stibogluconate Inhibition.

The observation that DU145 cells in culture were relatively insensitive to sodium stibogluconate (FIG. 34) suggested the possibility that DU145 cells might contain a subpopulation resistant to the drug. This notion was also consistent with the presence in sodium stibogluconate-treated mice of clusters of small DU145 tumors (FIG. 35C), which might be resulted from outgrowth of such sodium stibogluconate-resistant cells. Given that PRLs are oncogenic phosphatases expressed in DU145 cells (FIG. 33) and that sodium stibogluconate inhibits recombinant (FIG. 30) and intracellular PRLs (FIG. 31), whether DU145 cells contain a sodium stibogluconate-resistant cell population that expresses sodium stibogluconate-insensitive PRL mutants was further investigated.

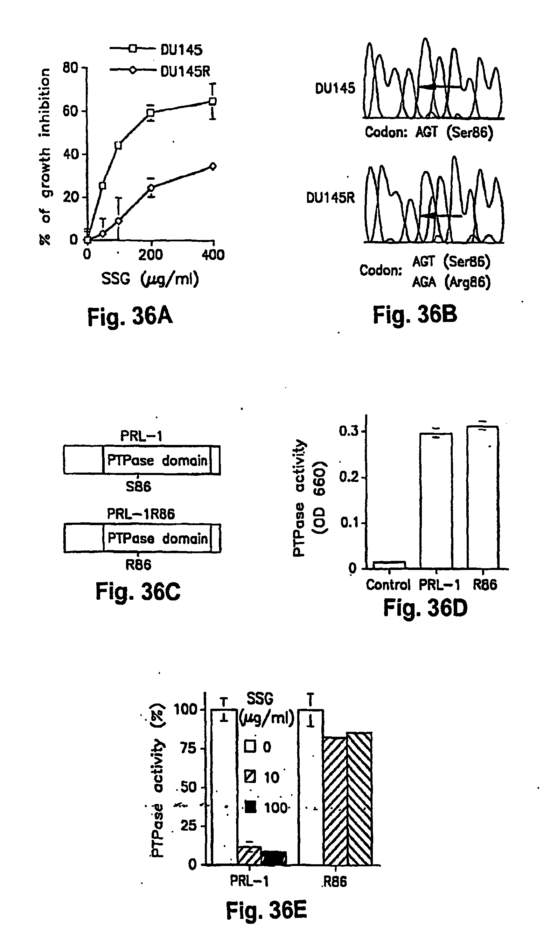

DU145 cells were cultured in the presence of sodium stibogluconate (100 µg/ml) for 4 weeks. While most of the cells died during the period, some of the cells survived and formed distinct clones. One of the clones (DU145R) was isolated for further characterization and showed growth resistance to sodium stibogluconate in culture in comparison to the parental DU145 cells (FIG. 36A, data represent mean+s.d. values of triplicate samples). Sequence analysis of the cDNAs of the coding region of PRLs from DU145 cells and the sodium stibogluconate-resistant colony revealed that the cDNAs of PRL-2 and PRL-3 were of wild type. Interestingly, the cDNA of PRL-1 from DU145R showed at position 259 the presence of nucleotide T, which corresponds to that of a wild type PRL-], as well as nucleotide A (FIG. 36B) that would result in the substitution of a serine (S86) with an arginine residue (R86) in the phosphatase domain of the PRL-1 protein (FIG. 36C). The remaining sequence of the PRL-1 cDNA from DU145R cells was of the wild type. PRL-1 cDNA from the parental DU145 cells was of the wild type (FIG. 36B).

A recombinant PRL-1 protein containing R86 was prepared and showed in vitro PTPase activity similar to that of wild type PRL-1 (FIG. 36D; data represent mean+s.d. values of triplicate samples). However, its PTPase activity was only reduced by less than 20% in the presence of sodium stibogluconate in contrast to the 90% inhibition of the wild type PRL-1 induced by the drug under comparable conditions (FIG. 36E; data represent mean+s.d. values of triplicate samples).

These results demonstrated that DU145 contained sodium stibogluconate-resistant cells in which a mutated PRL-1 protein was co-expressed with the wild type phosphatase. Because the mutant PRL-1 was an active phosphatase insensitive to sodium stibogluconate inhibition, it might act dominantly to mediate cancer cells' resistance to the drug. The fact that the mutation was undetectable by sequence analysis of PRL-1 cDNA of the parental DU145 cells suggested that it was only present in a small cell population, a notion consistent with the limited number of small DU145 tumors in sodium stibogluconate-treated mice (FIG. 35).

7. Intracellular PRL-1R86 is Insensitive to Sodium Stibogluconate Inhibition and Confers Resistance to Sodium Stibogluconate-Induced Growth Inhibition in WM9 Melanoma Cells.

To evaluate the role of PRL-1R86 in cancer cell resistance to sodium stibogluconate, cDNAs encoding Flag-tagged PRL-1 or R86 mutant were cloned into the pBabapuro vector (Yang, et al., Blood 91, 3746 (1998)) and transfected into WM9 human melanoma cell line, in which the endogenous PRLs were expressed and had no mutation in their coding region as determined by RT-PCR and sequencing analysis. Stable transfected cell populations were derived following puromycine selection.

To determine whether the R86 mutant in WM9 cells is an active phosphatase insensitive to sodium stibogluconate inhibition, WM9 transfectants were untreated or treated with sodium stibogluconate for 5 minutes, washed to remove cell-free drug and lysed in lysis buffer. Anti-Flag immunocomplexes from cell lysates were characterized by SDS-PAGE/Western blotting and PTPase assays. As expected, Flag-tagged PRL-1 and R86 mutant proteins were detected in the immucomplexes from the corresponding transfectants, but not from vector control cells (FIG. 37A). The immunocomplexes from untreated Flag-PRL-1 and Flag-R86 transfectants showed phosphatase activities well above the background activity of the vector control cells (FIG. 37B; data represent mean+s.d. values of triplicate samples), demonstrating that the PRL-1 and R86 expressed in the transfectants were both active phosphatases. Interestingly, phosphatase activities of immunocomplexes from sodium stibogluconate-treated R86 transfectant showed only a modest decrease (20-30%) in comparison to that of the untreated R86 cells (FIG. 37B) whereas phosphatase activities of the immunocomplexes from sodium stibogluconate-treated PRL-1 transfectant were inhibited 52-90 % in a sodium stibogluconate treatment dose-dependent manner (FIG. 37B). Thus the intracellular PRL-1R86 mutant phosphatase was insensitive to sodium stibogluconate inhibition.

To further assess whether expression of the PRL-1R86 mutant affects sodium stibogluconate-induced growth inhibition, the transfectants were cultured in the absence or presence of sodium stibogluconate for 6 days with viable cells determined by MTT assays. The transfectants showed similar growth in the absence of sodium stibogluconate (FIG. 37C; data represent mean+s.d. values of triplicate samples). In the presence of sodium stibogluconate, the growth of PRL-1 and vector control cells was inhibited in a dose-dependent manner (FIG. 37D, data represent mean+s.d. values of triplicate samples). However, the growth of the R86 transfectant was not inhibited by sodium stibogluconate at 12.5 or 25 µg/ml, which suppressed the growth of the other transfectants by 20-40 % (FIG. 37D). Sodium stibogluconate at higher doses (50 or 100 µg/ml) induced only modest growth inhibition of R86 cells in comparison to its effects on PRL-1 cells (FIG. 37D). Thus, sodium stibogluconate was less effective in inhibiting the growth of the PRL-1R86 transfectant, demonstrating that ectopic expression of PRL-1R86 in WM9 cells conferred resistance to the growth inhibitory activity of sodium stibogluconate.

8. Meglumine Antimonate (Glucantime) Inhibits SHP-1 and PRL-3

To assess whether other antimony based compounds would also act as PTPase inhibitors, the effect of meglumine antimonate (glucantime) against SHP-1 and PRL-3 was analyzed by in vitro PTPase assays.

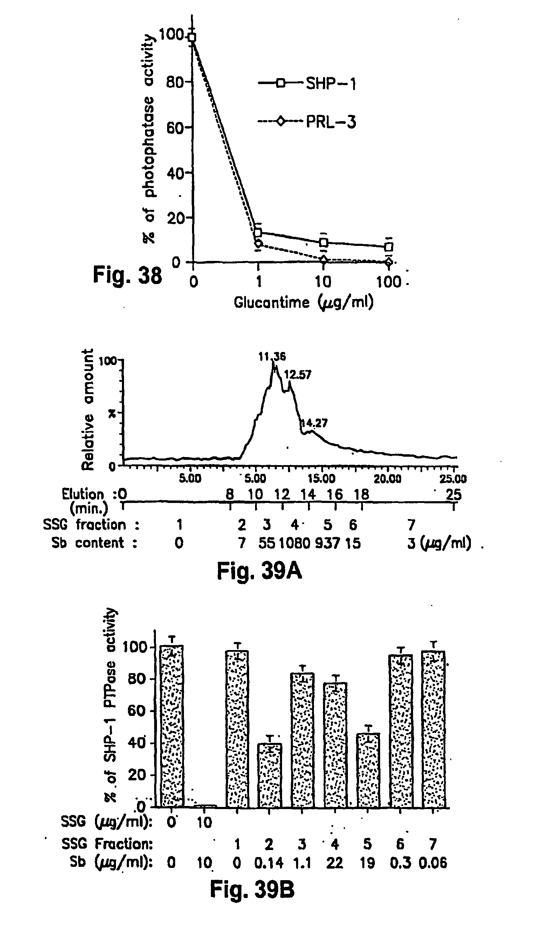

PTPase activity of SHP-1 and PRL-3 in dephosphorylating a synthetic phosphotyrosine peptide substrate was decreased in the presence of meglumine antimonate in a dose-dependent manner (FIG. 38). Meglumine antimonate levels above 1 µg/ml showed 85-95% inhibition of the PTPases (FIG. 38). With meglumine antimonate at 100 µg/ml approximately 90% of SHP-1 was inhibited and approximately 100% of PRL-3 was inhibited (FIG. 38).

C. Discussion

These results demonstrated that sodium stibogluconate is an inhibitor of PRLs. Sodium stibogluconate, in a dose-dependent manner, inhibited the activity of recombinant PRLs in vitro (FIG. 30) and intracellular PRLs in NIH-3T3 transfectants (FIG. 31). Sodium stibogluconate treatment resulted in near complete inactivation of recombinant PRL-3 in vitro (FIG. 30B) and intracellular PRLs (FIG. 31) at 10 µg/ml, similar to its potency against its previously identified PTPase target SHP-1 (Pathak et al., J. Imumol. 167, 3391 (2001)). In contrast, SHP-2 was less sensitive to sodium stibogluconate and required sodium stibogluconate at 100 µg/ml for a comparable level of inhibition while the drug had little activity against MKPI phosphatases as shown in previous studies described above. Importantly, the effective dose of sodium stibogluconate against PRLs is well within the clinically achievable in vivo levels of the drug, which is administrated at 10-20 mg/kg daily in standard sodium stibogluconate therapy (Herwaldt et al., Am. J. Trop. Med. Hyg. 46, 296 (1992)). Given that a brief exposure to sodium stibogluconate resulted in inhibition of intracellular PRLs with a lasting effect of more than 24 hours (FIG. 31), inactivation of PRLs in vivo could occur during sodium stibogluconate therapy. The observation that sodium stibogluconate-induced PRL-3 inactivation in vitro was removed by a washing process (FIG. 30E) indicates an irreversible inhibitory mechanism. Such a mode of action is consistent with the long duration of sodium stibogluconate-induced inhibition of intracellular PRL-2 (FIG. 31) and the association of more effective inactivation of PRL-3 with prolonged pre-incubation of sodium stibogluconate with the phosphatase (FIG. 30B).

This data also demonstrated that sodium stibogluconate has anti-cancer activity. Sodium stibogluconate at a dose-dependent manner inhibited in vitro growth of various human cancer cell lines, including prostate cancer cell line DU145 (FIG. 34). Sodium stibogluconate's anti-cancer activity in vivo was demonstrated by the inhibition of DU145 tumor outgrowth in nude mice by sodium stibogluconate at a non-toxic dose (FIG. 35). The fact that the other cancer cell lines were more sensitive than DU145 to sodium stibogluconate in vitro (FIG. 34) indicates the possibility that the drug might be even more effective against their tumors in mouse models under comparable experimental conditions. These results suggested sodium stibogluconate as a candidate therapeutic for cancer treatment and provide pre-clinical data for its further clinical evaluation.

Significantly, these results provided several lines of evidence suggesting that sodium stibogluconate anti-cancer activity is mediated at least in part by inactivation of PRLs in cancer cells. The cancer cell lines against which sodium stibogluconate showed a growth inhibitory activity in vitro (FIG. 34) all expressed PRLs (FIG. 33). These phosphatases were inhibited by sodium stibogluconate as recombinant proteins (FIG. 30) or in their intracellular environment (FIGS. 31 and 37). Moreover, we showed that a sodium stibogluconate-resistant clone of DU145 cells expressed a mutated form of PRL-1, an active phosphatase insensitive to inhibition by sodium stibogluconate (FIG. 36), that conferred resistance to sodium stibogluconate-induced growth inhibition when ectopically expressed in WM9 melanoma cells (FIG. 37). These results suggest that inactivation of PRL-1, and probably the other PRLs, plays a key role in sodium stibogluconate-induced growth inhibition of the cancer cells. In particular, PRL-1 might be mainly responsible for mediating the growth inhibitory activity of sodium stibogluconate at a dose range of 12.5-25 µg/ml, which showed no growth inhibitory activity against the PRL-TR86 transfectant, but was effective in suppressing the growth of the PRL-1 transfected cells (FIG. 37D). Consistent with this notion, these doses of sodium stibogluconate were effective in inhibiting recombinant and intracellular PRLs (FIGS. 30, 31, 36 and 37) but not the PRL-1R86 mutant (FIGS. 36 and 37B). Although sodium stibogluconate also showed a similar potency in inhibiting SHP-1 (FIG. 30), this PTPase expresses predominantly in hematopoietic cells (Yi, et al., Blood 78, 2222 (1991); Yi, et al., Molecular & Cellular Biol. 12, 836 (1992)) and is not expected to be present in the studied cancer cell lines that are not hematopoietic (FIG. 34). Indeed, absence of SHP-1 expression in WM9 melanoma cells was confirmed by western blotting using an anti-SHP-1 antibody. Thus, sodium stibogluconate growth inhibitory activity against WM9 cells and WM9 transfectants functioned independently of SHP-1. However, inhibition of SHP-1 by sodium stibogluconate may occur in hematopoietic cells and play a role in sodium stibogluconate activity to augment cytokine signaling that is negatively regulated by the PTPase (Pathak et al., J. Imumol. 167, 3391 (2001)).

Given the demonstrated growth inhibitory activity of sodium stibogluconate against cancer cell lines in vitro and against DU145 tumors in mice that is likely mediated via inactivation of PRLs in cancer cells, sodium stibogluconate might be beneficial in human malignancies in which the oncogenic phosphatases are consistently expressed and play a pathogenic role. Although only increased expression of PRL-3 in metastatic colon cancer has been reported so far (Bradbury, Lancet 358,1245 (2001); Saha, et al., Science 294, 1343 (2001)), the fact that PRLs were detected at significant expression levels in various human cancer cell lines (FIG. 33) suggests the possibility that expression of the phosphatases could be common in human malignancies. Further studies to assess the expression levels of PRLs in human tumor samples will provide crucial information in identification of types and stages of human malignancies potentially sensitive to sodium stibogluconate therapy for clinical evaluation. In this regard, identification of a sodium stibogluconate-insensitive PRL-1 mutant indicates the value of sequence analysis of PRLs to identify sodium stibogluconate-sensitive or sodium stibogluconate-resistant human tumors in cancer patients, in which the PRL-1 mutation could serve as a sodium stibogluconate-resistance marker. Moreover, the sodium stibogluconate-insensitive PRL-1 mutant provides a basis to develop inhibitors against sodium stibogluconate-insensitive PRLs as alternative anti-cancer therapeutics.

Identification of sodium stibogluconate as the first clinically usable inhibitor of PRLs with anti-cancer activity represents a significant breakthrough in developing PTPase inhibitors as targeted therapeutics and opens up new research areas for further mechanistic studies. The ability of antimony in sodium stibogluconate to form covalent bonds with sulfhydryl group (Berman and Grogl. 1988) and the existence of a catalytic cysteine residue in all tyrosine phosphatases (Hooft van Huijsduijnen. 1998) suggest modification of the cysteine by pentavalent antimony in sodium stibogluconate as a potential inactivation mechanism. This putative mode of action is consistent with the irreversible inhibition of recombinant PRL-3 (FIG. 30) and SHP-1 (Pathak et al., J. Imumol. 167, 3391 (2001)) by sodium stibogluconate as well as the long duration of sodium stibogluconate-induced inhibition of intracellular PRL-2 (FIG. 32). This putative mode further implicates the organic moiety of sodium stibogluconate in providing a configuration complementary to the PTPase catalytic pocket to facilitate antimony/cysteine interaction and, thus, define PTPase specificity of the inhibitor. Further, the proposed mode provides a rational explanation for the insensitivity of MKP1 (Pathak et al., J. Imumol, 167, 3391 (2001)) and PRL-1R86 mutant (FIG. 36 and 37) to sodium stibogluconate inhibition. Consistent with this hypothesis, meglumine antimonate (pentavalent antimony conjugated to N-methylglucamine) was found to have PTPase inhibitory activity against SHP-1 and PRL-3 (FIG. 38) as well as other PTPases (some of which were not affected by sodium stibogluconate). It might therefore be feasible to develop novel and more specific PTPase inhibitors based on compounds comprised of antimony conjugated to different organic moieties. Sodium stibogluconate may thus represent a new class of PTPase inhibitors that could be further developed as novel therapeutics and experimental tools.

VI. PTPase Inhibitory Activity is Associated with Selective Compounds in Sodium Stibogluconate

To determine whether select or all compounds in a sodium stibogluconate mixture are effective PTPase inhibitors and whether PTPase inhibitory activity of sodium stibogluconate is solely defined by Sb, sodium stibogluconate was fractionated by chromatography. Sb content and PTPase inhibitory activity of individual fractions were determined.

A. Materials and methods

A sodium stibogluconate mixture was separated by HPLC in a Jordi gel column (Jordi 100A; Jordi Associates, Bellingham, Mass.), eluted with water at 0.2 ml/min, and collected as fractions during elution. Relative amounts of compounds in the elates were monitored by mass spectrometry (full scan). Sb contents of sodium stibogluconate and sodium stibogluconate fractions were quantified by inductive coupled plasma mass spectrometry following standard procedures with Sb solution standards, sodium stibogluconate, and sodium stibogluconate fractions prepared in a uniformed matrix of 0.8 M HNO₃ and 1.2 M HCl. Indium was used as an internal standard. The calibration curve was stable over the course of the analysis (drift in slope=0.29%). Values of Sb contents of the samples had about. 10% maximum relative error based on the calculation of all systematic and random errors. Total amounts of Sb detected in the collected eluates were about 90% of input sodium stibogluconate for chromatography.

B. Results and Discussion

Compounds in the sodium stibogluconate mixture were eluted in a time-dependent manner during chromatography, with most of them eluted between 8 and 25 minutes as revealed by mass spectrometry scanning (FIG. 39A). Consistent with a lack of compounds in fraction 1 (eluate of 0-8 minutes), no Sb was detected in the fraction by inductively coupled plasma mass spectrometry (FIG. 39A). Fractions 2-7 showed various amounts of Sb content with the highest levels detected in fractions 4 and 5 that accounted for 96% of total Sb in the eluates (FIG. 39A).

Inhibitory activities of the fractions and the parental sodium stibogluconate mixture against recombinant SHP-1 PTPase was assessed by in vitro PTPase assays. Sodium stibogluconate at Sb concentration of 10 µg/ml inactivated SHP-1 (FIG. 39B; data represent the mean +/- SD values of triplicate samples). As expected because it contained no detectable compounds or Sb (FIG. 39B), fraction 1 showed no activity against SHP-1 (FIG. 39B). Fractions 6 and 7 also failed to inhibit the PTPase although they had low levels of Sb (FIG. 39B). Interestingly, fraction 2, with an Sb level similar to those in fractions 6 and 7, was active against SHP-1 (FIG. 39B). In contrast, fractions 3 and 4 showed only minor effects on SHP-1 PTPase activity (FIG. 39B) despite the fact that their Sb levels were .about. 10- to 20-fold higher than that of fraction 2 (FIG. 39B). Fraction 5 also showed a significant activity against SHP-1 although its Sb level was almost 100-fold higher that that of fraction 2 (FIG. 39B). Recombinant SHP-2 was also inhibited by fractions 2 and 5, but was not affected by the other fractions under comparable conditions.

These results demonstrated that inhibitory activity against recombinant SHPs associated with selective compounds in the sodium stibogluconate mixture in a manner not solely defined by Sb contents. The fact that fraction 2 accounted for <10% of the total compounds in sodium stibogluconate, but was effective in inhibiting PTPases despite its relatively low Sb concentration (FIG. 39A), suggests that only a small portion of the compounds in sodium stibogluconate are mainly responsible for the PTPase inhibitory activity of the drug. These results indicate that the active compounds in sodium stibogluconate might be purified as a more potent and less toxic PTPase-targeted therapeutic. Identifying more precisely the most active sodium stibogluconate species may also provide a basis for defining the chemical structure of sodium stibogluconate and interactions with targeted PTPases. These identified molecules may also provide a starting point for rational design of novel PTPase inhibitors.

VII. Levamisole, Pentamidine, and Ketoconazole had PTPase Inhibitory Activity In Vitro

To assess whether the drugs levamisole, pentamidine, and ketoconazole, which are known to be effective against leishmaniasis, would act as PTPase inhibitors, the effects of these drugs were examined in vitro.

A. Methods

In vitro PTPase assays were used to determine the effects of levamisole (Sigma), pentamidine (American Pharmaceutical Partners, Inc.), and ketoconazole (Sigma) on recombinant PTPases, following established procedures (Pathak et al., J. Imumol. 167, 3391 (2001); Pathak et al., Leukemia 16, 2285 (2002), Yi et al., J. Immunol. 169, 5978 (2002)) using a synthetic phosphotyrosine peptide or DiFMUP as the substrate as detailed above in section I.A.2.

B. Results and Discussion

1. Levamisole, Pentamidine, and Ketoconazole Demonstrate PTPase Inhibitory Activity In Vitro.

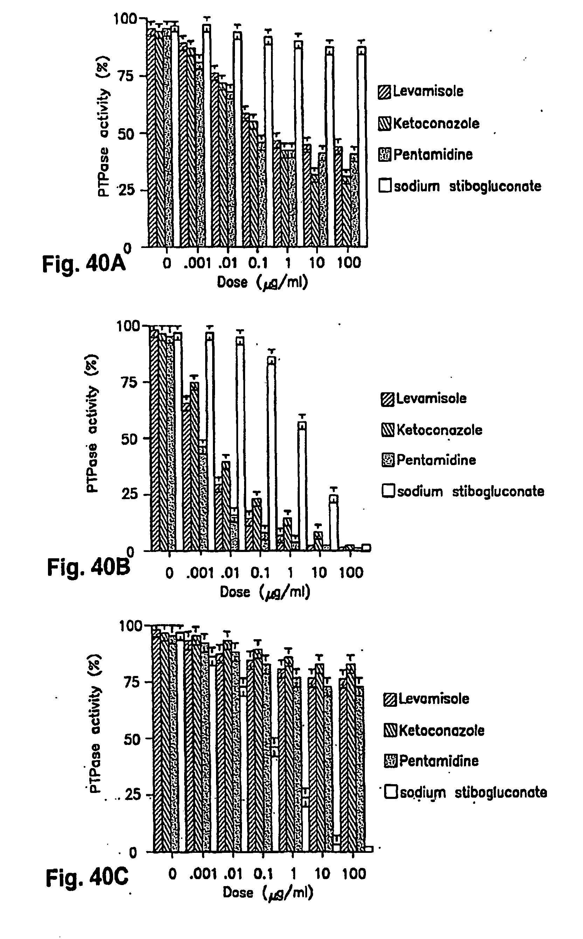

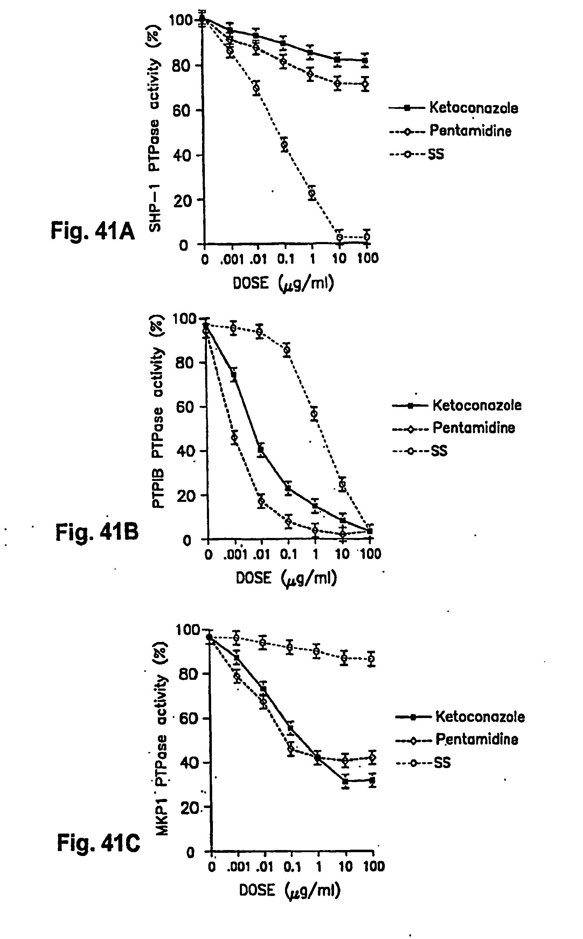

The activities of levamisole, pentamidine, and ketoconazole were determined against SHP-1, PTP1B, and MLP1 (FIGS. 34 and 35) and GSTm8 (FIG. 40C) in in vitro PTPase assays. Unlike sodium stibogluconate, levamisole, pentamidine, and ketoconazole showed no obvious inhibitory activity against SHP-1 (FIG. 41 A) or against GSTm8 (FIG. 40C) at therapeutic concentrations (3-4 µg/ml) or at higher concentrations (10-100 µg/ml). However, these drugs achieved significant inhibition (approximately 80-98%) of PTP1B activity at 0.1 to 1 µg/ml while sodium stibogluconate was only modestly effective (FIGS. 40B and 41B). Further, the three drugs also showed substantial inhibition of MKP1, to which SS had no obvious effects (FIG. 41C).

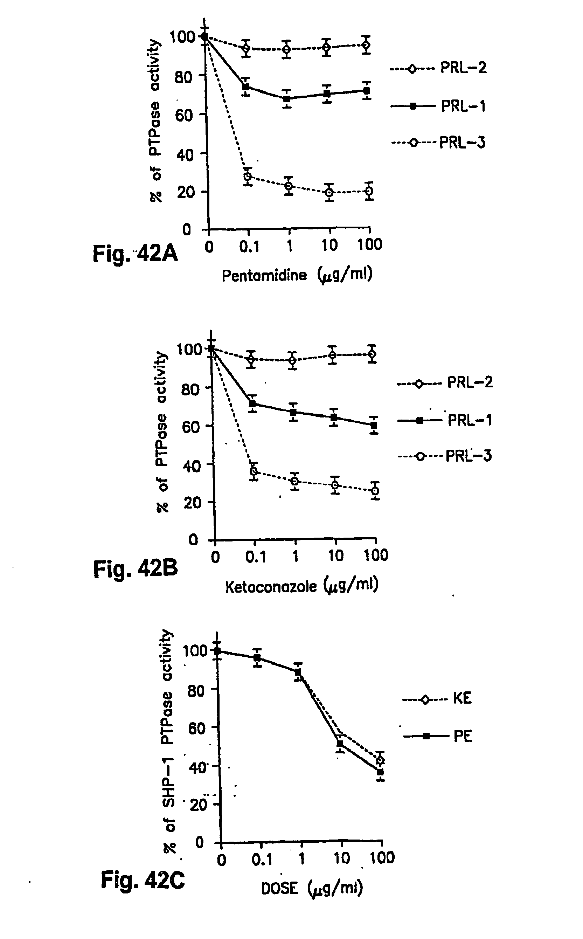

The activities of pentamidine and ketoconazole were also determined against PRL-1, PRL-2, and PRL-3 in in vitro PTPase assays (FIG. 42). Pentamidine was effective against PRLs-3 at therapeutic concentrations above 0.1-100 µg/ml decreasing PTPase activity to 20-30 % (FIG. 42A). Pentamidine was not very effective against PRL-1; the PTPase retained approximately 70-80% of its activity for 0.1-100 µg/ml dosing of the drug (FIG. 42A). Pentamidine was not effective against PRL-2 (FIG. 42A). Ketoconazole was effective against PRL-3 at therapeutic concentrations above 0.1-100 µg/ml decreasing PTPase activity to approximately 25-40% (FIG. 42B). Ketoconazole was not very effective against PRL-1 the PTPase retained approximately 60-70% of its activity for 0.1-100 µg/ml dosing of the drug (FIG. 42B). Ketoconazole was not effective against PRL-2 (FIG. 42B).

These results demonstrated in vitro that pentamidine and ketoconazole have inhibitory activities against certain PTPases and that PTPases have different sensitivities to sodium stibogluconate, pentamidine, and ketoconazole. The results suggested that pentamidine and ketoconazole may target cellular PTPases as a major mechanism of their anti-leishmania activity and that they may therefore have anti-cancer activities as well. Significantly, the apparent preference of sodium stibogluconate, pentamidine, and ketoconazole in targeting different PTPases suggested that these inhibitors may have activities against different types of cancer cells, each of which may require distinct PTPases for its malignant phenotype.

2. Pentamidine Inhibits the Growth of WM9 Cells and Augments IFN-Alpha-Induced Growth Inhibition of WM9 Cells In Vitro.

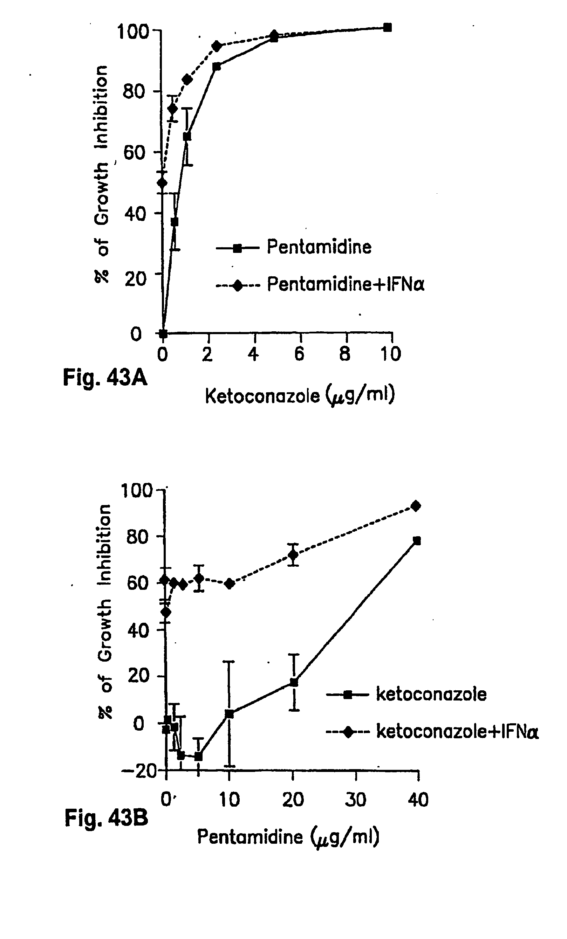

To assess their potential anti-cancer activities, the effects of pentamidine and ketoconazole as a single agent and in combination with IFN-alpha on WM9 cell growth in vitro were determined (FIG. 43).

Pentamidine showed a striking growth inhibitory activity as a single agent (FIG. 43A). Pentamidine achieved 86-97% inhibition at 2.5-5 µg/ml, concentrations that are similar to its therapeutic dosage (2-4 mg/kg) (FIG. 43A). The drug augmented IFN-alpha-induced growth inhibition, most obviously at 0.625-1.25 µg/ml concentrations. These results suggest that pentamidine has a significant anti-cancer activity and interacts with IFN-alpha.

Ketoconazole at concentrations of 0.625-20 µg/ml had no apparent activity against WM9 cells as a single agent or in combination with IFN-alpha (FIG. 43B). At a higher concentration (40 µg/ml), ketoconazole achieved 67% growth inhibition as a single agent and had a minor augmenting effect on IFN-alpha activity (FIG. 43B). Since both drugs showed similar activities in inhibiting PTP1B and MKP1 (FIG. 41), their differential effects against WM9 cells suggest that PTP1B and MKP1 are unlikely to be key target PTPases responsible for the anti-cancer activity of pentamidine.

While various features of the claimed invention are presented above, it should be understood that the features may be used singly or in any combination thereof. Therefore, the claimed invention is not to be limited to only the specific embodiments depicted herein.

Further, it should be understood that variations and modifications may occur to those skilled in the art to which the claimed invention pertains. The embodiments described herein are examples of the claimed invention. The disclosure may enable those skilled in the art to make and use embodiments having alternative elements that likewise correspond to the elements of the invention recited in the claims. The intended scope of the invention may thus include other embodiments that do not differ or that insubstantially differ from the literal language of the claims. The scope of the present invention is accordingly defined as set forth in the appended claims.

**TABLE 1**

| Growth inhibition of human tumor cell lines by sodium stibogluconate and IFN-alpha | | | | | | |
|---|---|---|---|---|---|---|
| | | % growth inhibition by day 6 (+/- s.d.) | | | | |
| | | | | | (SS 12.5 µg/ml; | |
| Cell Line | Tumor Type | SS | IFN.alpha. | SS + IFN.alpha. | SS | SS + IFN.alpha |
| DR | Burkitt's Lymphoma | 45(15) | 39(2) | 80(1) | 99(1) | 99(2) |
| U266 | Multiple myeloma | +3(4) | 78(10) | 93(5) | 64(10) | 100(7) |
| H9 | T-lymphoma | 8(16) | 86(3) | 91(3) | nd | 99(3) |
| Peer | T-ALL | +3(5) | 86(4) | 91(3) | nd | 98(2) |
| WM9 | Melanoma | 27(12) | 58(2) | 84(3) | 75(4) | 100(1) |
| WM35 | Melanoma | +8(21) | 19(3) | +3(11) | 2(19) | 29(10) |
| DU145 | Prostate cancer | 36(1) | 70(5) | 85(6) | 91(2) | 96(2) |
| C42 | Prostate cancer | 0(18) | +19(30) | 2(18) | 15(6) | 21(7) |
| MDA231 | Breast cancer | 64(9) | 79(2) | 93(2) | 97(5) | 95(3) |
| MDA235 | Breast cancer | 6(2) | 29(15) | 40(39) | 97(2) | 95(3) |
| WiT49-N1 | Wilms tumor | 50(8) | 22(11) | 31(10) | 97(3) | 92(0) |
| RC45 | Renal cell carcinoma | 18(13) | 70(15) | 79(7) | 66(13) | 85(7) |
| 5637 | Bladder carcinoma | 23(7) | 28(17) | 23(6) | 74(9) | 71(7) |

### Sequence CWU 1

[0329] 9 1 31 DNA DH5a Bacteria 1 ctggatcctg cgggggctgc tgcaggagcg c 31 2 29 DNA DH5a Bacteria 2 aagtcgacgc agcttgggga ggtggtgat 29 3 13 PRT Unknown Artificial peptide created to act as a PTPase substrate. 3 Arg Arg Leu Ile Glu Asp Ala Glu Tyr Ala Ala Arg Gly 1 5 10 4 32 DNA Human 4 taggatcccg ggaggcgcca tggctcggat ga 32 5 32 DNA Human 5 gagtcgacca taacgcagca ccgggtcttg tg 32 6 33 DNA Human 6 taggatcccc ataatgaacc gtccagcccc tgt 33 7 32 DNA Human 7 gagtcgacct gaacacagca atgcccattg gt 32 8 33 DNA Human 8 taggatcccc aacatggctc gaatgaaccg ccc 33 9 32 DNA Human 9 gagtcgactt gaatgcaaca gttgtttcta tg 32

## Claims

1. A therapeutic composition for use in the treatment of cancer comprising sodium stibogluconate and IL-2.

2. A therapeutic composition for use as defined in claim 1, wherein said cancer is selected from the group consisting of lymphoma, multiple myeloma, leukemia, melanoma, prostate cancer, breast cancer, renal cancer, bladder cancer, and combinations thereof

3. A therapeutic composition for use as defined in claim 1, wherein said sodium stibogluconate comprises a mixture of different compounds.

4. A therapeutic composition for use as defined in claim 3, wherein said mixture is fractionated and one or more fractions are eliminated.

5. A therapeutic composition for use as defined in claim 4, wherein said mixture is fractionated by high performance liquid chromatography.

6. A therapeutic composition for use in reducing the toxicity of IL-2 comprising a sodium stibogluconate and IL-2.

7. A therapeutic composition for use as defined in claim 6, wherein said sodium stibogluconate comprises a mixture of different compounds.

8. A therapeutic composition for use as defined in claim 7, wherein said mixture is fractionated and one or more fractions are eliminated.

9. A therapeutic composition for use as defined in claim 8, wherein said mixture is fractionated by high performance liquid chromatography.

10. Use of sodium stibogluconate and IL-2 for the preparation of a pharmaceutical composition for treating cancer.

11. The use as defined in claim 10, wherein said cancer is selected from the group consisting of lymphoma, multiple myeloma, leukemia, melanoma, prostate cancer, breast cancer, renal cancer, bladder cancer, and combinations thereof.

12. The use as defined in claim 10, wherein said sodium stibogluconate comprises a mixture of different compounds.

13. The use as defined in claim 12, wherein said mixture is fractionated and one or more fractions are eliminated.

14. The use as defined in claim 13, wherein said mixture is fractionated by high performance liquid chromatography.

15. Use of sodium stibogluconate and IL-2 for the preparation of a pharmaceutical composition for reducing the toxicity of IL-2.

16. The use as defined in claim 15, wherein said sodium stibogluconate comprises a mixture of different compounds.

17. The use as defined in claim 16, wherein said mixture is fractionated and one or more fractions are eliminated.

18. The use as defined in claim 17, wherein said mixture is fractionated by high performance liquid chromatography.

19. The use as defined in claim 15, wherein the sodium stibogluconate and IL-2 are administered sequentially.

20. The use as defined in claim 15, wherein the sodium stibogluconate and IL-2 are administered simultaneously.

21. Use of sodium stibogluconate and IL-2 for the preparation of a pharmaceutical composition for potentiating the therapeutic efficacy of IL-2.

22. The use as defined in claim 21, wherein the sodium stibogluconate and IL-2 are administered sequentially.

23. The use as defined in claim 21, wherein the sodium stibogluconate and IL-2 are administered simultaneously.

24. Use of sodium stibogluconate for the preparation of a pharmaceutical composition for potentiating the therapeutic efficacy of IL-2 in a subject undergoing IL-2 therapy.

## Patentansprüche

1. Therapeutische Zusammensetzung zur Verwendung bei der Behandlung von Krebs umfassend Natrium-Stibogluconat und IL-2.

2. Therapeutische Zusammensetzung zur Verwendung wie in Anspruch 1 definiert, wobei der Krebs ausgewählt ist aus der Gruppe bestehend aus Lymphom, multiples Myelom, Leukämie, Melanom, Prostatakrebs, Brustkrebs, Nierenkrebs, Blasenkrebs und Kombinationen davon.

3. Therapeutische Zusammensetzung zur Verwendung wie in Anspruch 1 definiert, wobei das Natrium-Stibogluconat eine Mischung aus verschiedenen Verbindungen umfasst.

4. Therapeutische Zusammensetzung zur Verwendung wie in Anspruch 3 definiert, wobei die Mischung fraktioniert ist und eine oder mehrere Fraktionen entfernt sind.

5. Therapeutische Zusammensetzung zur Verwendung wie in Anspruch 4 definiert, wobei die Mischung durch Hochleistungsflüssigkeitschromatographie fraktioniert ist.

6. Therapeutische Zusammensetzung zur Verwendung bei der Reduzierung der Toxizität von IL-2 umfassend ein Natrium-Stibogluconat und IL-2.

7. Therapeutische Zusammensetzung zur Verwendung wie in Anspruch 6 definiert, wobei das Natrium-Stibogluconat eine Mischung aus verschiedenen Verbindungen umfasst.

8. Therapeutische Zusammensetzung zur Verwendung wie in Anspruch 7 definiert, wobei die Mischung fraktioniert ist und eine oder mehrere Fraktionen entfernt sind.

9. Therapeutische Zusammensetzung zur Verwendung wie in Anspruch 8 definiert, wobei die Mischung durch Hochleistungsflüssigkeitschromatographie fraktioniert ist.

10. Verwendung von Natrium-Stibogluconat und IL-2 zur Herstellung einer pharmazeutischen Zusammensetzung zur Behandlung von Krebs.

11. Die Verwendung wie in Anspruch 10 definiert, wobei der Krebs ausgewählt ist aus der Gruppe bestehend aus Lymphom, multiples Myelom, Leukämie, Melanom, Prostatakrebs, Brustkrebs, Nierenkrebs, Blasenkrebs und Kombinationen davon.

12. Die Verwendung wie in Anspruch 10 definiert, wobei das Natrium-Stibogluconat eine Mischung aus verschiedenen Verbindungen umfasst.

13. Die Verwendung wie in Anspruch 12 definiert, wobei die Mischung fraktioniert ist und eine oder mehrere Fraktionen entfernt sind.

14. Die Verwendung wie in Anspruch 13 definiert, wobei die Mischung durch Hochleistungsflüssigkeitschromatographie fraktioniert ist.

15. Verwendung von Natrium-Stibogluconat und IL-2 für die Herstellung einer pharmazeutischen Zusammensetzung zur Reduzierung der Toxizität von IL-2.

16. Die Verwendung wie in Anspruch 15 definiert, wobei das Natrium-Stibogluconat eine Mischung aus verschiedenen Verbindungen umfasst.

17. Die Verwendung wie in Anspruch 16 definiert, wobei die Mischung fraktioniert ist und eine oder mehrere Fraktionen entfernt sind.

18. Die Verwendung wie in Anspruch 17 definiert, wobei die Mischung durch Hochleistungsflüssigkeitschromatographie fraktioniert ist.

19. Die Verwendung wie in Anspruch 15 definiert, wobei Natrium-Stibogluconat und IL-2 der Reihe nach verabreicht werden.

20. Die Verwendung wie in Anspruch 15 definiert, wobei Natrium-Stibogluconat und IL-2 simultan verabreicht werden.

21. Verwendung von Natrium-Stibogluconat und IL-2 für die Herstellung einer pharmazeutischen Zusammensetzung zur Potenzierung der therapeutischen Wirksamkeit von IL-2.

22. Die Verwendung wie in Anspruch 21 definiert, wobei Natrium-Stibogluconat und IL-2 der Reihe nach verabreicht wird.

23. Die Verwendung wie in Anspruch 21 definiert, wobei Natrium-Stibogluconat und IL-2 simultan verabreicht werden.

24. Verwendung von Natrium-Stibogluconat für die Herstellung einer pharmazeutischen Zusammensetzung zur Potenzierung der therapeutischen Wirksamkeit von IL-2 bei einem Probanden, der eine IL-2-Therapie durchmacht.

## Revendications

1. Composition thérapeutique pour son utilisation dans le traitement du cancer comprenant du stibogluconate de sodium et IL-2.

2. Composition thérapeutique pour son utilisation comme définie dans la revendication 1, dans laquelle ledit cancer est choisi dans le groupe consistant en le lymphome, le myélome multiple, la leucémie, le mélanome, le cancer de la prostate, le cancer du sein, le cancer du rein, le cancer de la vessie et leurs combinaisons.

3. Composition thérapeutique pour son utilisation comme définie dans la revendication 1, dans laquelle ledit stibogluconate de sodium comprend un mélange de différents composés.

4. Composition thérapeutique pour son utilisation comme définie dans la revendication 3, dans laquelle ledit mélange est fractionné et une ou plusieurs fractions sont éliminées.

5. Composition thérapeutique pour son utilisation comme définie dans la revendication 4, dans laquelle ledit mélange est fractionné par chromatographie liquide haute performance.

6. Composition thérapeutique pour son utilisation dans la réduction de la toxicité d'IL-2 comprenant un stibogluconate de sodium et IL-2.

7. Composition thérapeutique pour son utilisation comme définie dans la revendication 6, dans laquelle ledit stibogluconate de sodium comprend un mélange de différents composés.

8. Composition thérapeutique pour son utilisation comme définie dans la revendication 7, dans laquelle ledit mélange est fractionné et une ou plusieurs fractions sont éliminées.

9. Composition thérapeutique pour son utilisation comme définie dans la revendication 8, dans laquelle ledit mélange est fractionné par chromatographie liquide haute performance.

10. Utilisation de stibogluconate de sodium et IL-2 pour la préparation d'une composition pharmaceutique pour traiter le cancer.

11. Utilisation selon la revendication 10, dans laquelle ledit cancer est choisi dans le groupe consistant en le lymphome, le myélome multiple, la leucémie, le mélanome, le cancer de la prostate, le cancer du sein, le cancer du rein, le cancer de la vessie et leurs combinaisons.

12. Utilisation selon la revendication 10, dans laquelle ledit stibogluconate de sodium comprend un mélange de différents composés.

13. Utilisation selon la revendication 12, dans laquelle ledit mélange est fractionné et une ou plusieurs fractions sont éliminées.

14. Utilisation selon la revendication 13, dans laquelle ledit mélange est fractionné par chromatographie liquide haute performance.

15. Utilisation de stibogluconate de sodium et IL-2 pour la préparation d'une composition pharmaceutique pour réduire la toxicité d'IL-2.

16. Utilisation selon la revendication 15, dans laquelle ledit stibogluconate de sodium comprend un mélange de différents composés.

17. Utilisation selon la revendication 16, dans laquelle ledit mélange est fractionné et une ou plusieurs fractions sont éliminées.

18. Utilisation selon la revendication 17, dans laquelle ledit mélange est fractionné par chromatographie liquide haute performance.

19. Utilisation selon la revendication 15, dans laquelle le stibogluconate de sodium et IL-2 sont administrés de manière séquentielle.

20. Utilisation selon la revendication 15, dans laquelle le stibogluconate de sodium et IL-2 sont administrés de manière simultanée.

21. Utilisation de stibogluconate de sodium et IL-2 pour la préparation d'une composition pharmaceutique pour potentialiser l'efficacité thérapeutique d'IL-2.

22. Utilisation selon la revendication 21, dans laquelle le stibogluconate de sodium et IL-2 sont administrés de manière séquentielle.

23. Utilisation selon la revendication 21, dans laquelle le stibogluconate de sodium et IL-2 sont administrés de manière simultanée.

24. Utilisation de stibogluconate de sodium pour la préparation d'une composition pharmaceutique pour potentialiser l'efficacité thérapeutique d'IL-2 chez un sujet subissant une thérapie avec IL-2.
